# EUROPEAN PATENT SPECIFICATION

(11) **EP 0 799 218 B1**
(45) Date of publication and mention of the grant of the patent: **19.02.2003**
(21) Application number: 95941552.2
(22) Date of filing: 18.12.1995
(51) Int. Cl.: C07D 307/58, C07D 407/04, C07D 307/94, C07C 317/24, C07F 9/655, C07F 9/6553, C07F 9/6561, A61K 31/66

(54) **DIARYL-2-(5H)-FURANONES AS COX-2 INHIBITORS**
DIARYL-2(5H)-FUARANONE ALS COX-2-INHIBITOREN
DIARYL-2-(5H)-FURANONES INHIBITEURS DE COX-2

(30) Priority: 21.12.1994 US 361268
(43) Date of publication of application: 08.10.1997
(73) Proprietor: Merck Frosst Canada & Co., Halifax, Nova Scotia B3J 2X2 (CA)
(72) Inventor: BLACK, Cameron, Kirkland, Quebec H9H 3L1 (CA); GRIMM, Erich, Kirkland, Quebec H9H 3L1 (CA); LEGER, Serge, Kirkland, Quebec H9H 3L1 (CA); PRASIT, Petpiboon, Kirkland, Quebec H9H 3L1 (CA); WANG, Zhaoyin, Kirkland, Quebec H9H 3L1 (CA)
(74) Representative: Cole, William Gwyn
(86) International application number: CA9500715
(87) International publication number: WO96019469

(56) References cited:
- WO-A-94/15932
- WO-A-95/00501
- WO-A-95/21817
- US-A- 5 344 991
- US-A- 5 393 790
- US-A- 5 418 254
- JOURNAL OF MEDICINAL CHEMISTRY, vol. 38, no. 22, 27 October 1995 WASHINGTON US, pages 4570-4578, JAMES J. LI ET AL. '1,2-Diarylcyclopentenes as Selective Cyclooxygenase-2 Inhibitors and Orally Active Anti-inflammatory Agents'
- CHEMICAL ABSTRACTS, vol. 123, no. 17, 23 October 1995 Columbus, Ohio, US; abstract no. 227777r, DAVID B. REITZ ET AL. 'Selective cyclooxygenase inhibitors: novel diarylspirocycloalkenes are potent and orally active COX-2 inhibitors' page 1149; column 2; & BIOORG. MED. CHEM. LETT., vol. 5, no. 8, 1995 pages 867-872,
- CHEMICAL ABSTRACTS, vol. 123, no. 7, 14 August 1995 Columbus, Ohio, US; abstract no. 74203y, DAVID B. REITZ ET AL. 'Novel 1,2-diarylcyclopentenes are selective, potent, and orally active cyclooxygenase inhibitors' page 29; column 1; & MED. CHEM. RES., vol. 5, no. 5, 1995 pages 351-363,
- JOURNAL OF MEDICINAL CHEMISTRY, vol. 39, no. 1, 5 January 1996 WASHINGTON US, pages 253-266, HORNG-CHIH HUANG ET AL. 'Diaryl[2.4]heptenes as Orally Active, Highly Selective Cyclooxygenase-2-Inhibitors: Synthesis and Structure-Activity Relationships'

## Description

### BACKGROUND OF THE INVENTION

This invention relates to methods of treating cyclooxygenase mediated diseases and certain pharmaceutical compositions therefor.

Non-steroidal, antiinflammatory drugs exert most of their antiinflammatory, analgesic and antipyretic activity and inhibit hormone-induced uterine contractions and certain types of cancer growth through inhibition of prostaglandin G/H synthase, also known as cyclooxygenase. Initially, only one form of cyclooxygenase was known, this corresponding to cyclooxygenase-1 or the constitutive enzyme, as originally identified in bovine seminal vesicles. More recently the gene for a second inducible form of cyclooxygenase (cyclooxygenase-2) has been cloned, sequenced and characterized initially from chicken, murine and human sources. This enzyme is distinct from the cyclooxygenase-1 which has been cloned, sequenced and characterized from various sources including the sheep, the mouse and man. The second form of cyclooxygenase, cyclooxygenase-2, is rapidly and readily inducible by a number of agents including mitogens, endotoxin, hormones, cytokines and growth factors. As prostaglandins have both physiological and pathological roles, we have concluded that the constitutive enzyme, cyclooxygenase-1, is responsible, in large part, for endogenous basal release of prostaglandins and hence is important in their physiological functions such as the maintenance of gastrointestinal integrity and renal blood flow. In contrast, we have concluded that the inducible form, cyclooxygenase-2, is mainly responsible for the pathological effects of prostaglandins where rapid induction of the enzyme would occur in response to such agents as inflammatory agents, hormones, growth factors, and cytokines. Thus, a selective inhibitor of cyclooxygenase-2 will have similar antiinflammatory, antipyretic and analgesic properties to a conventional non-steroidal antiinflammatory drug, and in addition would inhibit hormone-induced uterine contractions and have potential anti-cancer effects, but will have a diminished ability to induce some of the mechanism-based side effects. In particular, such a compound should have a reduced potential for gastrointestinal toxicity, a reduced potential for renal side effects, a reduced effect on bleeding times and possibly a lessened ability to induce asthma attacks in aspirin-sensitive asthmatic subjects.

A brief description of the potential utilities of cyclooxygenase-2 inhibitors is given in an article by John Vane, Nature, Vol. 367, pp. 215-216, 1994 and in an article in Drug News and Perspectives Vol. 7, pp. 501-512, 1994.

WO-A-9415932 discloses thiophenes, furans and pyrroles as cyclooxygenase inhibitors. They differ from those of the present application by the nature and number of substituents attached to the thiophene or furan ring. In particular there is no disclosure of thiophenones or furanones.

WO-A-9500501, prior art for novelty purposes only, discloses furanones only being optionally substituted on the central ring by C₁₋₆alkyl.

### SUMMARY OF THE INVENTION

The invention encompasses the novel compound of Formula I as well as a method of treating cyclooxygenase-2 mediated diseases comprising administration to a patient in need of such treatment of a non-toxic therapeutically effective amount of a compound of Formula I.

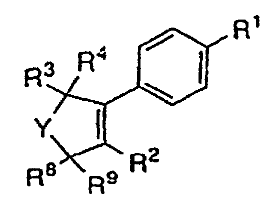

The invention also encompasses certain pharmaceutical compositions for treatment of cyclooxygenase-2 mediated diseases comprising compounds of Formula I.

### DETAILED DESCRIPTION OF THE INVENTION

The invention encompasses the novel compound of Formula I as well as a method of treating cyclooxygenase-2 mediated diseases comprising administration to a patient in need of such treatment of a non-toxic therapeutically effective amount of a compound of Formula I.

The present invention thus provides a compound of Formula 1. 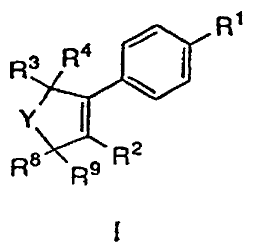 or a pharmaceutically acceptable salt thereof
wherein:
Y is selected from the group consisting of
   (a) C(R¹⁰)(R¹¹),
   (b) oxygen,
   (c) sulfur,
R¹ is selected from the group consisting of
   (a) S(O)₂CH₃,
   (b) S(O)₂NH₂,
   (c) S(O)₂NHC(O)CF₃,
   (d) S(O)(NH)NH₂,
   (e) S(O)(NH)NHC(O)CF₃,
   (f) P(O)(CH₃)NH₂,
   (g) P(O)(CH₃)₂,
R² is selected from the group consisting of
   (a) C₁₋₆alkyl,
   (b) C₃₋₇ cycloalkyl,
   (c) mono-, di- or tri-substituted phenyl or naphthyl wherein the substituent is selected from the group consisting of
      (1) hydrogen,
      (2) halo,
      (3) C₁₋₆alkoxy,
      (4) C₁₋₆alkylthio,
      (5) CN,
      (6) CF₃,
      (7) C₁₋₆alkyl,
      (8) N₃,
      (9) -CO₂H,
      (10) -CO₂-C₁₋₄alkyl,
      (11) -C(R⁵)(R⁶)-OH,
      (12) -C(R⁵)(R⁶)-O-C₁₋₄alkyl, and
      (13) -C₁₋₆alkyl-CO₂-R⁵;
      (14) benzyloxy,
      (15) -O-(C₁₋₆alkyl)-CO₂R⁵,
      (16) -O-(C₁₋₆alkyl)-NR⁵R⁶;
   (d) mono-, di- or tri-substituted heteroaryl wherein the heteroaryl is a monocyclic aromatic ring of 5 atoms, said ring having one hetero atom which is S, O, or N, and optionally 1, 2, or 3 additionally N atoms; or
      the heteroaryl is a monocyclic ring of 6 atoms, said ring having one hetero atom which is N, and optionally 1, 2, 3, or 4 additional N atoms, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R⁵)(R⁶)-OH, and
      (10) -C(R⁵)(R⁶)-O-C₁₋₄alkyl;
   (e) a mono- or di- substituted benzoheterocycle in which the heterocycle is a 5, 6, or 7-membered ring which may contain 1 or 2 heteroatoms chosen independently from O, S, or N and which may contain a carbonyl group or a sulfonyl group; the said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R⁵)(R⁶)-OH, and
      (10) -C(R⁵)(R⁶)-O-C₁₋₄alkyl;
   (f) a heterocycloalkyl group of 5-7 members which contains 1 or 2 heteroatoms chosen from O, S, or N and optionally contains a carbonyl group or a sulfonyl group.
   (g) a mono- or di- substituted benzocarbocycle in which the carbocycle is a 5, 6, or 7-membered ring which optionally contains a carbonyl group, the said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R⁵)(R⁶)-OH, and
      (10) -C(R⁵)(R⁶)-O-C₁₋₄alkyl;
R³ is a mono- or di- substituted phenyl, a mono- or di- substituted benzyl, a mono- or di- substituted heteroaryl, a mono- or di- substituted heteroarylmethyl wherein the substituents are selected from
   (1) hydrogen,
   (2) halo, including fluoro, chloro, bromo and iodo,
   (3) C₁₋₆alkyl,
   (4) C₁₋₆alkoxy,
   (5) C₁₋₆alkylthio,
   (6) CN,
   (7) CF₃,
   (8) N₃,
   (9) -C(R⁵)(R⁶)-OH, and
   (10) -C(R⁵)(R⁶)-O-C₁₋₄alkyl;
   C₁₋₇alkyl, CH₂OR⁷, CN, CH₂CN, C₁₋₆fluoroalkyl, F or CONR⁷₂ when Y is C(R¹⁰)(R¹¹), or
R³ is a mono- or di- substituted phenyl, a mono- or di- substituted benzyl, a mono- or di- substituted heteroaryl, a mono- or di- substituted heteroarylmethyl wherein the substituents are selected from
   (1) hydrogen,
   (2) halo, including fluoro, chloro, bromo and iodo,
   (3) C₁₋₆alkyl,
   (4) C₁₋₆alkoxy,
   (5) C₁₋₆alkylthio,
   (6) CN,
   (7) CF₃,
   (8) N₃,
   (9) -C(R⁵)(R⁶)-OH, and
   (10) -C(R⁵)(R⁶)-O-C₁₋₄alkyl;
   CH₂OR⁷, CN, CH₂CN, C₁₋₆fluoroalkyl, F or CONR⁷₂ when Y is O or S;
R⁴ is
   (a) a mono- or di- substituted phenyl, a mono- or di- substituted benzyl, a mono- or di- substituted heteroaryl or a mono- or di- substituted heteroarylmethyl as defined above, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R⁵)(R⁶)-OH, and
      (10) -C(R⁵)(R⁶)-O-C₁₋₄alkyl, or
   (b) C₁₋₇alkyl, CH₂OR⁷, CN, CH₂CN, C₁₋₆fluoroalkyl, F or CONR⁷₂ or
R³ and R⁴ together with the carbon to which they are attached form a saturated monocyclic ring of 3, 4, 5, 6 or 7 atoms, optionally containing 1 or 2 heteroatoms chosen independently from O, S, N, and optionally hexa-substituted by R¹² and optionally containing a carbonyl or sulfonyl group;
each R¹² is independently selected from the group consisting of
   (a) hydrogen,
   (b) C₁₋₆alkyl,
   (c) phenyl or monosubstituted phenyl wherein the substituents may be halo, C₁₋₆alkyl, C₁₋₆alkoxy, C₁₋₆alkylthio, CN, or CF₃,
   (d) benzyl or monosubstituted benzyl wherein the substituents may be halo, C₁₋₆alkyl, C₁₋₆alkoxy, C₁₋₆alkylthio, CN, or CF₃,
   (e) -CO₂-alkyl,
   (f) halo,
R⁵, R⁶ and R⁷ are each independently selected from the group consisting of
   (a) hydrogen, and
   (b) C₁₋₆alkyl,
   or R⁵ and R⁶ together with the carbon to which they are attached form a saturated monocyclic carbon ring of 3, 4, 5, 6 or 7 atoms;
R⁸ and R⁹ are independently selected from the group consisting of:
   (a) hydrogen,
   (b) C₁₋₇alkyl, or
   R⁸ and R⁹ together form a double bonded O or S;
R¹⁰ and R¹¹ are independently
   (a) hydrogen,
   (b) a mono- or di- substituted phenyl, a mono- or di- substituted benzyl, a mono- or di- substituted heteroaryl, or a mono- or di- substituted heteroarylmethyl, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R¹³)(R¹⁴)-OH, and
      (10) -C(R¹³)(R¹⁴)-O-C₁₋₄alkyl, or
   (c) C₁₋₇alkyl, CH₂OR⁷, CN, CH₂CN, C₁₋₆fluoroalkyl, F or CONR⁷₂, or
   (d) R¹⁰ and R¹¹ together form a double bonded O; or
   R¹⁰ and R¹¹ together with the carbon to which they are attached form a saturated monocyclic carbon ring of 3, 4, 5, 6 or 7 atoms;
R¹³ and R¹⁴ are independently selected from the group consisting of:
   (a) hydrogen,
   (b) C₁₋₇alkyl, or
   R¹³ and R¹⁴ together form a double bonded O or S.

For purposes of this specification heteroaryl as in R² is intended to include, but is not limited to optionally mono- or di-substituted
(1) furanyl,
(2) diazinyl, triazinyl, tetrazinyl,
(3) imidazolyl,
(4) isooxazolyl,
(5) isothiazolyl,
(6) oxadiazolyl,
(7) oxazolyl,
(8) pyrazolyl,
(9) pyrrolyl,
(10) thiadiazolyl,
(11) thiazolyl,
(12) thienyl,
(13) triazolyl, or
(14) tetrazolyl.

Similarly, for purposes of this specification cyclic groups such as a heterocycloalkyl or benzocarbocycle or benzoheterocycle such as in R² is intended to include, but is not limited to optionally mono- or di-substituted
(1) 2-indolyl,
(2) 3-indolyl,
(3) 1-methyl-5-indolyl
(4) 2-benzofuranyl,
(5) 3-benzofuranyl,
(6) 5-benzofuranyl,
(7) 6-benzofuranyl,
(8) 2-benzothienyl,
(9) 3-benzothienyl,
(10) 5-benzothienyl,
(11) 6-benzothienyl,

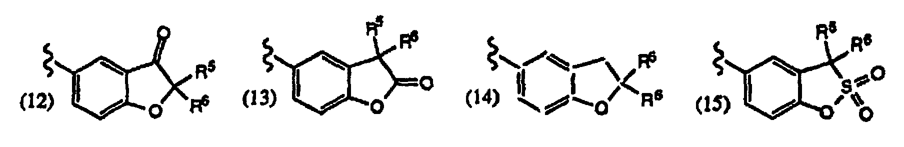

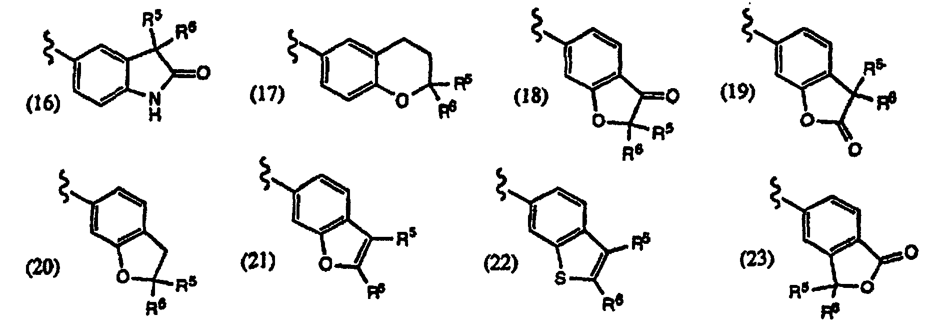

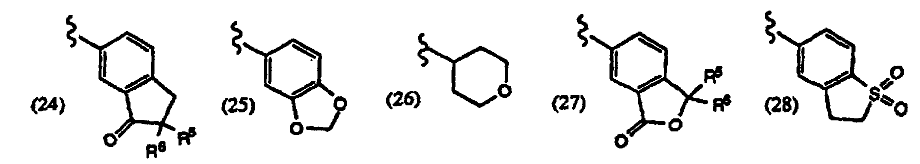

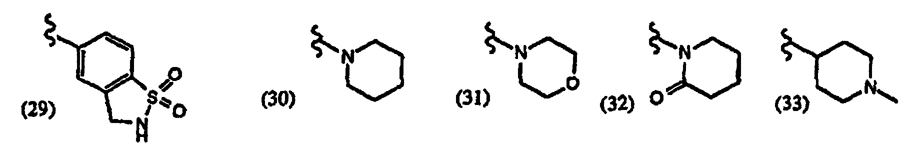

One genus of compounds of formula I is that in which R⁸ and R⁹ form a double-bonded O, Y is O.

Another genus is that in which R² is a mono-, di- or tri-substituted phenyl or naphthyl, and the remaining substituents are as defined for structure I.

Within this genus there is a class of compounds wherein wherein:
Y is O,
R¹ is selected from the group consisting of
   (a) S(O)₂CH₃,
   (b) S(O)₂NH₂,
   (c) S(O)₂NHC(O)CF₃,
   (d) S(O)(NH)NH₂,
   (e) S(O)(NH)NHC(O)CF₃,
   (f) P(O)(CH₃)NH₂,
   (g) P(O)(CH3)2
R² is selected from the group consisting of mono-, di- or tri-substituted phenyl or naphthyl wherein the substituent is selected from the group consisting of
   (1) hydrogen,
   (2) halo,
   (3) C₁₋₄alkoxy,
   (4) C₁₋₄alkylthio,
   (5) CN,
   (6) CF₃,
   (7) C₁₋₄alkyl,
   (8) N₃,
   (9) -CO₂H,
   (10) -CO₂-C₁₋₄alkyl,
   (11) -C(R⁵)(R⁶)-OH,
   (12) -C(R⁵)(R⁶)-O-C₁₋₄alkyl, and
   (13) -C₁₋₆alkyl-CO₂-R⁵,
   (14) benzyloxy,
   (15) -O-(C₁₋₄alkyl)-CO₂R⁵,
   (16) -O-(C₁₋₄alkyl)-NR⁵R⁶;
R³ is CH₂OR⁷, CN, CH₂CN, or C₁₋₆fluoroalkyl;
R⁴ is
   (a) substituted phenyl or substituted heteroaryl, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R⁵)(R⁶)-OH, and
      (10) -C(R⁵)(R⁶)-O-C₁₋₄alkyl, or
   (b) C₁₋₆alkyl, CH₂OR⁷, CN, CH₂CN, or C₁₋₆fluoroalkyl; or R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tetra-R¹²,
each R¹² is independently selected from the group consisting of
   (a) hydrogen,
   (b) C₁₋₆alkyl,
   (c) phenyl or monosubstituted phenyl wherein the substituents may be halo, C₁₋₆alkyl, C₁₋₆alkoxy, C₁₋₆alkylthio, CN, or CF₃,
   (d) benzyl or monosubstituted benzyl wherein the substituents may be halo, C₁₋₆alkyl, C₁₋₆alkoxy, C₁₋₆alkylthio, CN, or CF₃,
   (e) -CO₂-alkyl,
   (f) halo,
R⁵, R⁶ and R⁷ are each independently selected from the group consisting of
   (a) hydrogen, and
   (b) C₁₋₆alkyl,
   or R⁵ and R⁶ together with the carbon to which they are attached form a saturated monocyclic carbon ring of 3, 4, 5, 6 or 7 atoms.

Within this class are the compounds wherein
R¹ is selected from the group consisting of
   (a) S(O)₂CH₃,
   (b) S(O)₂NH₂,
R² is selected from the group consisting of mono-, di- or tri-substituted phenyl or naphthyl wherein the substituent is selected from the group consisting of
   (1) hydrogen,
   (2) halo,
   (3) C₁₋₄alkoxy,
   (4) C₁₋₄alkylthio,
   (5) CN,
   (6) CF₃,
   (7) C₁₋₄alkyl,
   (8) N₃, and
   (9) -C(R⁵)(R⁶)-OH;
R³ is CH₂OR⁷, CN, CH₂CN, or C₁₋₃fluoroalkyl;
R⁴ is
   (a) substituted phenyl or substituted heteroaryl, said substituents are selected from the group consisting of
      (1) hydrogen,
      (2) halo, including fluoro, chloro, bromo and iodo,
      (3) C₁₋₃alkyl,
      (4) C₁₋₃alkoxy,
      (5) C₁₋₄alkylthio,
      (6) CN,
      (7) CF₃, or
   (b) C₁₋₃alkyl, CH₂OR⁷, CN, CH₂CN, or C₁₋₃fluoroalkyl;
R⁵, R⁶ and R⁷ are each independently selected from the group consisting of
   (a) hydrogen, and
   (b) C₁₋₃alkyl.

Another genus of the invention encompasses compounds of formula Ib 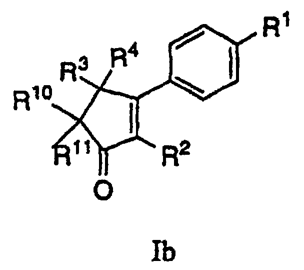 wherein
Y is C(R¹⁰)(R¹¹);
R¹ is selected from the group consisting of
   (a) S(O)₂CH₃,
   (b) S(O)₂NH₂,
   (c) S(O)₂NHC(O)CF₃,
   (d) S(O)(NH)NH₂,
   (e) S(O)(NH)NHC(O)CF₃,
   (f) P(O)(CH₃)NH₂,
   (g) P(O)(CH3)2
R² is selected from the group consisting of cyclohexyl, and
   mono-, di- or tri-substituted phenyl or naphthyl wherein the substituent is selected from the group consisting of
   (1) hydrogen,
   (2) halo,
   (3) C₁₋₆alkoxy,
   (4) C₁₋₆alkylthio,
   (5) CN,
   (6) CF₃,
   (7) C₁₋₆alkyl,
   (8) N₃,
   (9) -CO₂H,
   (10) -CO₂-C₁₋₄alkyl,
   (11) -C(R⁵)(R⁶)-OH,
   (12) -C(R⁵)(R⁶)-O-C₁₋₄alkyl, and
   (13) -C₁₋₆alkyl-CO₂-R⁵;
   (14) benzyloxy,
   (15) -O-(C₁₋₆alkyl)-CO₂R⁵
   (16) -O-(C₁₋₆alkyl)-NR⁵R⁶
R³ is CH₂OR⁷, CN, CH₂CN, or C₁₋₆fluoroalkyl;
R⁴ is
   (a) substituted phenyl or substituted heteroaryl, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R⁵)(R⁶)-OH, and
      (10) -C(R⁵)(R⁶)-O-C₁₋₄alkyl, or
   (b) C₁₋₆alkyl, CH₂OR⁷, CN, CH₂CN, or C₁₋₆fluoroalkyl; or R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tetra-R¹²,
each R¹² is independently selected from the group consisting of
   (a) hydrogen,
   (b) C₁₋₆alkyl,
   (c) phenyl or monosubstituted phenyl wherein the substituents may be halo, C₁₋₆alkyl, C₁₋₆alkoxy, C₁₋₆alkylthio, CN, or CF₃,
   (d) benzyl or monosubstituted benzyl wherein the substituents may be halo, C₁₋₆alkyl, C₁₋₆alkoxy, C₁₋₆alkylthio, CN, or CF₃,
   (e) -CO₂-alkyl
   (f) halo,
R⁵, R⁶ and R⁷ are each independently selected from the group consisting of
   (a) hydrogen, and
   (b) C₁₋₆alkyl,
   or R⁵ and R⁶ together with the carbon to which they are attached form a saturated monocyclic carbon ring of 3, 4, 5, 6 or 7 atoms;
R¹⁰ and R¹¹ are independently
   (a) hydrogen,
   (b) substituted phenyl or substituted heteroaryl, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
      (8) N₃,
      (9) -C(R¹³)(R¹⁴)-OH, and
      (10) -C(R¹³)(R¹⁴)-O-C₁₋₄alkyl, or
   (c) C₁₋₇alkyl, CH₂OR⁷, CN, CH₂CN, or C₁₋₆fluoroalkyl; or
R¹⁰ and R¹¹ together with the carbon to which they are attached form a saturated monocyclic carbon ring of 3, 4, 5, 6 or 7 atoms;
R¹³ and R¹⁴ are independently selected from the group consisting of:
   (a) hydrogen,
   (b) C₁₋₆alkyl, or
   R¹³ and R¹⁴ together form a double bonded O or S.

Within this genus are the compounds wherein:
R¹ is selected from the group consisting of
   (a) S(O)₂CH₃,
   (b) S(O)₂NH₂,
   (c) S(O)(NH)NH₂,
R² is selected from the group consisting of mono-, di- or tri-substituted phenyl or naphthyl wherein the substituent is selected from the group consisting of
   (1) hydrogen,
   (2) halo,
   (3) C₁₋₆alkoxy,
   (4) C₁₋₄alkylthio,
   (5) CN,
   (6) CF₃,
   (7) C₁₋₄alkyl,
   (8) N₃,
   (9) -CO₂H,
   (10) -CO₂-C₁₋₄alkyl,
   (11) -C(R⁵)(R⁶)-OH,
   (12) -C(R⁵)(R⁶)-O-C₁₋₄alkyl, and
   (13) -C₁₋₄alkyl-CO₂-R⁵;
R³ is CH₂OR⁷, CN, CH₂CN, or C₁₋₆fluoroalkyl;
R⁴ is
   (a) substituted phenyl or substituted heteroaryl, said substituents are selected from the group consisting of
      (1) hydrogen,
      (2) fluoro, chloro and bromo,
      (3) C₁₋₄alkyl,
      (4) C₁₋₄alkoxy,
      (5) C₁₋₄alkylthio,
      (6) CN,
      (7) CF₃, or
   (b) C₁₋₄alkyl, CH₂OR⁷, CN, CH₂CN, or C₁₋₄fluoroalkyl; or R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tri-R¹²,
each R¹² is independently selected from the group consisting of
   (a) hydrogen,
   (b) C₁₋₄alkyl,
   (c) phenyl or monosubstituted phenyl wherein the substituents may be halo, C₁₋₄alkyl, C₁₋₄alkoxy, C₁₋₆alkylthio, CN, or CF₃,
   (d) benzyl or monosubstituted benzyl wherein the substituents may be halo, C₁₋₄alkyl, C₁₋₄alkoxy, C₁₋₄alkylthio, CN, or CF₃,
   (e) -CO₂-C₁₋₄alkyl,
   (f) halo,
R⁵, R⁶ and R⁷ are each independently selected from the group consisting of
   (a) hydrogen, and
   (b) C₁₋₄alkyl,
   or R⁵ and R⁶ together with the carbon to which they are attached form a saturated monocyclic carbon ring of 3, 4, 5, 6 or 7 atoms;
R¹⁰ and R¹¹ are independently
   (a) hydrogen,
   (b) substituted phenyl or substituted heteroaryl, said substituents are selected from the group consisting of
      (1) hydrogen,
      (2) halo, including fluoro, chloro, bromo and iodo,
      (3) C₁₋₆alkyl,
      (4) C₁₋₆alkoxy,
      (5) C₁₋₆alkylthio,
      (6) CN,
      (7) CF₃,
   (c) C₁₋₄alkyl, CH₂OR⁷, CN, CH₂CN, or C₁₋₄fluoroalkyl; or
   R¹⁰ and R¹¹ together with the carbon to which they are attached form a saturated monocyclic carbon ring of 3, 4, 5, 6 or 7 atoms;
R¹³ and R¹⁴ are independently selected from the group consisting of:
   (a) hydrogen,
   (b) C₁₋₄alkyl.

The invention is illustrated by the compounds of Examples 1 through 14 as disclosed herein as well as the compounds of Table I through IV.

For purposes of this specification, alkyl is defined to include linear, branched, and cyclic structures, with C₁₋₆alkyl including, but not restricted to, methyl, ethyl, propyl, 2-propyl, n-, i-, s- and t-butyl, pentyl, hexyl, 1,1-dimethylethyl, cyclopropyl, cyclobutyl, cyclopentyl and cyclohexyl. Similarly, C₁₋₆alkoxy is intended to include alkoxy groups of from 1 to 6 carbon atoms of a straight, branched, or cyclic configuration. Examples of lower alkoxy groups include methoxy, ethoxy, propoxy, isopropoxy, cyclopropyloxy, cyclohexyloxy, and the like. Likewise, C₁₋₆alkylthio is intended to include alkylthio groups of from 1 to 6 carbon atoms of a straight, branched or cyclic configuration. Examples of lower alkylthio groups include methylthio, n-propylthio, isopropylthio, cyclohexylthio, etc. By way of illustration, the propylthio group signifies -SCH₂CH₂CH₃. C₁₋₆fluoroalkyl includes alkyl groups of from 1 to 6 carbon atoms of a straight, branched or cyclic configuration, in which one or more hydrogen is replaced by fluorine. Examples are -CHF₂, CH₂F, -CF₃, -CH₂CF₃, c-pr-F₅, c-Hex-F₁₁, and the like. Halo includes F, Cl, Br, or I. Heteroaryl includes furan, thiophene, pyrrole, isoxazole, isothiazole, pyrazole, oxazole, thiazole, imidazole, 1,2,3-oxadiazole, 1,2,3-thiadiazole, 1,2,3-triazole, 1,3,4-oxadiazole, 1,3,4-thiadiazole, 1,3,4-triazole, 1,2,5-oxadiazole, 1,2,5-thiadiazole, pyridine, pyridazine, pyrimidine, pyrazine, 1,2,4-triazine, 1,3,5-triazine, 1,2,4,5-tetrazine, and the like.

Exemplifying the invention are:
(a) 5,5-Dimethyl-4-(4-(methylsulfonyl)phenyl)-3-(4-methoxyphenyl)-2-(5H)-furanone,
(b) 5,5-Dimethyl-3-(3-methoxyphenyl)-4-(4-(methylsulfonyl)phenyl)-2-(5H)-furanone,
(c) 5,5-Dimethyl-3-(4-isopropylphenyl)-4-(4-(methylsulfonyl)phenyl)-2-(5H)-furanone,
(d) 5,5-Dimethyl-4-(4-(methylsulfonyl)phenyl)-3-phenyl-2-(5H)-furanone,
(e) 3-(Benzo[1,3]dioxol-5-yl)-5,5-dimethyl-4-(4-(methylsulfonyl)phenyl)-2-(5H)-furanone,
(f) 5,5-Dimethyl-3-(4-methylphenyl)-4-(4-(methylsulfonyl)phenyl)-2-(5H)-furanone,
(g) 5,5-Dimethyl-4-(4-(methylsulfonyl)phenyl)-3-(4-trifluoromethylphenyl)-2-(5H)-furanone,
(h) 5,5-Dimethyl-3-(3-methylphenyl)-4-(4-(methylsulfonyl)phenyl)-2-(5H)-furanone,
(i) 5,5-Dimethyl-3-cyclohexyl-4-(4-(methylsulfonyl) phenyl)-2-(5H)-furanone,
(j) 4-(4-(Methylsulfonyl)phenyl)-3-phenyl-1-oxa-spiro[4,4]non-3-en-2-one,
(k) 5,5-Dimethyl-3-(4-(methylsulfonyl)phenyl)-2-phenylocyclopent-2-enone,
(l) 4,4-Dimethyl-3-(4-(methylsulfonyl)phenyl)-2-phenylcyclopent-2-enone,
(m) 7-(4-(methylsulfonyl)phenyl)-6-phenyl-4-oxa-spiro[2,4]-hept-6-en-5-one, and
(n) 5,5-Bis(fluoromethyl)-3-phenyl-4-(4-(methylsulfonyl)phenyl)-2-(5H)-furanone.

Also exemplifying the invention are the compounds of Examples 15-41.

Some of the compounds described herein contain one or more asymmetric centers and may thus give rise to diastereomers and optical isomers. The present invention is meant to comprehend such possible diastereomers as well as their racemic and resolved, enantiomerically pure forms and pharmaceutically acceptable salts thereof.

Some of the compounds described herein contain olefinic double bonds, and unless specified otherwise, are meant to include both E and Z geometric isomers.

In a second embodiment, the invention encompasses pharmaceutical compositions for inhibiting cyclooxygenase and for treating cyclooxygenase mediated diseases as disclosed herein comprising a pharmaceutically acceptable carrier and a non-toxic therapeutically effective amount of compound of formula I as described above.

Within this embodiment the invention encompasses pharmaceutical compositions for inhibiting cyclooxygenase-2 and for treating cyclooxygenase-2 mediated diseases as disclosed herein comprising a pharmaceutically acceptable carrier and a non-toxic therapeutically effective amount of compound of formula I as described above.

In a third embodiment, the invention encompasses the use of compounds in a method of inhibiting cyclooxygenase and treating cyclooxygenase mediated diseases, advantageously treated by an active agent that selectively inhibits COX-2 in preference to COX-1 as disclosed herein comprising: administration to a patient in need of such treatment of a non-toxic therapeutically effective amount of a compound of Formula I as disclosed herein.

The pharmaceutical compositions of the present invention comprise a compound of Formula I as an active ingredient or a pharmaceutically acceptable salt, thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine. methylglucamine, morpholine, piperazine, piperidine, polyamine resins, procaine, purines, theobromine, triethylamine, trimethylamine, tripropylamine, tromethamine, and the like.

It will be understood that in the discussion of methods of treatment which follows, references to the compounds of Formula I are meant to also include the pharmaceutically acceptable salts.

The Compound of Formula I is useful for the relief of pain, fever and inflammation of a variety of conditions including rheumatic fever, symptoms associated with influenza or other viral infections, common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, burns, injuries, following surgical and dental procedures. In addition, such a compound may inhibit cellular neoplastic transformations and metastic tumor growth and hence can be used in the treatment of cancer. Compound I may also be of use in the treatment and/or prevention of cyclooxygenase-mediated proliferative disorders such as may occur in diabetic retinopathy and tumour angiogenesis.

Compound I will also inhibit prostanoid-induced smooth muscle contraction by preventing the synthesis of contractile prostanoids and hence may be of use in the treatment of dysmenorrhea, premature labor, asthma and eosinophil related disorders. It will also be of use in the treatment of Alzheimer's disease, and for the prevention of bone loss (treatment of osteoporosis).

By virtue of its high cyclooxygenase-2 (COX-2) activity and/or its specificity for cyclooxygenase-2 over cyclooxygenase-1 (COX-1), Compound I will prove useful as an alternative to conventional non-steroidal antiinflammatory drugs (NSAID'S) particularly where such non-steroidal antiinflammatory drugs may be contra-indicated such as in patients with peptic ulcers, gastritis, regional enteritis, ulcerative colitis, diverticulitis or with a recurrent history of gastrointestinal lesions; GI bleeding, coagulation disorders including anemia such as hypoprothrombinemia, haemophilia or other bleeding problems; kidney disease; those prior to surgery or taking anticoagulants.

Similarly, Compound I, will be useful as a partial or complete substitute for conventional NSAID'S in preparations wherein they are presently co-administered with other agents or ingredients. Thus in further aspects, the invention encompasses pharmaceutical compositions for treating cyclooxygenase-2 mediated diseases as defined above comprising a non-toxic therapeutically effective amount of the compound of Formula I as defined above and one or more ingredients such as another pain reliever including acetominophen or phenacetin; a potentiator including caffeine; an H₂-antagonist, aluminum or magnesium hydroxide, simethicone, a decongestant including phenylephrine, phenylpropanolamine, pseudophedrine, oxymetazoline, ephinephrine, naphazoline, xylometazoline, propylhexedrine, or levo-desoxyephedrine; an antiitussive including codeine, hydrocodone, caramiphen, carbetapentane, or dextramethorphan; a diuretic; a sedating or non-sedating antihistamine. In addition the invention encompasses a method of treating cyclooxygenase mediated diseases comprising: administration to a patient in need of such treatment a non-toxic therapeutically effect amount of the compound of Formula I, optionally co-administered with one or more of such ingredients as listed immediately above.

For the treatment of any of these cyclooxygenase mediated diseases Compound I may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle sheep, dogs, cats, etc., the compound of the invention is effective in the treatment of humans.

As indicated above, pharmaceutical compositions for treating cyclooxygenase-2 mediated diseases as defined may optionally include one or more ingredients as listed above.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Patent 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water or miscible solvents such as propylene glycol, PEGs and ethanol, or an oil medium, for example peanut oil, liquid paraffin, or olive oil.

Aqueous suspensions contain the active material in admixture with excipients suitable for the manufacture of aqueous suspensions. Such excipients are suspending agents, for example, sodium carboxymethylcellulose, methylcellulose, hydroxy-propylmethycellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example, arachis oil, olive oil, sesame oil or coconut oil, or in mineral oil such as liquid paraffin. The oily suspensions may contain a thickening agent, for example, beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The pharmaceutical compositions of the invention may also be in the form of an oil-in-water emulsions. The oily phase may be a vegetable oil, for example, olive oil or arachis oil, or a mineral oil, for example, liquid paraffin or mixtures of these. Suitable emulsifying agents may be naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions may also contain sweetening and flavouring agents.

Syrups and elixirs may be formulated with sweetening agents, for example, glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example, as a solution in 1,3-butane diol. Among the acceptable vehicles and solvents that may be employed are water, Ringer's solution and isotonic sodium chloride solution. Cosolvents such as ethanol, propylene glycol or polyethylene glycols may also be used. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compound I may also be administered in the form of a suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, gels, solutions or suspensions, etc., containing the compound of Formula I are employed. (For purposes of this application, topical application shall include mouth washes and gargles.) Topical formulations may generally be comprised of a pharmaceutical carrier, cosolvent, emulsifier, penetration enhancer, preservative system, and emollient.

Dosage levels of the order of from about 0.01 mg to about 140 mg/kg of body weight per day are useful in the treatment of the above-indicated conditions, or alternatively about 0.5 mg to about 7 g per patient per day. For example, inflammation may be effectively treated by the administration of from about 0.01 to 50 mg of the compound per kilogram of body weight per day, or alternatively about 0.5 mg to about 3.5 g per patient per day.

The amount of active ingredient that may be combined with the carrier materials to produce a single dosage form will vary depending upon the host treated and the particular mode of administration. For example, a formulation intended for the oral administration of humans may contain from 0.5 mg to 5 g of active agent compounded with an appropriate and convenient amount of carrier material which may vary from about 5 to about 95 percent of the total composition. Dosage unit forms will generally contain between from about 1 mg to about 500 mg of an active ingredient, typically 25 mg, 50 mg, 100 mg, 200 mg, 300 mg, 400 mg, 500 mg, 600 mg, 800 mg, or 1000 mg.

It will be understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of the present invention can be prepared according to the following methods.

### METHOD A

Friedel-Crafts-type acylation of thioanisole with an appropriate acid chloride provides ketone **1**. A mixture of **1,** an aqueous base such as NaOH, an organic solvent such as CCl₄/toluene and a phase transfer catalyst such as Aliquat 333™ is stirred in air at room temperature to provide **2**. Acylation of **2** with an appropriately substituted aryl acetic acid chloride in the presence a base such as pyridine followed by treatment with DBU affords lactone **3.** Oxidation of **3** with OXONE™ or a peracid such as MMPP yields the final product **4** (Rₐ=Me). Alternatively, compound **2** can be oxidized with OXONE™ or a peracid such as MMPP to its corresponding sulfone **5** (Rₐ=Me), which can then be acylated and cyclized under the same conditions as described for **2** to give the final product **4.** By methods known in the art, the -SMe group of **2** or **3** can be converted to the -SO₂NH₂ group.

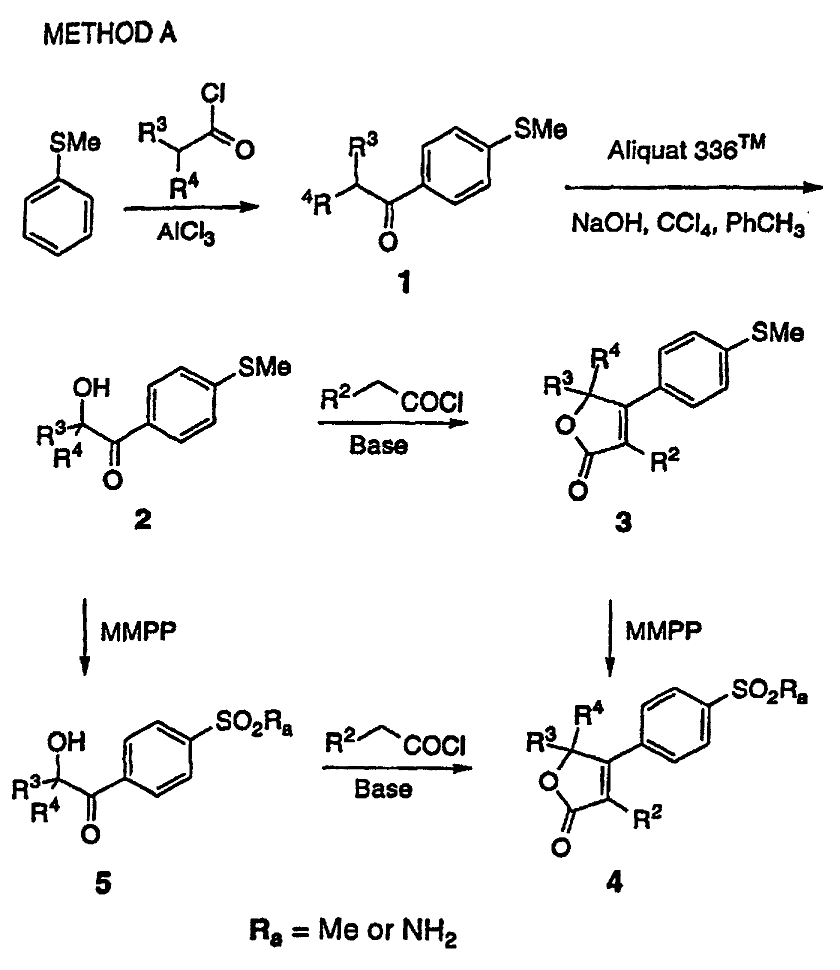

### Method B

Reaction of a 4,4-disubstituted-2-cyclopenten-1-one with iodine in pyridine and an inert solvent provides iodide **6,** which can be coupled with an appropriately substituted aryl boronic acid in the presence of a Pd° catalyst to give enone **7**. Addition of 4-methylthiophenyllithium to **7** affords allylic alcohol **8,** which, upon oxidation with PCC or PDC, can be transformed to enone **9**. Oxidation of **9** with OXONE™ or a peracid such as MMPP provides the desired product **10.** "Ar" is as defined for R² (c) and (d).

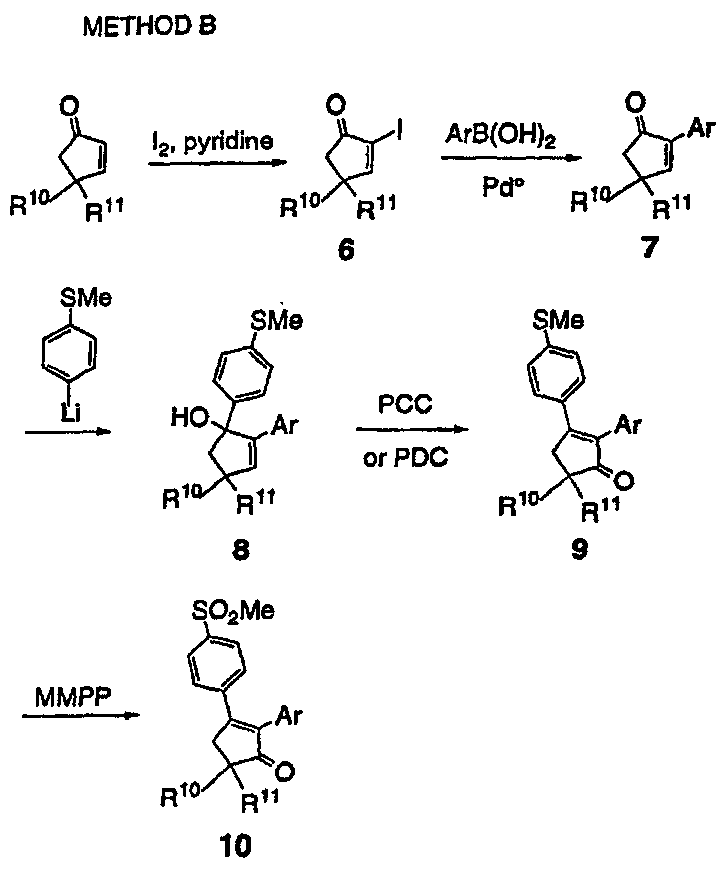

### Method C

2,2-Disubstituted pent-4-enal (prepared by the method described by P. D. Magnus and M. S. Nobbs, Syn. Comm. **1980,** *10,* 273) is reacted with an appropriate organometallic reagent to provide alcohol **11,** which can then be oxidized to ketone **12.** Ketone **12** is subjected to ozonolysis, followed by treatment with a base such as DBU, to afford cyclic enone **13**. Addition of 4-methylthiophenyllithium to **13** gives allylic alcohol **14**. Oxidation of **14** with MMPP introduces the required methylsulfonyl group in **15.** Oxidation of **15** with PCC affords the final product **16.** "Ar" in Method C is as defined for R² (c) and (d).

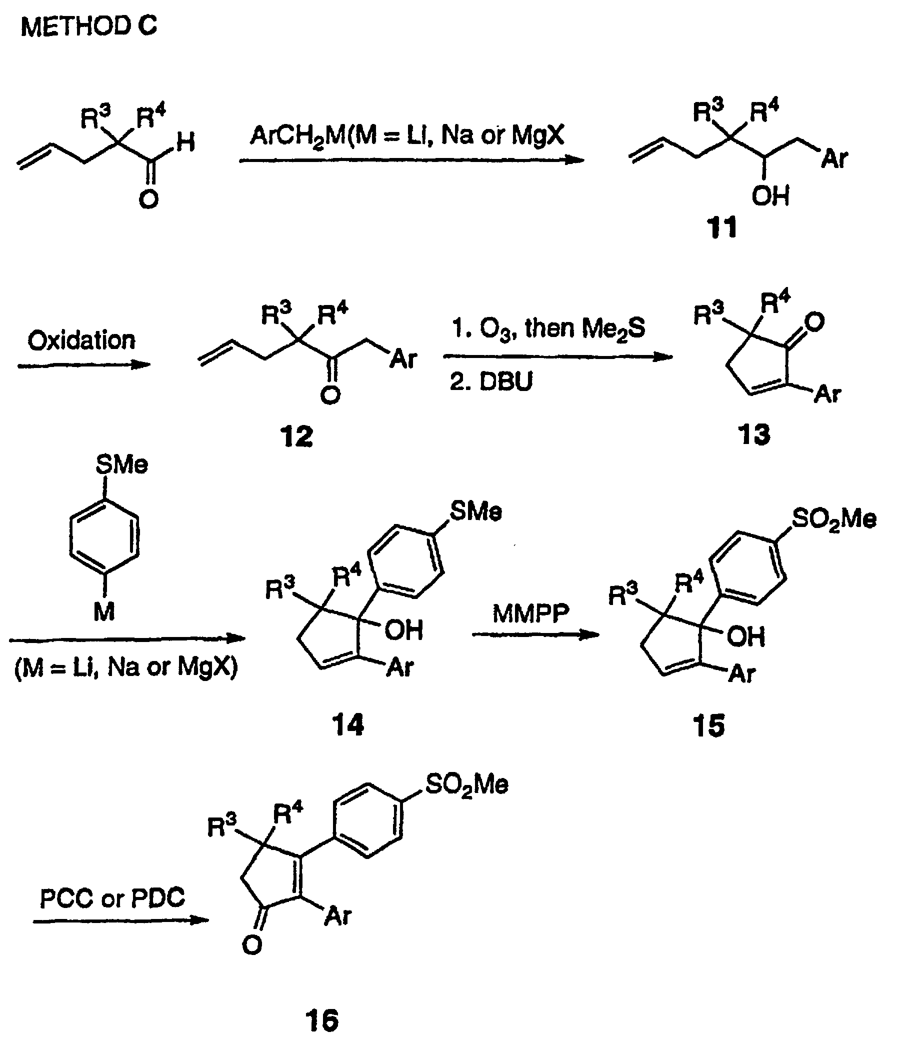

### METHOD D:

An appropriately substituted phenyl bromomethyl ketone **17** is reacted with an appropriately substituted acetic acid in a solvent such as acetonitrile in the presence of a base such as triethylamine and then treated with DBU to afford lactone **18**. Lactone **18** is reacted with an excess of aqueous formaldehyde solution in the presence of a base such as K₂CO₃ in an inert solvent to provide a mixture of alcohols **19** and **20**, which are separated by silica gel chromatography. Alcohol **19** is then treated with methanesulfonyl chloride and a base to yield compound **21.** Cyclopropanation of **21** with R¹²₂CN₂ can be accomplished using a palladium or rhodium catalyst or with R¹²₂CHX and a base to afford the desired product **22**.

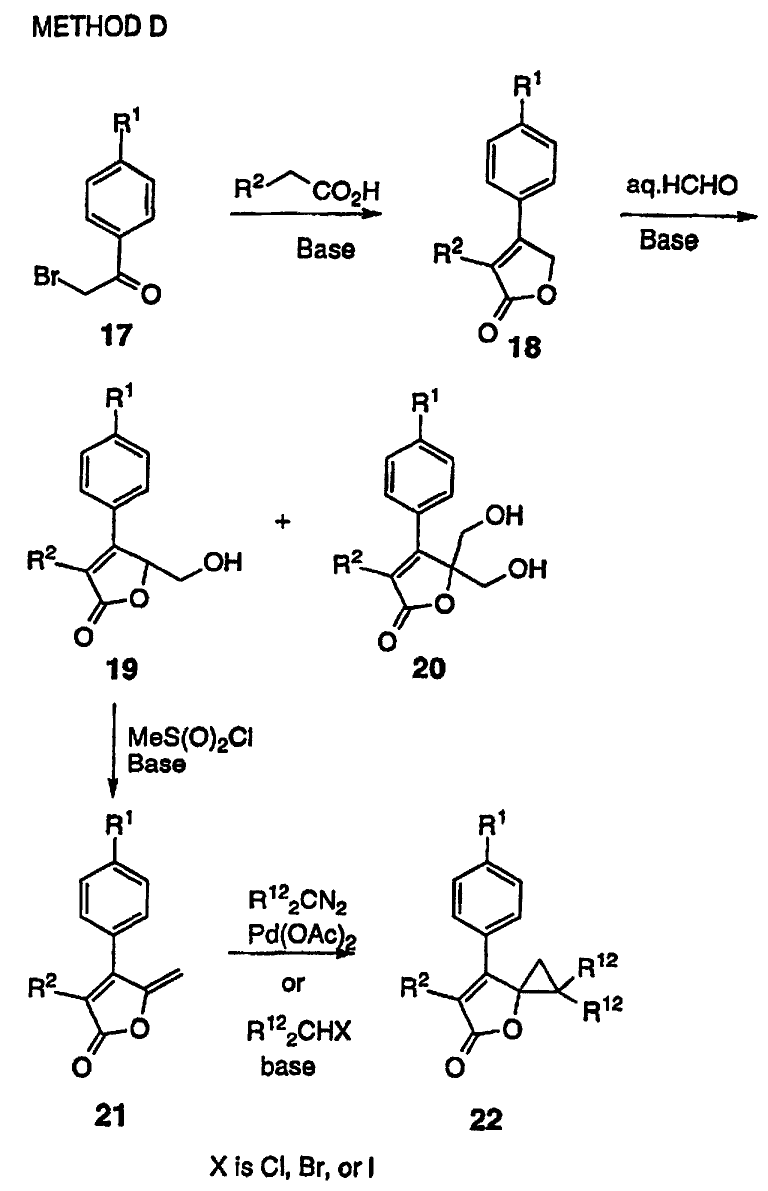

### METHOD E:

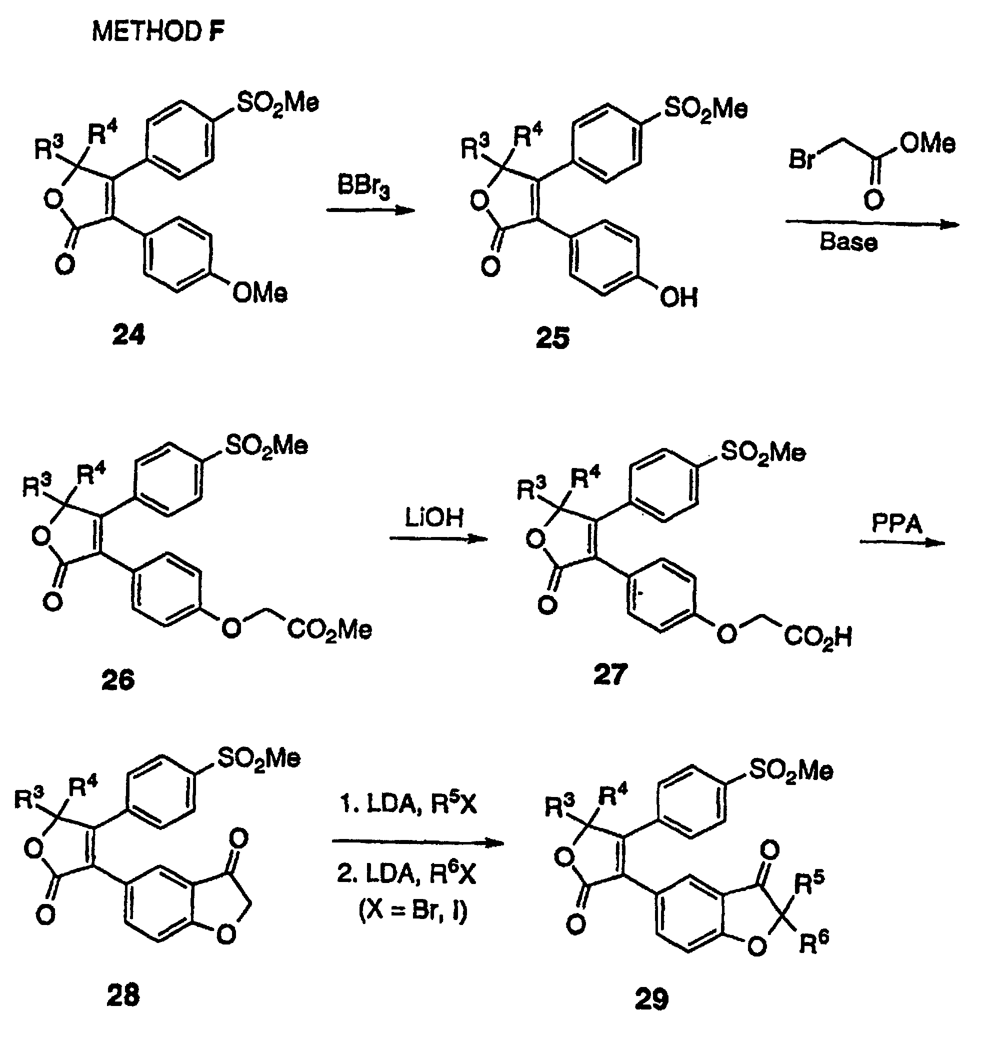

### Method G

Phenol **25** may be treated with NaH in DMF and then alkylated with an allylic bromide to give ether **30.** Claisen rearrangement of **30** under thermal conditions gives *ortho*-allyl phenol **31.** Oxidation of the double bond in **31** with OsO₄/NMO followed by cleavage with NaIO₄ provides the lactol **32**. Jones oxidation then provides lactone **33**.

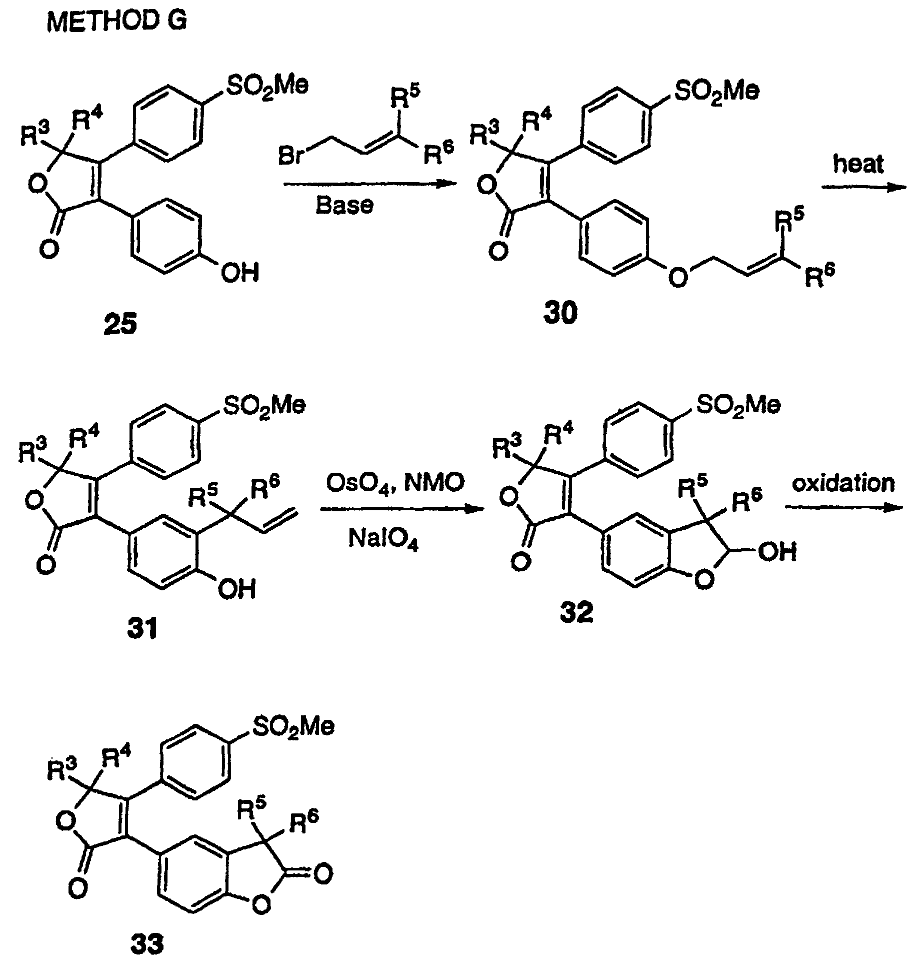

### Method H

Acid **27** is transformed to acid chloride **34** by treatment with oxalyl chloride in the presence of catalytic DMF. Treatment of **35** with two types of Grignard reagent, R⁵MgX and R⁶MgX, gives tertiary alcohol **35**. Cyclization of **35** with a Lewis acid then provides ether **36**.

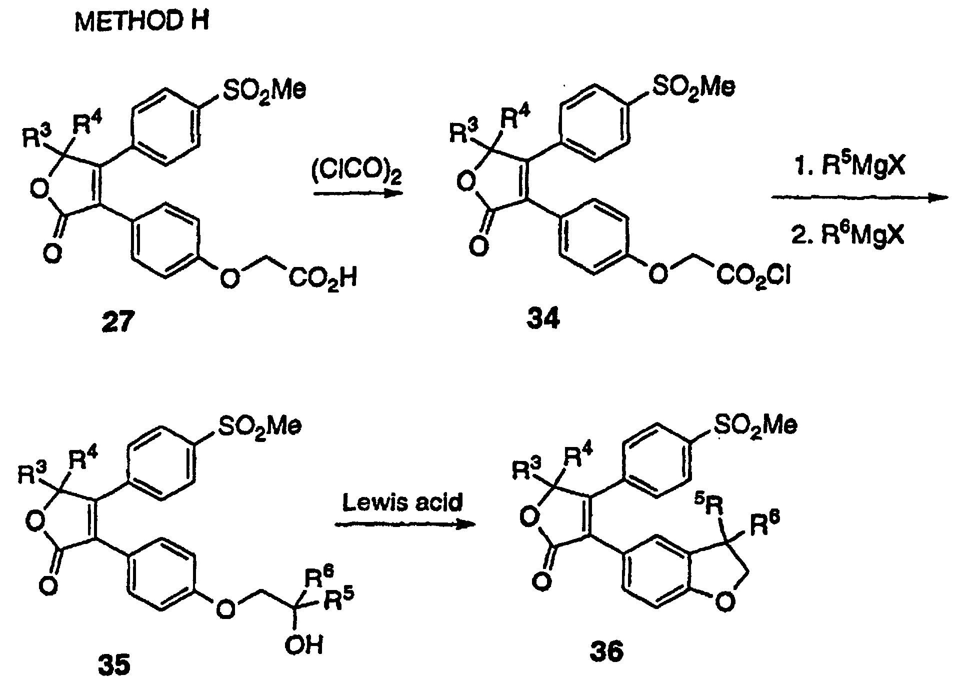

### Method I

Lactol **32** may be treated with Et₃SiH in TFA to provide cyclic ether **36**.

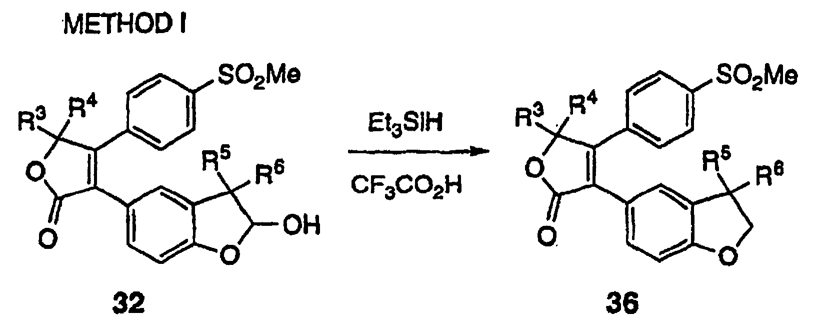

### Method J

Olefin **31** may be hydroborated with (Sia)₂BH followed by peroxide oxidation to give terminal alcohol **37**. Jones oxidation accompanied by lactonization provides lactone **38**.

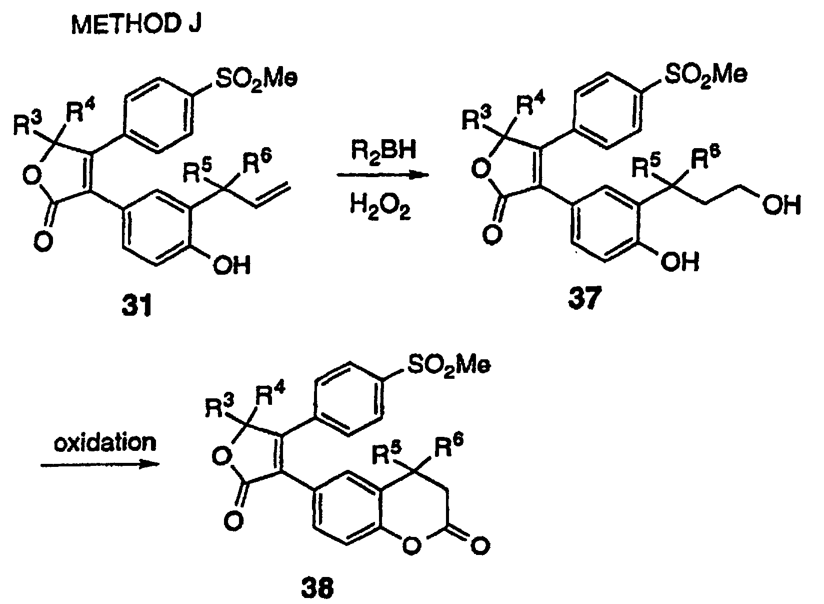

### Method K

A mixture of phenol **25** and an alkyl or benzylic halide R-X in DMF may be treated with NaH to give ether **39.**

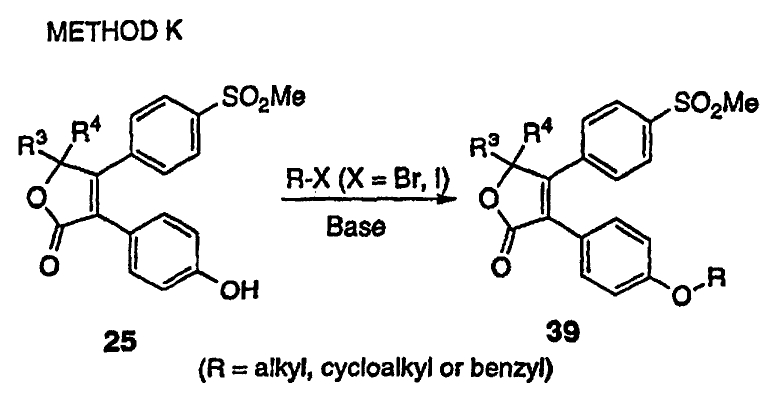

### Method L

An arylhalide or aryl triflate **40** may be treated with a vinyl boronate or a trialkylstannylacetylene in the presence of a Pd° catalyst to provide the corresponding vinylic or acetylenic aromatics **41**.

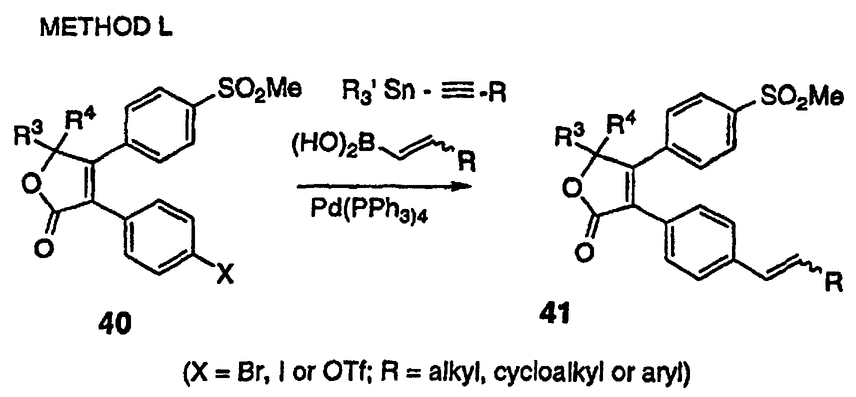

### Method M

δ-Valerolactam may be treated with NaH in DMF in the presence of methyl bromoacetate to give ester **42.** This is hydrolized under basic conditions to give acid **43** which can be coupled with alcohol **5** under DCC/DMAP conditions to give ester **44.** Treatment with NaH gives lactone **45** which, when treated with excess LiAlH₄ gives aminodiol **46.** Oxidation of **46** then provides amino lactone **47.**

The diol **20,** obtained in Method D, is treated with excess Et₂NSF₃ in an inert solvent such as CH₂Cl₂ to provide the desired product **23.**

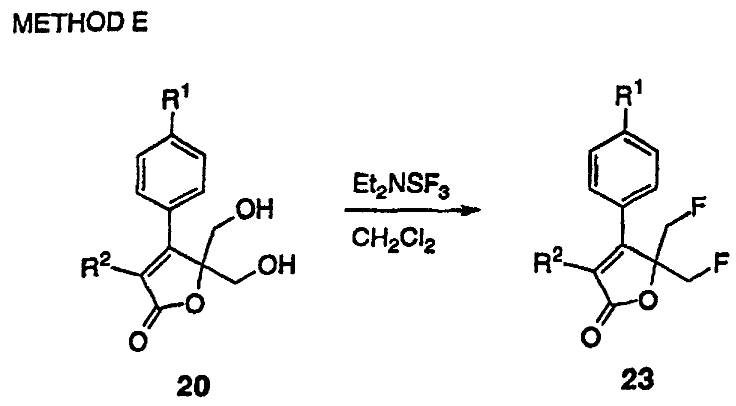

### Method F

Arylmethyl ether **24,** obtained in Method A, may be demethylated by treatment with BBr₃. Treatment of the resulting phenol **26** with NaH in DMF followed by treatment with methyl bromoacetate provides ester **26,** which may be hydrolized under alkaline conditions to provide acid **27.** Friedel-Crafts cyclization of **27** in PPA provides cyclic ketone **28.** This ketone may be alkylated by enolization with LDA followed by quenching with electrophile R⁵-X. This process may be repeated with electrophile R⁶-X to provide a mono- or di-alkylated ketone **29.**

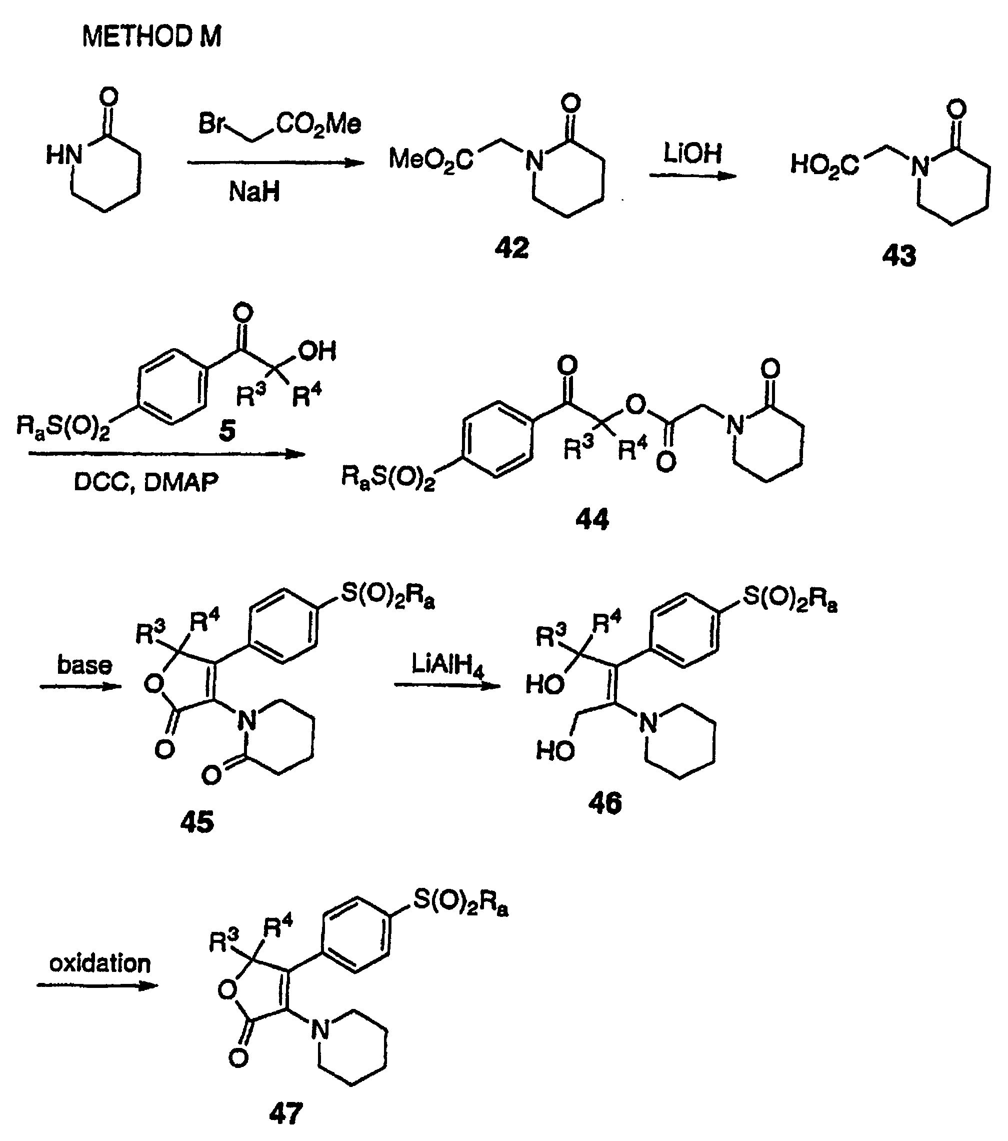

### METHOD N

Esterification of 1-hydroxy-cyclobutanecarboxylic acid with diazomethane provides ester **48** which can be acylated with an appropiately substituted aryl acetic acid chloride in the presence of a base such as pyridine followed by treatment with DBU to afford lactone **49.** Treatment of **49** with trifluoromethanesulfonic anhydride in the presence of a base such as triethylamine gives triflate **50.** Cross-coupling of **50** with 4-(methylthio)benzeneboronic acid under palladium catalysis gives product **51**. By methods known in the art, the -SMe group of **51** can be oxidized to the corresponding sulfone **52** (Rₐ = Me) or it can be converted to the -SO₂NH₂ group.

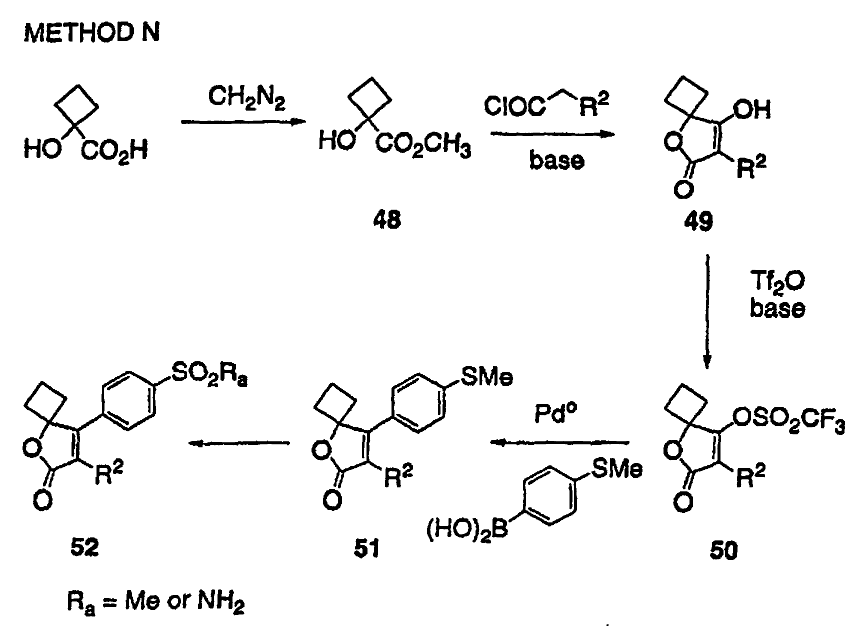

### Representative Compounds

Tables **I** and **II** illustrate novel compounds of the present invention. Tables **III** and **IV** illustrate compounds of formula I, which are representative of the present invention.

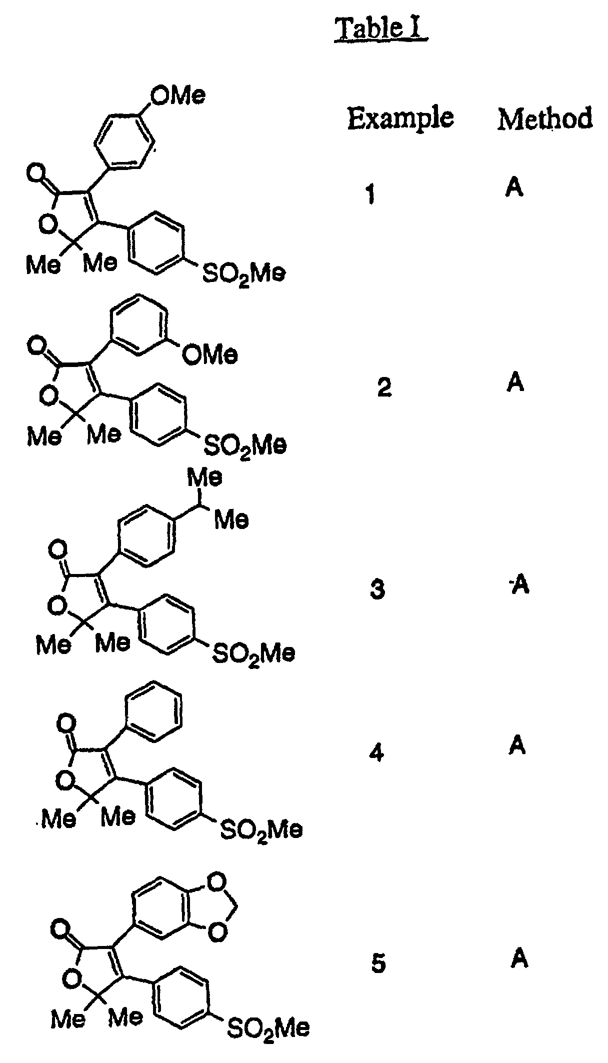

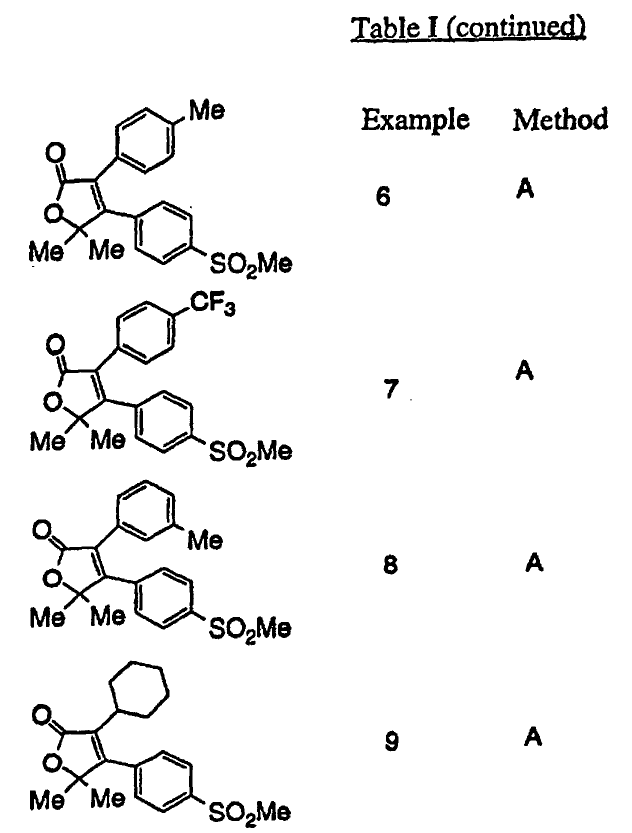

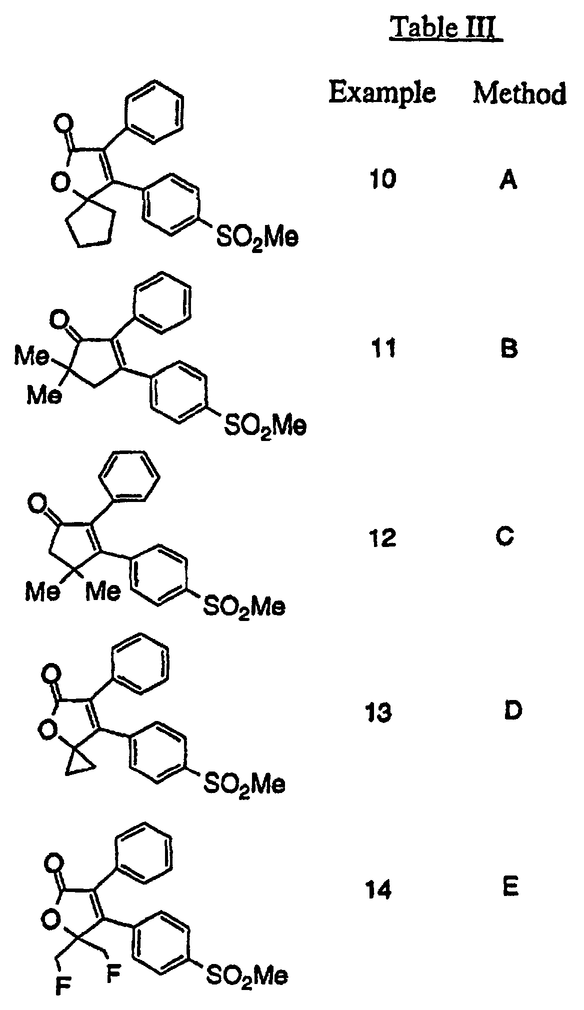

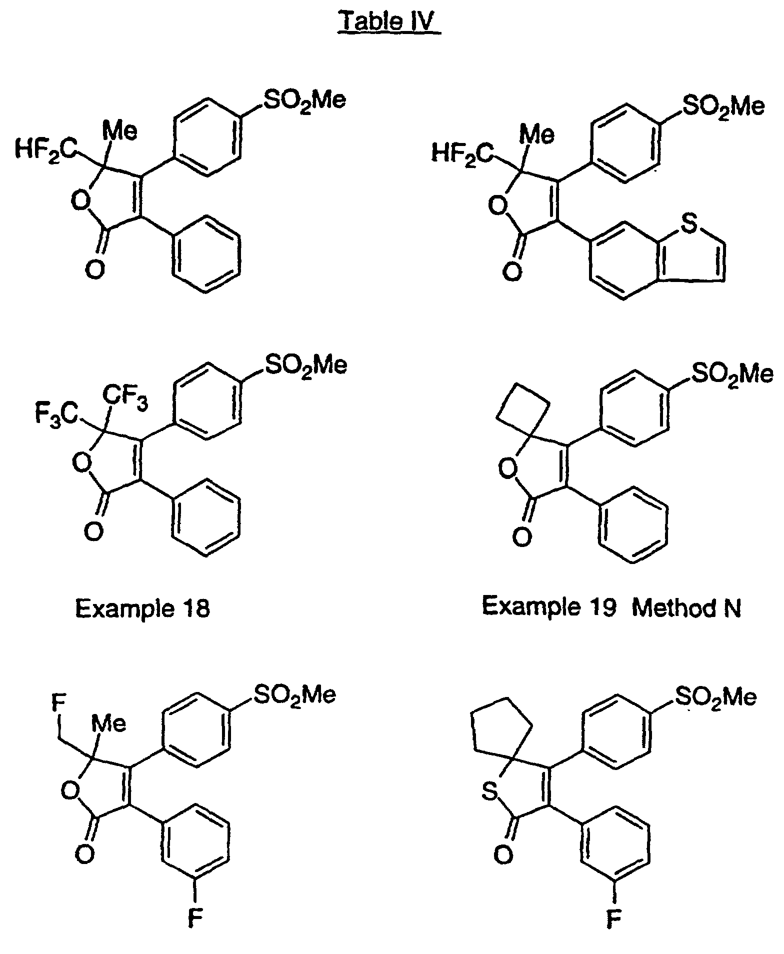

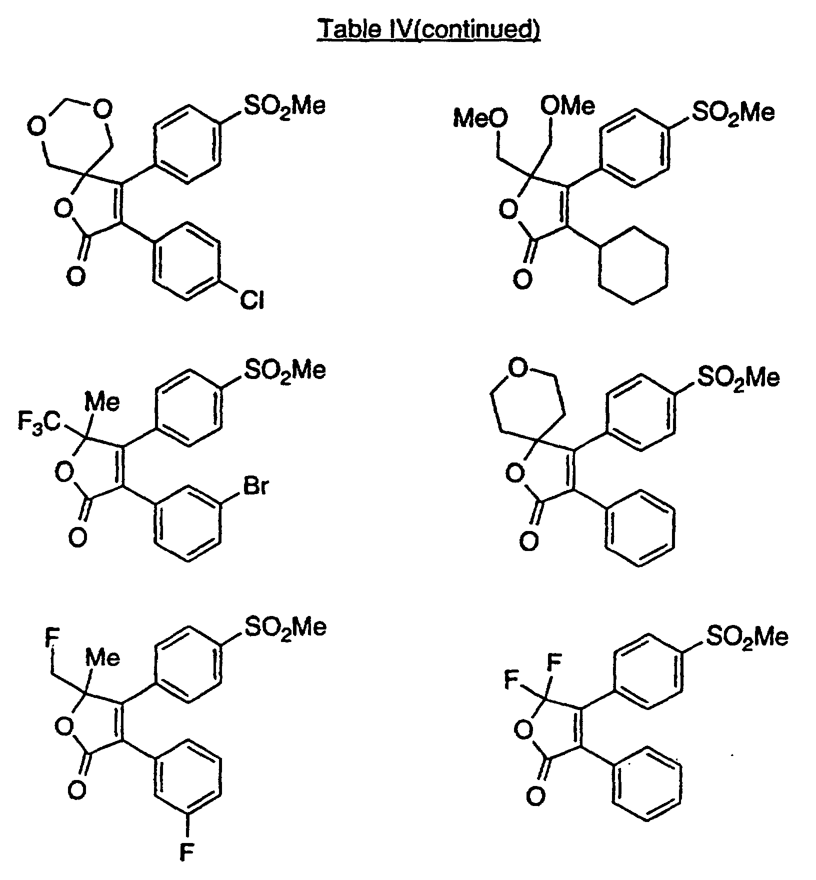

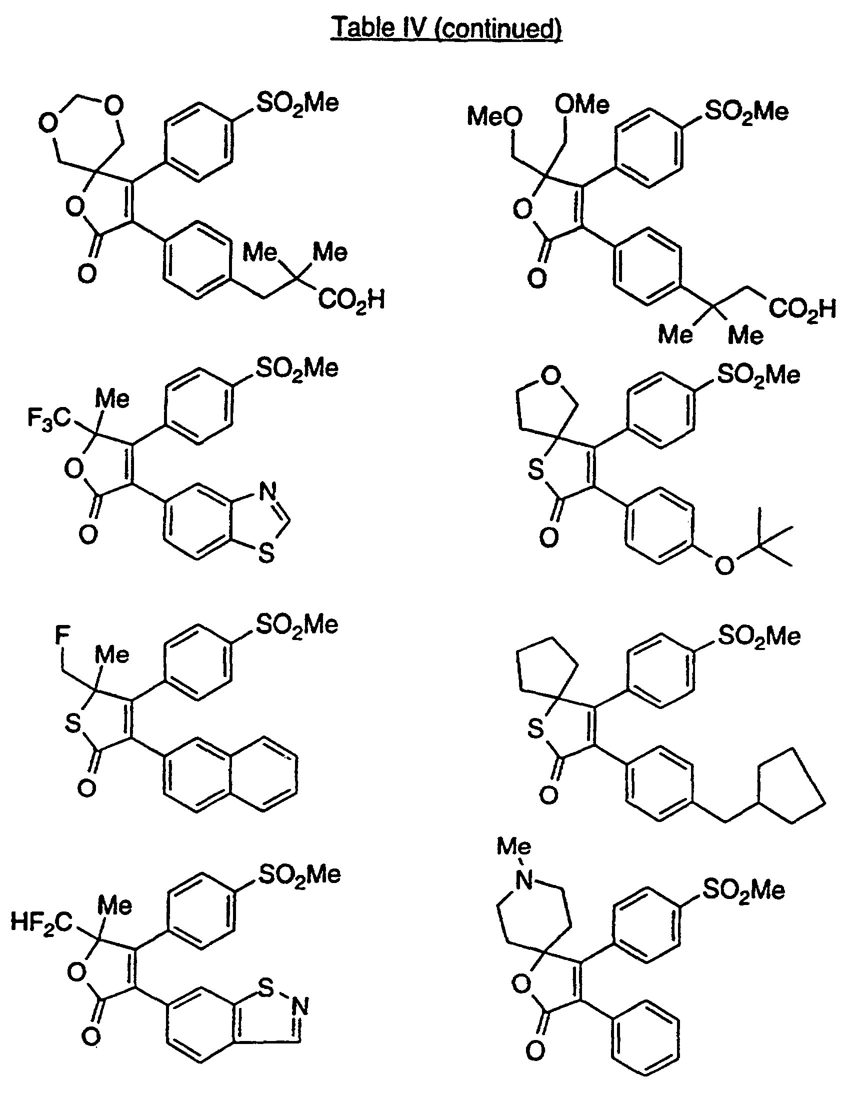

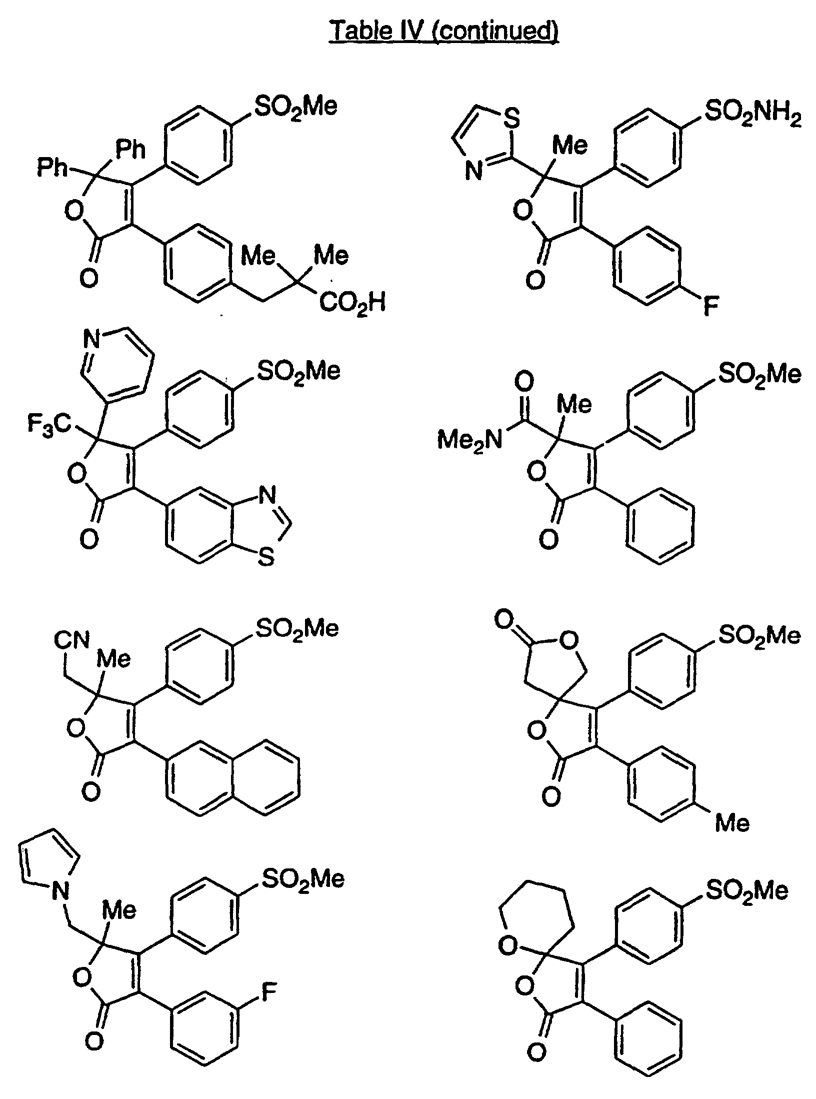

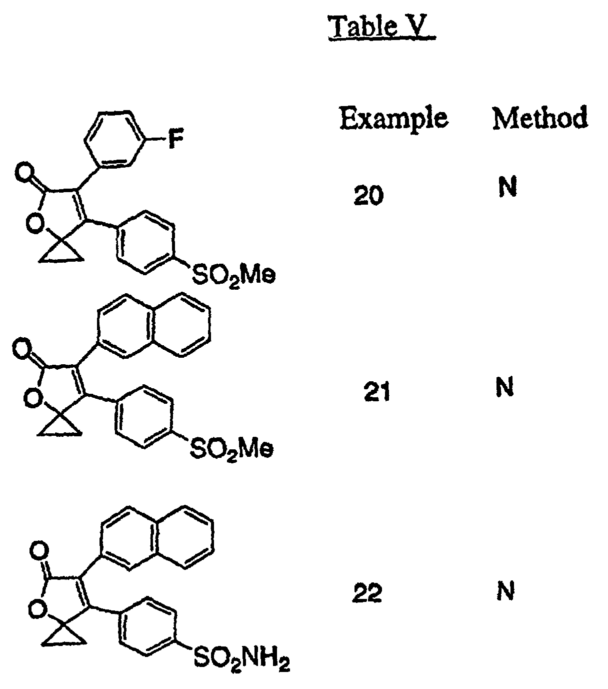

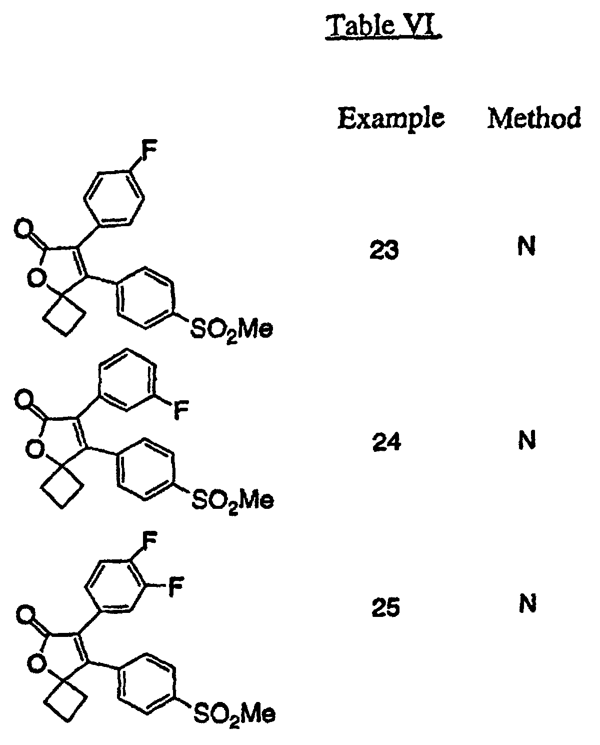

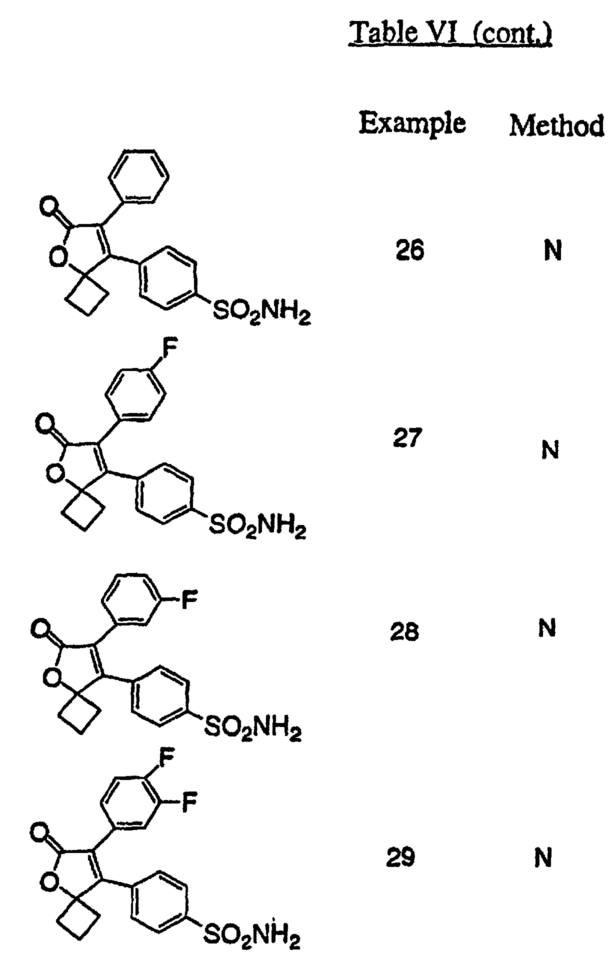

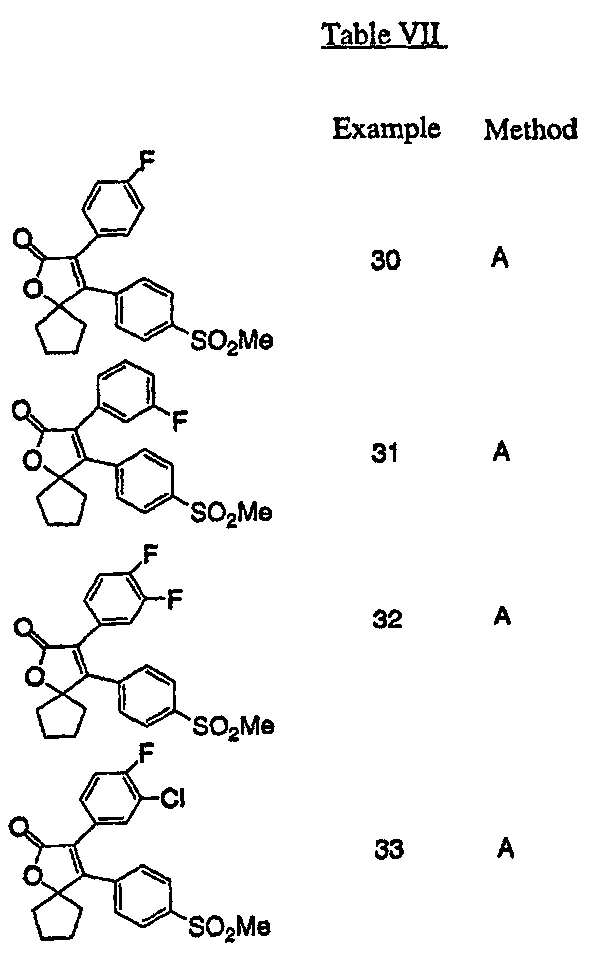

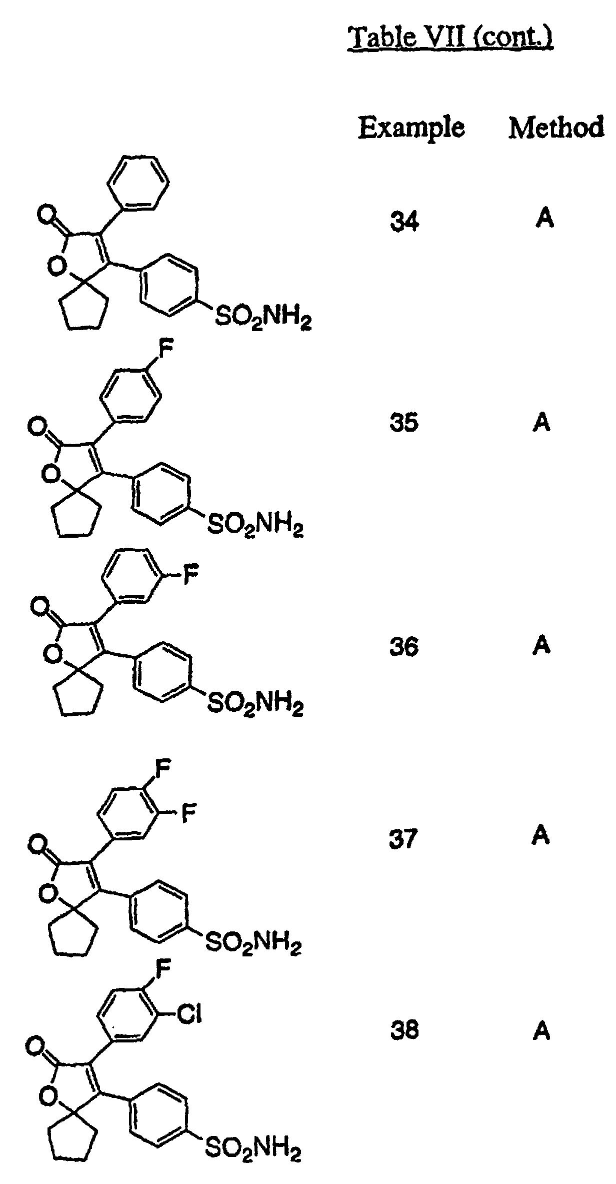

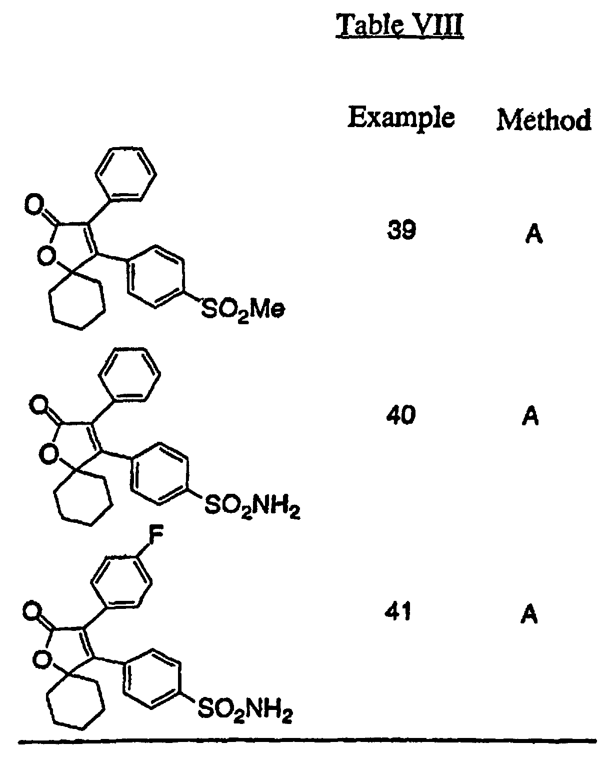

### Assays for determining Biological Activity

The compound of Formula I can be tested using the following assays to determine their cyclooxygenase-2 inhibiting activity.

### Inhibition of Cyclooxygenase Activity

Compounds were tested as inhibitors of cyclooxygenase activity in whole cell cyclooxygenase assays. Both of these assays measured prostaglandin E₂ synthesis in response to arachidonic acid, using a radioimmunoassay. Cells used for these assays were'human osteosarcoma 143 cells (which specifically express cyclooxygenase-2) and human U-937 cells (which specifically express cyclooxygenase-1). In these assays, 100% activity is defined as the difference between prostaglandin E₂ synthesis in the absence and presence of arachidonate.

### Assay

For cyclooxygenase assays, osteosarcoma cells are cultured in 1 mL of media in 24-well multidishes (Nunclon) until confluent (1-2 x 10⁵ cells/well). U-937 cells are grown in spinner flasks and resuspended to a final density of 1.5 x 10⁶ cells/mL in 24-well multidishes (Nunclon). Following washing and resuspension of osteosarcoma and U-937 cells in 1 mL of HBSS, 1 µL of a DMSO solution of test compound or DMSO vehicle is added, and samples gently mixed. All assays are performed in triplicate. Samples are then incubated for 5 or 15 minutes at 37°C, prior to the addition of arachidonic acid. Arachidonic acid (peroxide-free, Cayman Chemical) is prepared as a 10 mM stock solution in ethanol and further diluted 10-fold in HBSS. An aliquot of 10 µL of this diluted solution is added to the cells to give a final arachidonic acid concentration of 10 µM. Control samples are incubated with ethanol vehicle instead of arachidonic acid. Samples are again gently mixed and incubated for a further 10 min at 37°C. For osteosarcoma cells, reactions are then stopped by the addition of 100 µL of 1N HCl with mixing and by the rapid removal of the solution from cell monolayers. For U-937 cells, reactions are stopped by the addition of 100 µL of 1N HCl with mixing. Samples are then neutralized by the addition of 100 µL of 1N NaOH and PGE₂ levels measured by radioimmunoassay.

### Rat Paw Edema Assay - Protocol

Male Sprague-Dawley rats (150 - 200 g) were fasted overnight and were given po either vehicle (1% methocel or 5% Tween 80) or a test compound. One hr later, a line was drawn using a permanent marker at the level above the ankle in one hind paw to define the area of the paw to be monitored. The paw volume (V₀) was measured using a plethysmometer (Ugo-Basile, Italy) based on the principle of water displacement The animals were then injected subplantarly with 50 µl of 1% carrageenan solution in saline (FMC Corp, Maine) into the paw using an insulin syringe with a 25-gauge needle (i.e. 500 µg carrageenan per paw). Three hr later, the paw volume (V₃) was measured and the increases in paw volume (V₃ - V₀) were calculated. The animals were sacrificed by CO₂ aphyxiation and the absence or presence of stomach lesions scored. Data were compared with the vehicle-control values and percent inhibition calculated. Since a maximum of 60-70% inhibition (paw edema) was obtained with standard NSAIDs, ED₃₀ values were used for comparison. All treatment groups were coded to eliminate observer bias.

### NSAID-Induced Gastrophathy in Rats

### Rationale

The major side effect of conventional NSAIDs is their ability to produce gastric lesions in man. This action is believed to be caused by inhibition of COX-1 in the gastrointestinal tract. Rats are particularly sensitive to the actions of NSAIDS. In fact, rat models have been used commonly in the past to evaluate the gastrointestinal side effects of current conventional NSAIDs. In the present assay, NSAID-induced gastrointestinal damage is observed by measuring fecal ⁵¹Cr excretion after systemic injection of ⁵¹Cr-labeled red blood cells. Fecal ⁵¹Cr excretion is a well-established and sensitive technique to detect gastrointestinal integrity in animals and man.

### Methods

Male Sprague Dawley rats (150 - 200 g) are administered orally a test compound either once (acute dosing) or b.i.d. for 5 days (chronic dosing). Immediately after the administration of the last dose, the rats ate injected via a tail vein with 0.5 mL of ⁵¹Cr-labeled red blood cells from a donor rat. The animals are placed individually in metabolism cages with food and water ad *lib.* Feces are collected for a 48 h period and ⁵¹Cr fecal excretion is calculated as a percent of total injected dose.

⁵¹Cr-labeled red blood cells are prepared using the following procedures. Ten mL of blood is collected in heparinized tubes via the vena cava from a donor rat. Plasma is removed by centrifugation and replenished with equal volume of HBSS. The red blood cells are incubated with 400 µCi of sodium ⁵¹chromate for 30 min at 37°C. At the end of the incubation, the red blood cells are washed twice with 20 mL HBSS to remove free sodium ⁵¹chromate. The red blood cells are finally reconstituted in 10 mL HBSS and 0.5 mL of the solution (about 20 µCi) is injected per rat.

### Protein-Losing Gastropathy in Squirrel Monkeys

### Rationale

Protein-losing gastropathy (manifested as appearance of cirulating cells and plasma proteins in the GI tract) is a significant and dose-limiting adverse response to standard NSAIDs. This can be quantitatively assessed by intravenous administration of ⁵¹CrCl₃ solution. This isotopic ion can avidly bind to cell and serum globins and cell endoplasmic reticulum. Measurement of radioactivity appearing in feces collected for 24 h after administration of the isotope thus provides a sensitive and quantitative index of protein-losing gastropathy.

### Methods

Groups of male squirrel monkeys (0.8 to 1.4 kg) are treated by gavage with either 1% methocel or 5% Tween 80 in H₂O vehicles, (3 mL/kg b.i.d.) or test compounds at doses from 1 - 100 mg/kg b.i.d for 5 days. Intravenous ⁵¹Cr (5 µCi/kg in 1 ml/kg PBS) is administered 1 h after the last drug/vehicle dose, and feces collected for 24 h in a metabolism cage and assessed for excreted ⁵¹Cr by gamma-counting. Venous blood is sampled 1 h and 8 h after the last drug dose, and plasma concentrations of drug measured by RP-HPLC.

### Human Whole Blood Assay

### Rationale

Human whole blood provides a protein and cell-rich milieu appropriate for the study of biochemical efficacy of anti-inflammatory compounds such as selective Cox-2 inhibitors. Studies have shown that normal human blood does not contain the Cox-2 enzyme. This is consistent with the observation that Cox-2 inhibitors have no effect on PGE₂ production in normal blood. These inhibitors are active only after incubation of human whole blood with LPS (lipopolysaccharide), which induces Cox-2. This assay can be used to evaluate the inhibitory effect of selective Cox-2 inhibitors on PGE2 production: As well, platelets in whole blood contain a large amount of the Cox-1 enzyme. Immediately following blood clotting, platelets are activated through a thrombin-mediated mechanism. This reaction results in the production of thromboxane B₂ (TxB₂) via activation of Cox-1. Thus, the effect of test compounds on TxB₂ levels levels following blood clotting can be examined and used as an index for Cox-1 activity. Therefore, the degree of selectivity by the test compound can be determined by measuring the levels of PGE₂ after LPS induction (Cox-2) and TxB₂ following blood clotting (Cox-1) in the same assay.

### METHOD

### A. Cox-2 (LPS-induced PGE₂ production)

Fresh blood was collected in heparinized tubes by venipuncture from both male and female volunteers. The subjects had no apparent inflammatory conditions and had not taken any NSAIDs for at least 7 days prior to blood collection. Plasma was immediately obtained from a 2mL blood aliquot to use as blank (basal levels of PGE₂). The remaining blood was incubated with LPS (100 µg/ml final concentration, Sigma Chem, #L-2630 from E. coli; diluted in 0.1% BSA-Phosphate buffered saline) for 5 minutes at room temperature. Five hundred µL aliquots of blood were incubated with either 2 µL vehicle (DMSO) or 2 µL of a test compound at final concentrations varying from 10nM to 30µM for 24 hours at 37 °C. At the end of the incubation, the blood was centrifuged at 12,000 x g for 5 minutes to obtain plasma. A 100 µL aliquot of plasma was mixed with 400 µL of methanol for protein precipitation. The supernatant *was* obtained and was assayed for PGE₂ using a radioimmunoassay kit (Amersham, RPA#530) after conversion of PGE₂ to its methyl oximate derivative according to the manufacturer's procedure.

### B. Cox-1 (Clotting-induced TxB₂ production)

Fresh blood was collected into vacutainers containing no anticoagulants. Aliquots of 500 µL were immediately transferred to siliconized microcentrifuge tubes preloaded with 2 µL of either DMSO or a test compound at final concentrations varying from 10nM to 30 µM. The tubes were vortexed and incubated at 37 °C for 1 hour to allow blood to clot. At the end of incubation, serum was obtained by centrifugation (12,000 x g for 5 min.). A 100 µL aliquot of serum was mixed with 400 µL of methanol for protein precipitation. The supernatant was obtained and was assayed for TxB₂ using a enzyme immunoassay kit (Cayman, #519031) according to the manufacturer's instruction.

### Representative Biological Data

Compounds of the present invention are inhibitors of cyclooxygenase-2 and are thereby useful in the treatment of cyclooxygenase-2 mediated diseases as enumerated above. The activities of the compounds against cyclooxygenase may be seen in the representative results shown below. In the assay, inhibition is determined by measuring the amount of prostaglandin E₂ (PGE₂) synthesized in the presence of arachidonic acid, cyclooxygenase-1 or cyclooxygenase-2 and a putative inhibitor. The IC₅₀ values represent the concentration of putative inhibitor required to return PGE₂ synthesis to 50% of that obtained as compared to the uninhibited control.

The results for inhibition of PGE₂ production may be seen in Table V.

**Table V**

| **Example** | **HWB Cox-2** **IC**_{**50**}**(µM)** | **HWB Cox-1** **IC**_{**50**} **(µM)** | **Rat Paw Edema** **ED**_{**30**} **(mg/kg)** |
|---|---|---|---|
| 2 | 0.81 | >30 | 0.30 |
| 4 | 0.17 | >30 | 0.26 |
| 10 | 0.37 | 30 | 0.41 |
| 11 | 0.06 | 2.2 | 1.17 |
| 13 | 0.60 | >30 | 0.30 |
| 14 | 1.1 | >30 | 0.62 |

### The following abbreviations have the indicated meanings

- Ac =: acetyl
- AIBN =: 2.2--azobisisobutyronitrile
- Bn =: benzyl
- DBU =: diazabicyclo[5.4.0]undec-7-ene
- DCC =: 1,3-dicyclohexylcarbodiimide
- DMAP=: 4-(dimethylamino)pyridine
- DMF =: N,N-dimethylformamide
- DMSO=: dimethyl sulfoxide
- Et₃N =: triethylamine
- Fur =: furandiyl
- HBSS =: Hank's balanced salt solution
- HWB =: human whole blood
- KHMDS=: potassium hexamethyldisilazane
- LDA =: lithium diisopropylamide
- MMPP=: magnesium monoperoxyphthalate
- Ms =: methanesulfonyl = mesyl
- MsO =: methanesulfonate = mesylate
- NBS =: N-bromosuccinimide
- NCS =: N-chlorosuccinimide
- NIS =: N-iodosuccinimide
- NSAID=: non-steroidal anti-inflammatory drug
- PCC =: pyridinium chlorochromate
- PDC =: pyridinium dichromate
- Ph =: phenyl
- Phe =: benzenediyl
- PPA =: polyphosphoric acid
- Pye =: pyridinediyl
- r.t. =: room temperature
- rac. =: racemic
- Tf =: trifluoromethanesulfonyl = triflyl
- TFA =: trifluoroacetic acid
- TfO =: trifluoromethanesulfonate = triflate
- Th =: 2- or 3-thienyl
- THF =: tetrahydrofuran
- Thi =: thiophenediyl
- TLC =: thin layer chromatography
- Ts =: p-toluenesulfonyl = tosyl
- TsO =: p-toluenesulfonate = tosylate
- Tz =: 1H (or 2H)-tetrazol-5-yl
- C₃H₅ =: allyl
- -SO₂Me=: methyl sulfone
- -SO₂NH₂=: sulfonamide

### Alkyl group abbreviations

- Me =: methyl
- Et =: ethyl
- n-Pr =: normal propyl
- i-Pr =: isopropyl
- n-Bu =: normal butyl
- i-Bu =: isobutyl
- s-Bu =: secondary butyl
- t-Bu =: tertiary butyl
- c-Pr =: cyclopropyl
- c-Bu =: cyclobutyl
- c-Pen =: cyclopentyl
- c-Hex =: cyclohexyl

The invention will now be illustrated by the following nonlimiting examples in which, unless stated otherwise:
(i) all operations were carried out at room or ambient temperature, that is, at a temperature in the range 18-25°C;
(ii) evaporation of solvent was carried out using a rotary evaporator under reduced pressure (600-4000 pascals: 4.5-30 mm Hg) with a bath temperature of up to 60°C;
(iii) he course of reactions was followed by thin layer chromatography (TLC) and reaction times are given for illustration only; (iv) melting points are uncorrected and 'd' indicates decomposition; the melting points given are those obtained for the materials prepared as described; polymorphism may result in isolation of materials with different melting points in some preparations;
(v) the structure and purity of all final products were assured by at least one of the following techniques: TLC, mass spectrometry, nuclear magnetic resonance (NMR) spectrometry or microanalytical data;
(vi) yields are given for illustration only;
(vii) when given, NMR data is in the form of delta (δ) values for major diagnostic protons, given in parts per million (ppm) relative to tetramethylsilane (TMS) as internal standard, determined at 300 MHz or 400 MHz using the indicated solvent; conventional abbreviations used for signal shape are: s. singlet; d. doublet; t. triplet; m. multiplet; br. broad; etc.: in addition "Ar" signifies an aromatic signal;
(viii) chemical symbols have their usual meanings; the following abbreviations have also been used v (volume), w (weight), b.p. (boiling point), m.p. (melting point), L (liter(s)), mL (milliliters), g (gram(s)), mg (milligrams(s)), mol (moles), mmol (millimoles), eq (equivalent(s)).

### EXAMPLE 1

### 5,5-Dimethyl-4-(4-(methylsulfonyl)phenyl)-3-(4-methoxyphenyl)-2-(5H)-furanone

### Step 1: 2-Methyl-1-(4-(methylthio)phenyl)-propan-1-one

To a suspension of aluminum chloride (136 g, 1.02 mol) in chloroform (1.0 L) cooled to -10 °C, was added dropwise isobutyrylchloride (115 mL, 1.10 mol). Then thioanisole (100 mL, 0.85 mol) was added dropwise. Upon completion of addition the reaction was allowed to proceed at r.t. for 1.5h. The reaction was cooled to 10 °C and quenched by addition of water (750 mL). The organic layer was separated, washed with water (2 x 500 mL), saturated NaHCO₃ solution(2 x 500 mL), brine (1 x 500 mL), and then dried over Na₂SO₄. After concentration *in vacuo*., the resulting crude product crystallized upon standing under high vacuum for 30 min to give the title compound as a brown solid.

### Step 2: 2-Hydroxy-2-methyl-1-(4-(methylthio)phenyl)-propan-1-one

To a solution of 2-methyl-1-(4-(methylthio)phenyl)-propan-1-one (28.5 g, 147 mmol, Step 1), Aliquat 336 (11.0 mL, 24 mmol) and carbon tetrachloride (21 mL, 218 mmol) in toluene (43 mL) was added sodium hydroxide (12.9 g, pellets, 322 mmol). The reaction was stirred at 15 °C for 2h and then at r.t. for 16h. The reaction was diluted with water (100 mL), brine (100 mL) and EtOAc (300 mL). The aqueous phase was acidified with 1 N HCl and extracted with EtOAc (100 mL). The combined organic layers were dried over Na₂SO₄ and concentrated. The crude product was purified by silica gel chromatography eluted with 15% EtOAc in hexane to give the title compound as a thick syrup.

### Step 3: 2-Hydroxy-2-methyl-1-(4-methylsulfonylphenyl)-propan-1-one

To a cold (4 °C) solution of 2-hydroxy-2-methyl-1-(4-(methylthio)phenyl)-propan-1-one (45.0 g, 214 mmol, Step 2) in t-butanol (500 mL) and CH₂Cl₂ (500 mL) was added a solution of OXONE™ (194 g, 316 mmol) in water (1.4 L). The resulting suspension was stirred at r.t. for 18h. The reaction was diluted with EtOAc (400 mL) and the layers were separated. The aqueous layer was extracted with EtOAc (2 x 250 mL). The combined organic layers were dried over Na₂SO₄ and concentrated *in vacuo*. The crude product was dissolved in diethyl ether (250 mL), hexane was added (150 mL) and the product was swished for 2h. The product was collected by filtration to give the title compound as a yellow solid.

### Step 4: 5,5-Dimethyl-4-(4-(methylsulfonyl)phenyl)-3-(4-methoxyphenyl)-2-(5H)-furanone

To a cold solution (0 °C) of 2-hydroxy-2-methyl-1-(4-(methylsulfonyl)phenyl)-propane-1-one (726 mg, 3.5 mmol, Step 3), pyridine (0.5 mL, 6.2 mmol) in CH₂Cl₂ (10 mL) was added 4-methoxyphenylacetyl chloride (1.5 g, 8.4 mmol) over 2 minutes. The resulting yellow solution was stirred at r.t. for 18h. Then DBU was added in portions every 30 min. (3 x 1.0 mL, 3 x 6.7 mmol). The reaction was diluted with EtOAc (100 mL) and 1 N HCl (50 mL). The organic layer was washed with water (50 mL), dried over MgSO₄ and concentrated. The crude product was purified first by flash chromatography eluted with 40% EtOAc in hexane and then by crystallization in EtOAc and hexane (1:1, 26 mL) to give the title compound as a white solid.
¹H NMR (400 MHz, CD₃COCD₃) 1.60 (s, 6H), 3.18 (s, 3H), 3.76 (s, 3H), 6.83 (dt, 2H), 7.32 (dt, 2H), 7.65 (dt, 2H), 8.05 (dt, 2H), m.p. 148 °C.

### EXAMPLE 2

### 5,5-Dimethyl-3-(3-methoxyphenyl)-4-(4-(methylsulfonyl)phenyl)-2-(5H)-furanone

¹H NMR (d₆-acetone, 400 MHz): δ 1.62 (6H, s), 3.17 (3H, s), 3.61 (3H, s), 6.85 (1H, dt), 6.91 (1H, dd), 6.96 (1H, dt), 7.18 (1H, t), 7.68 (2H, dm), 8.05 (2H, dm).

### EXAMPLE 3

### 5,5-Dimethyl-3-(4-isopropylphenyl)-4-(4-(methylsulfonyl)phenyl)-2-(5H)-furanone

¹H NMR (d₆-acetone, 400 MHz): δ 1.17 (6H, d), 1.60 (6H, s), 2.90 (1H, m), 3.18 (3H, s), 7.15 (2H, d), 7.32 (2H, d), 7.67 (2H, d), 8.05 (2H, d).

### EXAMPLE 4

### 5,5-Dimethyl-4-(4-(methylsulfonyl)phenyl)-3-phenyl-2-(5H)-furanone

| | | | |
|---|---|---|---|
| Analysis | calculated for C₁₉H₁₈O₄S | | |
| | C, 66.65; | H, 5.30; | S, 9.36 |
| Found | C, 66.36; | H, 5.32; | S, 9.48 |

m.p. 159°C

### EXAMPLE 5

### 3-Benzo[1,3]-dioxol-5-yl-5,5-dimethyl-4-(4-(methylsulfonyl)phenyl)-2-(5H)-furanone

| | | |
|---|---|---|
| Analysis | calculated for C₂₀H₁₈O₆S | |
| | C, 62.17; | H, 4.70 |
| Found | C, 62.52; | H, 4.82 |

### EXAMPLE 6

### 5,5-Dimethyl-3-(4-methylphenyl)-4-(4-methylsulfonyl)phenyl)-2-(5H)-furanone

m.p. 108-109 °C

| | |
|---|---|
| M.S. (DCI, CH₄) | calculated for M⁺: 356 |
| | Found for M⁺+1: 357 |

### EXAMPLE 7

### 5,5-Dimethyl-4-(4-(methylsulfonyl)phenyl)-3-(4-trifluoromethylphenyl)-2-(5H)-furanone

m.p. 155-156 °C

### EXAMPLE 8

### 5,5-Dimethyl-3-(3-methylphenyl)-4-(4-(methylsulfonyl)phenyl)-2-(5H)- furanone

m.p. 167-168 °C

### EXAMPLE 9

### 5,5-Dimethyl-3-cyclohexyl-4-(4-(methylsulfonyl)phenyl)-2-(5H)-furanone

### Step 1 Cyclohexylacetyl chloride

To a 0 °C solution of cyclohexaneacetic acid (2.12g, 14.9 mmol) in 30 mL of CH₂Cl₂ was added two drops of DMF followed by oxalyl chloride (1.3 mL, 14.9 mmol). The solution was warned to r.t. and stirred overnight. The solvent was remove *in vacuo* to give 1.60 g of the title compound as a colourless liquid.

¹H NMR (d₆-acetone, 300 MHz): 2.78 (d, 2H), 1.92 (m, 1H), 1.85-1.60 (m, 5H), 1.40-0.95 (m, 5H).

### Step 2 Cyclohexylacetic acid, 1,1-dimethyl-2-((4-methylsulfonyl)-phenyl)-2-oxo-ethyl ester

To a 0 °C solution of the alcohol from Example 1, Step 3 in 15 mL CH₂Cl₂ was added pyridine (0.35 mL, 4.3 mmol). A solution of cyclohexylacetyl chloride (473 mg, 1.95 mmol) in 5 mL CH₂Cl₂ was then added via cannula. A small amount of DMAP was added, and the solution was stirred overnight at r.t. The mixture was diluted with CH₂Cl₂ and washed successively with 1M NaOH, 1M HCl and brine, filtered through cotton and evaporated. Purification by chromatogrphy (30% EtOAc/ hexanes) provided 504 mg of the title ester.

¹H NMR (d₆-acetone, 300 MHz) : 8.20 (m, 2H), 8.04 (m, 2H), 3.14 (s, 3H), 2.12 (d, 2H), 1.70 (s, 6H), 1.57 (m, 3H), 1.40 (m, 2H), 1.12 (m, 4H), 0.80 (m, 2H).

### Step 3: 5,5-Dimethyl-3-cyclohexyl-4-(4-(methylsulfonyl)phenyl)-2-(5H)-furanone

To a 0 °C solution of 500 mg (1.36 mmol) of cyclohexylacetic acid, 1,1-dimethyl-2-((4-methylsulfonyl)-phenyl)-2-oxo-ethyl ester in 25 ml DMF was added 157 mg (3.92 mmol) of hexane-washed 60% NaH dispersion. Stirred 5h, allowing the solution to warm to room temperature, then quenched with 1M HCl. Partitioned between 1:1 EtOAc/ether and H₂O. The organic layer was washed with H₂O, brine and dried over Na₂SO₄. Purification by chromatography (40→50% EtOAc/hexanes) gave 160 mg (34%) of the title compound as an oil.

¹H NMR (d₆-acetone, 300 MHz): 8.09 (m, 2H), 7.62 (m, 2H), 3.20 (s, 3H), 2.18 (m, 1H), 1.75-1.55 (m, 6H), 1.47 (s, 6H), 1.30-1.05 (m, 4H).

### EXAMPLE 10

### 4-(4-(Methylsulfonyl)phenyl)-3-phenyl-1-oxa-spiro[4,4]non-3-en-2-one

### Step 1: Cyclopentyl-(4-(methylthio)phenyl)-methanone

To a suspension of anhydrous aluminum chloride (9.3 g, 69.6 mmol) in 58 mL CHCl₃ at 0 °C was added dropwise cyclopentanecarbonyl chloride (10.0 g, 75.4 mmol), followed by thioanisole (7.21 g, 58.0 mmol). The ice bath was removed and the mixture was stirred at room temperature for 2h. Water (200 ml) was added with cooling, the layers were separated and the aqueous layer was extracted with CHCl₃ (3 x 50 mL). The combined aqueous layers were dried over MgSO₄, filtered and concentrated. The residue was chromatographed on silica gel (4% EtOAc/hexane) to give 11.9 g of the title ketone (93%).
¹H NMR (acetone-d₆, 400 MHz) 7.94 (d, 2H, J = 8.7 Hz), 7.36 (d, 2H, J = 8.7 Hz), 3.79 (q, 1H), 2.56 (s, 3H), 2.00-1.71 (m, 4H), 1.70-1.50 (m, 4H).

### Step 2: (1-Hydroxycyclopentyl)-(4-(methylthio)phenyl)-methanone

To a solution of the ketone from Step 1 (7.2 g, 32.7 mmol) in 4.7 ml CCl₄ and 9.6 ml toluene was added Aliquat 336 (2.11 g, 5.20 mmol) and powdered NaOH (2.88 g, 71.9 mmol) and the mixture was stirred for 16h at r.t. To the brown mixture was added 100 ml of 5% aq. HCl and extracted with EtOAc (4 x 100 ml). The combined organic layers were washed with brine, dried over MgSO₄, filtered and concentrated. Chromatography on silica gel (20% EtOAc/hexane) gave 5.4 g of the title compound as a white waxy solid (70%).
¹H NMR (acetone-d₆, 400 MHz) 8.11 (d, 2H, J = 8.5 Hz), 7.31 (d, 2H, J = 8.5 Hz), 4.63 (s, 1H, disappears by D₂O wash), 2.56 (s, 3H), 2.24 (mc, 2H), 1.89 (mc, 4H), 1.71 (mc, 2H).

### Step 3: 4-(4-(Methylthio)phenyl)-3-phenyl-1-oxa-spiro[4.4]non-3-en-2-one

To the alcohol from Step 2 (924 mg, 3.91 mmol) in 10 mL CH₂Cl₂ at 0 °C was added pyridine (928 mg, 11.7 mmol) followed by phenylacetyl chloride (1.51 g, 9.77 mmol). The ice bath was removed and the mixture was stirred at room temperature for 15h. Aq. NH₄CI (100 mL) was added and the mixture was extracted with EtOAc (3 x 50 mL). The combined organic layers were washed with brine, dried over MgSO₄, filtered and concentrated. The residue was dissolved in 16 mL CH₂Cl₂, DBU (595 mg, 3.91 mmol) was added and the brown solution was stirred at room temperature for 0.5h. 5% aq. HCl (100 mL) was added and the mixture was extracted with EtOAc (4 x 50 mL). The combined organic layers were washed with brine, dried over MgSO₄, filtered and concentrated to give 1.40 g (quantitative) of the title compound as a yellow solid, used directly in the next step. An analytical sample had mp. 147-148 °C after stirring with Et₂O/hexane (2:1).

### Step 4: 4-(4-(Methylsulfonyl)phenyl)-3-phenyl-1-oxa-spiro[4.4]non-3-en-2-one

The thioether from Step 3 (1.31 g, 3.91 mmol) in 40 mL CH₂Cl₂ and 10 mL MeOH was cooled in an ice bath. MMPP (2.66 g, 4.30 mmol) was added in two portions. The ice bath was removed and the mixture was stirred for 1h at room temperature. The suspension was filtered, and the filtrate was washed with saturated aq. NaHCO₃, and H₂O. After drying over MgSO₄ and evaporation of the solvent, a yellow solid was obtained. Stirring this solid with 10 mL of Et₂O for 0.5h and filtration gave 1.48 g of the title compound as a colorless solid, m.p. 142-143 °C.

### EXAMPLE 11

### 5,5-Dimethyl-3-(4-(methylsulfonyl)phenyl)-2-phenylcyclopent-2-enone

### Step 1 4,4-Dimethyl-2-iodo-2-cyclopenten-1-one

To a cold (4 °C) solution of iodine (4.5 g, 18 mmol) in carbon tetrachloride (15 mL) and pyridine (15 mL), was added over 1 min. a solution of 4,4-dimethyl-2-cyclopenten-1-one (930 mg, 8.4 mmol) in carbon tetrachloride (15 mL) and pyridine (15 mL). The resulting red-brown solution was stirred at r.t. for 22h. The reaction was diluted with Et₂O (200 mL), washed successively with water (50 mL), 1.0 N HCl (50 mL), water (50 mL), and aqueous Na₂S₂O₅ 20% w/v, then dried over MgSO₄ and concentrated *in vacuo*. The crude product was purified by flash chromatography eluting with 10% EtOAc/hexanes to give the title compound as a white solid.

### Step 2 4,4-Dimethyl-2-phenyl-2-cyclopenten-1-one

A 250 mL round bottom flask was charged with 4,4-dimethyl-2-iodo-2-cyclopenten-1-one from Step 1 (1.59 g, 6.7 mmol), bis(benzontrile)-palladium(II) chloride (235 mg, 0.61 mmol), phenylboronic acid (983 mg, 8.1 mmol), triphenylarsine (308 mg, 1.0 mmol), 2.0 M Na₂CO₃ (8.4 mL, 16.8 mmol) and benzene (70 mL). The reaction flask was well purged with nitrogen and the reaction was stirred at 80 °C for 2.5h. The reaction was allowed to cool to r.t., diluted with NH₄OAc buffer 25% w/v (100 mL) and extracted with EtOAc. The organic extract was dried over MgSO₄ and concentrated *in vacuo*. The crude product was purified by flash chromatography eluting with 10% EtOAc in hexane to give the title compound as a light yellow syrup.

### Step 3 4,4-Dimethyl-1-(4-(methylthio)phenyl)-2-phenylcyclopent-2-enol

To a solution of 4-bromothioanisole (2.51 g, 12.3 mmol) in THF (20 mL) at -78 °C was added dropwise a solution of 2.5 M n-butyl lithium in hexane (4.8 mL, 12.0 mmol). The resulting suspension was stirred at this temperature for 1h and then a solution of 4,4-dimethyl-2-phenyl-2-cyclopenten-1-one from Step 2 (1.25 g, 6.7 mmol) in THF (20 mL) was added slowly *via* cannula to the cold reaction. The reaction was stirred for 15 min at -78 °C and then for 30 min. at r.t. The reaction was stopped by dilution with NH₄OAc buffer 25% w/v and extracted with EtOAc. The organic layer was dried over MgSO₄ and concentrated *in vacuo*. The crude product was purified by flash chromatography eluting with 10% EtOAc/hexanes to give the title compound as a colorless gum.

### Step 4 5,5-Dimethyl-3-(4-(methylthio)phenyl)-2-phenylcyclopent-2-enone

To a suspension of 4,4-dimethyl-1-(4-(methylthio)phenyl)-2-phenylcyclopent-2-enol from Step 3 (2.0 g, 6.4 mmol) and powdered 4Å molecular sieves (2 g) in CH₂Cl₂ (50 mL) was added pyridinium chlorochromate (1.6 g, 7.4 mmol). The resulting suspension was vigorously stirred for 18h. Then powdered 4Å molecular sieves (2 g) and PCC (1.6 g, 7.4 mmol) were added and the resullting brown suspension was stirred for another 24h. The reaction was diluted with CH₂Cl₂ (100 mL) followed by Et₂O (600 mL) with vigorous stirring for 24h. The reaction was filtered on a cake of silica gel which was washed with Et₂O (2 x 100 mL) and the filtrate was concentrated *in vacuo*. The crude product was purified by flash chromatography eluted with 2% EtOAc in toluene to give the title compound as a light yellow gum.

### Step 5 5,5-Dimethyl-3-(4-(methylsulfonyl)phenyl)-2-phenylocyclopent-2-enone

To a cold solution (4 °C) of 5,5-dimethyl-3-(4-(methylthio)phenyl)-2-phenylcyclopent-2-enone from Step 4 (563 mg, 1.8 mmol) in CH₂Cl₂ (16 mL) and in methanol (2 mL) was added monoperoxyphthalic acid, magnesium salt hexahydrate 80% (1.47 g, 2.4 mmol). The resulting suspension was stirred at r.t. for 18h. The reaction was diluted with EtOAc (150 mL) and a mixture of aq. NaHCO₃ sat./water (1:1). The organic layer was dried over MgSO₄ and concentrated *in vacuo.* The crude product was purified first by flash chromatography eluted with 40% of EtOAc/hexanes and then by crystallization in EtOAc/hexanes (1:3, 30 mL) to give the title compound as light yellow needles.
¹H NMR (400 MHz, CD₃COCD₃) 1.26 (s, 6H), 3.05 (s, 2H), 3.12 (s, 3H), 7.20 (m, 2H), 7.33 (m, 3H), 7.63 (dt, 2H), 7.89 (dt, 2H), m.p. 117°C.

### EXAMPLE 12

### 4,4-Dimethyl-3-(4-(methylsulfonyl)phenyl)-2-phenylcyclopent-2-enone

### Step 1: 3,3-Dimethyl-1-phenylhex-5-en-2-ol

To a solution of 2,2-dimethylpent-4-enal (11.2 g) in 200 mL of Et₂O cooled to 0 °C was added dropwise a solution of benzylmagnesium chloride (70 mL, 2M in THF). After stirring for 1h at 0 °C, the reaction was quenched with 200 mL of saturated solution of NH₄Cl and the product was extracted with 200 mL of 1:1 EtOAc/hexane. The extract was dried over Na₂SO₄ and concentrated. The residue was distilled under reduced pressure to give 16 g of the title compound.

### Step 2: 3,3-Dimethyl-1-phenylhex-5-en-2-one

To a solution of the product of Step 1 (15 g) and 50 mL of *i*-Pr₂NEt in 250 mL of CH₂Cl₂ cooled at 0 °C was added dropwise a solution of sulfur trioxide pyridine complex (23.8 g) in 100 mL of DMSO. After stirring for 10 min, the reaction mixture was treated with 200 mL H₂O and extracted with 200 mL of 1:1 EtOAc/hexane. The extract was dried over MgSO₄ and concentrated to give 15 g of the title compound as a yellow oil.
¹H NMR (acetone-d₆, 400 MHz) 1.17 (6H, s), 2.36 (2H, dd), 3.87 (2H, s), 5.0-5.15 (2H, m), 5.65-5.80 (1H, m), 7.12-7.30 (5H, m).

### Step 3: 5,5-Dimethyl-2-phenylcyclopent-2-enone

A solution of the product of Step 2 (15 g) in 70 mL of MeOH and 50 mL of CH₂Cl₂ was treated with a stream of ozone/oxygen until the solution turned light blue. Dimethylsulfide (20 mL) was added and the reaction mixture was stirred for 2h at room temperature and then treated with 5 g of triphenylphosphine for an additional 0.5h. DBU (1 mL) was then added to the reaction mixture. After stirring for 1h at room temperature, 1 mL of AcOH was added and the reaction mixture was concentrated. The residue was dissolved in 100 mL of 20:1 hexanes/EtOAc and the solution was filtered through a pad of silica gel. The filtrate was concentrated to give 11 g of the title compound as a yellow oil.
¹H NMR (acetone-d₆,400 MHz) 1.15 (6H, s), 2.60 (2H, d), 7.25-7.40 (5H, m), 7.95 (1H, t).

### Step 4: 5,5-Dimethyl-1-(4-(methylthio)phenyl)-2-phenylcyclopent-2-enol

To a -78 °C solution of p-bromothioanisole (5 g) in 100 mL of THF was added a solution n-BuLi (10 mL, 2.5 M in hexane). After stirring for 45 min. at -78 °C, a solution of the product of Step 3 (2.4 g) in 10 mL of THF was added dropwise. The reaction mixture was stirred for 10 min. and then quenched with 100 mL of saturated solution of NH₄Cl. The product was extracted with 300 mL of 2:1 hexanes/EtOAc. The extract was dried over Na₂SO₄ and concentrated. The residue was purified by chromatography eluting with 10:1 hexane/EtOAc to give 3.5 g of the title compound.
¹H NMR (acetone-d₆, 400 MHz) 0.60 (3H, s), 1.25 (3H, s), 2.26 (1H, dd), 2.42 (1H, dd), 2.48 (3H, s), 4.12 (1H, s), 6.43 (1H, t), 7.10-7.40 (9H, m).

### Step 5: 5,5 Dimethyl-1-(4-(methylsulfonyl)phenyl)-2-phenylcyclopent-2-enol

To a solution of the product of Step 4 (1.92 g) in 130 mL of 10:1 CH₂Cl₂/MeOH cooled at 0 °C was added 5.5 g of MMPP. The reaction was stirred for 0.5h at room temperature, diluted with 100 mL of 5:1 hexanes/EtOAc and then filtered through a pad of silica gel. The filtrate was concentrated and the residue was purified by chromatography eluting with 2:1 hexanes/EtOAc to give 1.8 g of the title compound.
¹H NMR (acetone-d₆, 400 MHz,) δ 0.58 (3H, s), 1.27 (3H, s), 2.32 (1H, dd), 2.52 (1H, dd), 3.09 (3H, s), 4.46 (1H, s), 6.53 (1H, t), 7.12 -7.20 (5H, m), 7.37 (2H, d), 7.85 (2H, d).

### Step 6: 4,4-Dimethyl-3-(4-(methylsulfonyl)pheny)-2-phenyl-2-cyclopenten-1-one

To a suspension of PCC (4 g) and 10 g of 4Å molecular seives in 100 mL of CH₂Cl₂ was added a solution of the product of Step 5 (1.2 g) in 10 mL CH₂Cl₂. The reaction mixture was stirred for 12h and then diluted with 200 mL of 1:1 hexanes/EtOAc, and filtered through a pad of silica gel. The filtrate was concentrated and the residue was purified by chromatography eluting with 3:2 hexanes/EtOAc to give 200 mg of the title compound as a white solid.
¹H NMR (acetone-d₆, 400 MHz) δ1.36 (6H s), 2.62 (2H, s), 3.14 (3H, s), 7.10-7.20 (5H, m),7.53 (2H, d), 7.96 (2H, d).

### EXAMPLE 13

### 7-(4-(Methylsulfonyl)phenyl)-6-phenyl-4-oxa-spiro[2,4]-hept-6-en-5-one

### Step 1: 3-(Phenyl)-4-(4-(methylsulfonyl)phenyl)-2-(5H)-furanone

To a solution of phenylacetic acid (27.4 g, 201 mmol) and α-bromo-4-(methylsulfonyl)acetophenone (60 g, 216 mmol) in acetonitrile (630 mL) at 25 °C was added slowly triethylamine (30.8 mL). The mixture was stirred for 20 min at r.t. then cooled in an ice bath. DBU (60.1 mL) was slowly added. After 20 min, the mixture was quenched by the addition of 1N HCl followed by 2.4 L of ice water. The precipitate was filtered and rinsed with water to give 64 g of crude product. This solid was dissolved in 750 mL of dichloromethane, dried over MgSO₄ and filtered. Purification by flash chromatography eluting with 10% EtOAc/CH₂Cl₂, followed by an ethyl acetate swish provided 36.6 g (58%) of the title compound.

| | | | |
|---|---|---|---|
| Analysis | calculated for C₁₇H₁₄O₄S | | |
| | C, 64.95; | H, 4.49; | S, 10.20 |
| Found: | C, 64.63; | H, 4.65; | S, 10.44 |

### Step 2: 5-Hydroxymethyl-4-(4-(methylsulfonyl)phenyl-3-phenyl-2-(5H)-furanone

A mixture of 3-phenyl-4-(4-(methylsulfonyl)phenyl)-2-(*5H*)-furanone from Step 1 (0.31 g, 1.0 mmol), 37% aqueous formaldehyde solution (0.1 mL), THF (20 mL) and water (5 mL) was treated with K₂CO₃ (20 mg, 0.15 mmol). After stirring for 1h at room temperature, the reaction mixture was quenched with 15 mL of saturated aqueous NH₄Cl and extracted with 50 mL of EtOAc. The extract was dried over MgSO₄ and concentrated. The residue was purified by flash chromatography eluting with 3:1 EtOAc/hexanes to give 50 mg of the title compound along with 120 mg of 5,5-bis(hydroxymethyl)-3-phenyl-4-(4-(methylsulfonyl)phenyl)-2-(5H)-furanone.
¹H NMR (acetone-d₆, 400 MHz) δ 3.15 (3H, s), 3.60 (1H, m), 4.03 (1H, m), 4.30 (1H, t), 5.72 (1H, t), 7.36 (5H, m), 7.66 (2H, d), 7.98 (2H, d).

### Step 3: 5-Methylene-4-(4-(methylsulfonyl)phenyl)-3-phenyl-2-(5H)-furanone

To a solution of the product of Step 2 (18 mg) and Et₃N (0.15 mL) in 10 mL of CH₂Cl₂ was added dropwise methanesulfonyl chloride at 0 °C. After stirring for 10 min at 0 °C, the reaction mixture was treated with 5 mL of saturated NaHCO₃ and extracted with 30 mL of 1:1 EtOAc/hexane. The extract was dried over MgSO₄ and concentrated to give 15 mg of the title compound as a white solid.
¹H NMR (acetone-d₆, 400 MHz) δ 3.16 (3H, s), 4.92 (1H, d), 5.40 (1H, d), 7.30-7.42 (5H, m), 7.68 (2H, d), 8.06 (2H, d).

### Step 4: 7-(4-(methylsulfonyl)(phenyl)-6-phenyl-4-oxa-spiro[2,4]-hept-6-en-5one

A solution of the compound from Step 3 (450 mg) and Pd(OAc)₂ (150 mg) in 200 ml of Et₂O was cooled to 0 °C and treated with excess CH₂N₂ (in ether) until no starting material was observed by TLC. The reaction mixture was then filtered through a pad of silica gel and concentrated. The residue was purified by flash chromatography eluting with 2:1 hexanes/EtOAc to give 180 mg of the title compound as a yellow solid.
1H NMR (acetone-d₆, 400 MHz) δ 1.32 (2H, m), 1.70 (2H, m), 3.16 (3H, s), 7.24-7.32 (3H, m), 7.36-7.44 (2H, m), 7.62 (2H, d), 8.05 (2H, d).

### EXAMPLE 14

### 5,5-Bisfluoromethyl-3-phenyl-4-(4-(methylsulfonyl)phenyl)-2-(5H)-furanone

### Step 1: 5,5-Bis(hydroxymethyl)-3-phenyl-4-(4-(methylsulfonyl)phenyl)-2-(5H)-furanone

The title compound was obtained by the procedure described in Step 2 of Example 13.
¹H NMR (acetone-d₆, 400 MHz) δ 3.16 (3H, s), 3.79 (2H, dd), 3.94 (2H, dd), 4.56 (2H, t), 7.20-7.35 (5H, m), 7.68 (2H, d), 8.04 (2H, d).

### Step 2 5,5-Bisfluoromethyl-3-phenyl-4-(4-(methylsulfonyl)phenyl)-2-(5H)-furanone

A solution of 5,5-bis(hydroxymethyl)-3-phenyl-4-(4-(methylsulfonyl)phenyl)-2-(*5H*)-furanone from Step 1 (0.55 g, 1.45 mmol) in 8 mL of CH₂Cl₂ was cooled to -78 °C and treated with Et₂NSF₃ (4 mL, 30 mmol). The mixture was then refluxed for 15h and quenched by carefully pouring into 100 mL of saturated aqueous solution of NaHCO₃. The product was extracted with 150 mL of EtOAc. The extract was dried over MgSO₄ and concentrated. The residue was purified by flash chromatography eluting with 2:1 EtOAc/hexanes to give 0.4 g of the title product as a white solid.
¹H NMR (acetone-d₆, 400 MHz) δ 3.14 (3H, s), 4.95 (4H, d), 7.25-7.40 (5H, m), 7.60 (2H, d), 8.06 (2H, d).

### EXAMPLE 16

### 3-(3,5-Difluoro-phenyl)-4-(4-methylsufonyl-phenyl)-1-oxa-spiro[4,4]non-3-en-2-one

M.p. 186°C. Anal. calcd. for C₁₉H₁₆F₂O₄S: C, 60.31; H, 4.26; S, 8.47. Found: C, 60.34; H, 4.38; S, 8.66.

### EXAMPLE 18

### 4-(4-Methylsulfonyl-phenyl)-3-phenyl-5,5-bis-trifluoromethyl-5H-furanone

M.p. 138°C. Anal. calcd. for C₁₉H₁₂F₆O₄S: C, 50.67; H, 2.69; S, 7.12. Found: C, 50.90; H, 2.82; S, 7.14.

### EXAMPLE 19

### 8-(4-Methylsulfonyl-phenyl)-7-phenyl-5-oxa-spiro[3,4]oct-7-en-6-one

### Step 1: 8-Hydroxy-7-phenyl-5-oxa-spiro[3,4]oct-7-en-6-one

To a solution of 1-hydroxy-cyclobutanecarboxylic acid (W.R. Jones *et al*. Organometallics **6**, 2579 (*1987*) (3.8 g, 32.7 mmol) in Et₂O (50 ml) at 0°C was added an etheral solution of CH₂N₂ until the yellow color persisted. The solvent was evaporated to give 3.5 g of the corresponding methyl ester. The crude ester was dissolved in CH₂Cl₂ (66 ml) and cooled to 0°C. Pyridine (4.22 g, 53.3 mmol, 4.30 ml) was added, followed by dropwise addition of phenylacetyl chloride (6.18 g, 40 mmol, 5.3 ml) over a 0.5 h period. The mixture was stirred at 0°C for 3 h and then poured into 5% aq. HCl (200 ml). The layers were separated and the aq. layer was extracted with CH₂Cl₂ (3x50 ml). The combined org. layers were washed with dil, aq. NaHCO₃ and brine (100 ml each) and dried over MgSO₄. Evaporation of the solvent and purification of the crude product by chromatography with 20% EtOAc in hexane gave the ester as an oil in quantitative yield.
A portion of the ester (2.18 g, 8.78 mmol) was dissolved in CH₂Cl₂ (44 ml) and cooled to 0°C. DBU (1.47 g, 9.66 mmol, 1.44 ml) was added and the mixture was allowed to stir at room temperature for 48 h. The dark solution was poured into 10% aq. HCl (100 ml) and the layers were separated. The aq. layer was extracted with CH₂Cl₂ (3x25 ml) and the combined org. layers were dried over MgSO₄. Evaporation of the solvent and purification by chromatography (10% EtOAc in toluene, 2.5% acetic acid) gave 460 mg of the title compound, mp 188-189°C.

### Step 2: Trifluoro-methanesulfonic acid 6-oxo-7-phenyl-5-oxa-spiro[3,4]oct-7-en-8-yl ester

To a solution of the lactone (460 mg, 2.12 mmol) in CH₂Cl₂ (15 ml) at 0°C was added NEt₃ (258 mg, 2.55 mmol, 356 µl) followed by Tf₂O (720 mg, 2.55 mmol, 429 µl). The solution was stirred for 1 h at 0°C and then poured into water. The layers were separated and the aq. layer was extracted with CH₂Cl₂ (3x20 ml). The combined org. layers were dried over MgSO₄ and the solvent was evaporated to give 550 mg of the product as an oil.

### Step 3: 4-(Methylthio)benzeneboronic acid

*n*-BuLi in hexane (2.5*M*, 625 mmol, 250 ml) is added slowly to a mechanically stirred solution of 4-bromothioanisole (100 g, 492 mmol) in THF (11) below -55°C (internal temp.). After aging at -72°C for 1 h trimethylborate (73 ml, 1.3 eq.) is added at such a rate to keep the internal temperature below -55°C. After 0.25 h at -78°C the solution was allowed to warm to 0°C for 10 min. *2N* HCL (500 ml) is added and the solvent is evaporated. Water (500 ml) is added and the product is extracted with EtOAc (1.5 l). The organic layer is dried over Na₂SO₄, concentrated and the solid is stirred in hexane (500 ml) for 2 h. Filtartion gave 61.9 g (75 %) of the desired product. ¹H NMR (acetone-d₆, 400 MHz) δ 7.80 (d, 2H), 7.22 (d, 2H), 7.15 (s, 2H), 2.50 (s, 3H).

### Step 4: 8-(4-Methylsulfanyl-phenyl)-7-phenyl-5-oxa-spiro[3,4]oct-7-en-6-one

To a solution of the triflate (550 mg, 1.58 mmol) from step 2 in THF (10 ml) and boronic acid from step 3 (318 mg, 1.90 mmol) was added an aq. solution of Na₂CO₃ (2.0*M*, 3.16 mmol, 1.60 ml) and Pd(PPh₃)₄ (91 mg, 0.079 mmol). The mixture was heated for 2 h at 70°C, cooled and poured into sat. aq. NH₄Cl. The aq. layer was extracted with Et₂O and the combined org, layers were dried over MgSO₄. Purification via flash chromatography (20% EtOAc in hexane) gave 460 mg of the title compound as a foam.

### Step 5: 8-(4-Methylsulfonyl-phenyl)-7-phenyl-5-oxa-spiro[3,4]oct-7-en-6-one

To the thioether from step 3 (460 mg, 1.43 mmol) in CH₂Cl₂ (17 ml) and MeOH (4.3 ml) at 0°C was added MMPP (970 mg, 1.57 mmol) in two portions. The ice bath was removed and the mixture was stirred at room temperature for 2 h. The suspension was filtered and the filtrate was washed with sat. aq. NaHCO₃ and water. After drying over MgSO₄ and evaporation of the solvent a yellow foam was obtained. Stirring of this foam with 10 ml Et₂O for 1 h and filtration gave 417 mg of the title compound as a colorless solid, m.p. 159-160°C.

### EXAMPLE 20

### 6-(3-Fluorophenyl)-7-(4-(methylsulfonyl)phenyl)-4-oxa-spiro[2,4]hept-6-en-5-one

Following method N, example 19 and substituting 1-hydroxy-cyclopropanecarboxylic acid for 1-hydroxy-cyclobutanecarboxylic acid, the tide compound was obtained as a colorless solid. M.p. 130-131°C.

### EXAMPLE 21

### 7-(4-Methylsulfonylphenyl)-6-naphthalen-2-yl-4-oxa-spiro[2,4]hept-6-en-5-one

Following method N, example 19 and substituting 1-hydroxy-cyclopropanecarboxylic acid for 1-hydroxy-cyclobutanecarboxylic acid, the title compound was obtained as a colorless solid. ¹H NMR (acetone-d₆, 400 MHz) δ 1.36-1.39 (2H, m), 1.73-1.76 (2H, m), 3.16 (3H, s), 7.35-7.38 (1H, m), 7.48-7.52 (2H, m), 7.65-7.85 (5H, m), 8.03-8.08 (3H, m).

### EXAMPLE 22

### 4-(6-Naphthalen-2-yl-5-oxo-4-oxa-spiro[2,4]hept-6-en-7-yl)-benzenesulfonamide

Following method N, example 19 and substituting 1-hydroxy-cyclopropanecarboxylic acid for 1-hydroxy-cyclobutanecarboxylic acid, the title compound was obtained as a colorless solid. ¹H NMR (acetone-d₆, 300 MHz) δ 1.32-1.48 (2H, m), 1.70-1.75 (2H, m), 6.70 (1H, bs), 7.34 (1H, dd), 7.46-7.60 (4H, m), 7.70-8.10 (6H, m).

### EXAMPLE 23

### 7-(4-Fluorophenyl)-8-(4-methylsulfonylphenyl)-5-oxa-spiro[3,4]oct-7-en-6-one

Anal. calcd. for C₂₀H₁₇FO₄S: C, 64.50; H, 4.60. Found: C, 64.46; H, 4.62.

### EXAMPLE 24

### 7-(3-Fluorophenyl)-8-(4-methylsulfonylphenyl)-5-oxa-spiro[3,4]oct-7-en-6-one

Anal. calcd. for C₂₀H₁₇FO₄S: C, 64.50; H, 4.60. Found: C, 64.44; H. 4.75.

### EXAMPLE 25

### 7-(3,4-Difluorophenyl)-8-(4-methylsulfonylphenyl)-5-oxa-spiro[3,4]oct-7-en-6-one

¹H NMR (acetone-d₆, 400 MHz) δ 1.15-1.22 (1H, m), 1.82-1.90 (1H, m), 2.62-2.80 (4H, m), 3.20 (3H, s), 7.10-7.25 (2H, m), 7.32-7.40 (1H, m), 7.82-7.86 (2H, dd), 8.10-8.16 (2H,dd).

### EXAMPLE 26

### 4-(6-Oxo-7-phenyl-5-oxa-spiro[3,4]oct-7-en-8-yl)-benzenesulfonamide

M.p. 162-163°C.

### EXAMPLE 27

### 4-[7-(4-Fluorophenyl)-6-oxo-5-oxa-spiro[3,4]oct-7-en-8-yl]-benzenesulfonamide

Anal. calcd. for C₁₉H₁₆FNO₄S: C, 61.12; H, 4.32; N, 3.75. Found: C, 61.18; H, 4.49; N, 3.77.

### EXAMPLE 28

### 4-[7-(3-Fluorophenyl)-6-oxo-5-oxa-spiro[3,4]oct-7-en-8-yl]-benzenesulfonamide

Anal. calcd. for C₁₉H₁₆FNO₄S: C, 61.12; H, 4.32; N, 3.75. Found: C, 61.00; H, 4.56; N, 3.76.

### EXAMPLE 29

### 4-[7-(3,4-Difluorophenyl)-6-oxo-5-oxa-spiro[3,4]oct-7-en-8-yl]-benzenesulfonamide

¹H NMR (acetone-d₆, 400 MHz) δ 1.12-1.24 (1H, m), 1.80-1.92 (1H,m), 2.61-2.70 (4H, m), 6.73 (1H, bs), 7.12-7.26 (2H, m), 7.31-7.40 (1H, m), 7.73-7.75 (2H, dd), 8.03-8.06 (2H, dd).

### EXAMPLE 30

### 3-(4-Fluorophenyl)-4-(4-(methylsulfonyl)phenyl)-1-oxa-spiro[4,4]non-3-en-2-one

M.p. 166-167°C. Anal. calcd. for C₂₁H₁₉FO₄S: C, 65.27; H, 4.96; S, 8.30. Found: C, 65.11; H, 5.10; S, 8.07.

### EXAMPLE 31

### 3-(3-Fluorophenyl)-4-(4-(methylsulfonyl)phenyl)-1-oxa-spiro[4,4]non-3-en-2-one

M.p. 158-159°C. Anal. calcd. for C₂₁H₁₉FO₄S: C, 65.27; H, 4.96; S, 8.30. Found: C, 65.13; H, 5.12; S, 8.56.

### EXAMPLE 32

### 3-(3,4-Difluorophenyl)-4-(4-(methylsulfonyl)phenyl)-1-oxa-spiro[4,4]non-3-en-2-one

M.p. 149.5-150.5°C. Anal. calcd. for C₂₁H₁₈F₂O₄S: C, 62.37; H, 4.49; S, 7.93. Found: C, 62.43; H, 4.64; S, 8.42.

### EXAMPLE 33

### 3-(3-Chloro-4-fluorophenyl)-4-(4-(methylsulfonyl)phenyl)-1-oxa-spiro[4,4]non-3-en-2-one

¹H NMR (acetone-d₆, 300 MHz) δ 1.60-2.20 (8H, m), 3.20 (3H, s), 7.20 (1H, t), 7.30 (1H, m), 7.55 (1H, dd), 7.70 (2H, d), 8.10 (2H, d). Anal. calcd. for C₂₁H₁₈ClFO₄S: C, 60.00; H, 4.28. Found: C, 60.02; H, 4.42.

### EXAMPLE 34

### 4-(2-Oxo-3-phenyl-1-oxa-spiro[4,4]non-3-en-4-yl)-benzenesulfonamide

M.p. 173°C. Anal. calcd. for C₂₀H₁₉NO₄S^{.}1/2 EtOAc: C, 63.91; H, 5.61; N, 3.39; S, 7.75. Found: C, 64.14; H, 5.74; N, 3.36; S, 7.70.

### EXAMPLE 35

### 4-[3-(4-Fluorophenyl)-2-oxo-1-oxa-spiro[4,4]non-3-en-4-yl)]-benzenesulfonamide

M.p. 169°C. Anal. calcd. for C₂₀H₁₈FNO₄S^{.}1/2 EtOAc: C, 61.24; H, 5.14; N, 3.25; S, 7.43. Found: C, 61.19; H, 5.29; N, 3.21; S, 7.40.

### EXAMPLE 36

### 4-[3-(3-Fluorophenyl)-2-oxo-1-oxa-spiro[4,4]non-3-en-4-yl)]-benzenesulfonamide

M.p. 142-144°C. Anal. calcd. for C₂₀H₁₈FO₄S: C, 62.00; H, 4.68; N, 3.62. Found: C, 61.83; H, 4.90; N, 3.53.

### EXAMPLE 37

### 4-[3-(3,4-Difluorophenyl)-2-oxo-1-oxa-spiro[4,4]non-3-en-4-yl)]-benzenesulfonamide

M.p. >92°C. Anal. calcd. for C₂₀H₁₇F₂O₄S: C, 59.25; H, 4.23; N, 3.45. Found: C, 59.22; H, 4.34; N, 3.37.

### EXAMPLE 38

### 4-[3-(3-Chloro-4-fluorophenyl)-2-oxo-1-oxa-spiro[4,4non-3en-4yl]-benzenesulfonamide

¹H NMR (acetone-d₆, 300 MHz) δ 1.70-2.20 (8H, m), 6.60 (2H, bs), 7.20 (1H, t), 7.25 (1H,m), 7.55 (3H, m), 8.00 (2H, d). M/z 422 (M+H)⁺.

### EXAMPLE 40

### 4-(2-Oxo-3-phenyl-1-oxa-spiro[4,5]dec-3-en-4-yl)-benzenesulfonamide

M.p. 213°C. Anal. calcd. for C₂₁H₂₁NO₄S: C, 65.78; H, 5.52; N, 3.65; S, 8.36. Found: C, 65.62; H, 5.56; N, 3.47; S, 8.44.

### EXAMPLE 41

### 4-[3-(4-Fluorophenyl)-2-oxo-1-oxa-spiro[4,5]dec-3-en-4-yl)-benzenesulfonamide

M.p. 202-203°C. Anal. calcd. for C₂₁H₂₀FNO₄S: C, 62.83; H, 5.02; N, 3.49; S, 7.99. Found: C, 62.66; H, 5.04; N, 3.48; S, 8.08.

## Claims

1. A compound of formula I 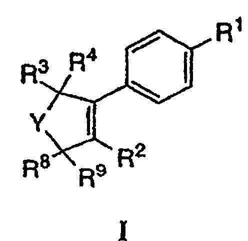 or a pharmaceutically acceptable salt thereof
wherein:
Y is selected from the group consisting of
(a) C(R¹⁰)(R¹¹),
(b) oxygen,
(c) sulfur,
R¹ is selected from the group consisting of
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² is selected from the group consisting of
(a) C₁₋₆alkyl,
(b) C₃₋₇ cycloalkyl,
(c) mono-, di- or tri-substituted phenyl or naphthyl wherein the substituent is selected from the group consisting of
(1) hydrogen,
(2) halo,
(3) C₁₋₆alkoxy,
(4) C₁₋₆alkylthio,
(5) CN,
(6) CF₃,
(7) C₁₋₆alkyl,
(8) N₃,
(9) -CO₂H,
(10) -CO₂-C₁₋₄alkyl,
(11) -C(R⁵)(R⁶)-OH,
(12) -C(R⁵)(R⁶)-O-C₁₋₄alkyl, and
(13) -C₁₋₆alkyl-CO₂-R⁵;
(14) benzyloxy,
(15) -O-(C₁₋₆alkyl)-CO₂R⁵,
(16) -O-(C₁₋₆alkyl)-NR⁵R⁶;
(d) mono-, di- or tri-substituted heteroaryl wherein the heteroaryl is a monocyclic aromatic ring of 5 atoms, said ring having one hetero atom which is S, O, or N, and optionally 1, 2, or 3 additionally N atoms; or
the heteroaryl is a monocyclic ring of 6 atoms, said ring having one hetero atom which is N, and optionally 1, 2, 3, or 4 additional N atoms, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
(e) a mono- or di- substituted benzoheterocycle in which the heterocycle is a 5, 6, or 7-membered ring which may contain 1 or 2 heteroatoms chosen independently from O, S, or N and which may contain a carbonyl group or a sulfonyl group; the said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
(f) a heterocycloalkyl group of 5-7 members which contains 1 or 2 heteroatoms chosen from O, S, or N and optionally contains a carbonyl group or a sulfonyl group.
(g) a mono- or di- substituted benzocarbocycle in which the carbocycle is a 5, 6, or 7-membered ring which optionally contains a carbonyl group, the said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
R³ is a mono- or di- substituted phenyl, a mono- or di- substituted benzyl, a mono- or di- substituted heteroaryl, a mono- or di- substituted heteroarylmethyl wherein the substituents are selected from
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
C₁₋₇alkyl, CH₂OR⁷, CN, CH₂CN, C₁₋₆fluoroalkyl, F or CONR⁷₂ when Y is C(R¹⁰)(R¹¹), or
R³ is a mono- or di- substituted phenyl, a mono- or di- substituted benzyl, a mono- or di- substituted heteroaryl, a mono- or di- substituted heteroarylmethyl wherein the substituents are selected from
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
CH₂OR⁷, CN, CH₂CN, C₁₋₆fluoroalkyl, F or CONR⁷₂ when Y is O or S;
R⁴ is
(a) a mono- or di- substituted phenyl, a mono- or di- substituted benzyl, a mono- or di- substituted heteroaryl or a mono- or di- substituted heteroarylmethyl as defined above, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl, or
(b) C₁₋₇alkyl, CH₂OR⁷, CN, CH₂CN, C₁₋₆fluoroalkyl, F or CONR⁷₂ or
R³ and R⁴ together with the carbon to which they are attached form a saturated monocyclic ring of 3, 4, 5, 6 or 7 atoms, optionally containing 1 or 2 heteroatoms chosen independently from O, S, N, and optionally hexa-substituted by R¹² and optionally containing a carbonyl or sulfonyl group;
each R¹² is independently selected from the group consisting of
(a) hydrogen,
(b) C₁₋₆alkyl,
(c) phenyl or monosubstituted phenyl wherein the substituents may be halo, C₁₋₆alkyl, C₁₋₆alkoxy, C₁₋₆alkylthio, CN, or CF₃,
(d) benzyl or monosubstituted benzyl wherein the substituents may be halo, C₁₋₆alkyl, C₁₋₆alkoxy, C₁₋₆alkylthio, CN, or CF₃,
(e) -CO₂-alkyl,
(f) halo,
R⁵, R⁶ and R⁷ are each independently selected from the group consisting of:
(a) hydrogen, and
(b) C₁₋₆alkyl,
or R⁵ and R⁶ together with the carbon to which they are attached form a saturated monocyclic carbon ring of 3, 4, 5, 6 or 7 atoms;
R⁸ and R⁹ are independently selected from the group consisting of:
(a) hydrogen,
(b) C₁₋₇alkyl, or
R⁸ and R⁹ together form a double bonded O or S;
R¹⁰ and R¹¹ are independently
(a) hydrogen,
(b) a mono- or di- substituted phenyl, a mono- or di- substituted benzyl, a mono- or di- substituted heteroaryl, or a mono- or di- substituted heteroarylmethyl, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R¹³)(R¹⁴)-OH, and
(10) -C(R¹³)(R¹⁴)-O-C₁₋₄alkyl, or
(c) C₁₋₇alkyl, CH₂OR⁷, CN, CH₂CN, C₁₋₆fluoroalkyl, F or CONR⁷₂, or
(d) R¹⁰ and R¹¹ together form a double bonded O; or R¹⁰ and R¹¹ together with the carbon to which they are attached form a saturated monocyclic carbon ring of 3, 4, 5, 6 or 7 atoms;
R¹³ and R¹⁴ are independently selected from the group consisting of:
(a) hydrogen,
(b) C₁₋₇alkyl, or
R¹³ and R¹⁴ together form a double bonded O or S.

2. A compound according to Claim 1 of formula Ia 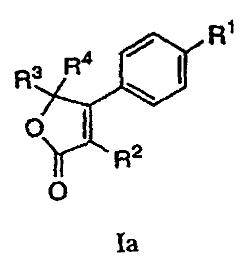 wherein:
R¹ is selected from the group consisting of
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH3)2
R² is selected from the group consisting of
(a) C₁₋₆alkyl,
(b) C₃, C₄, C₅ or C₆ cycloalkyl,
(c) mono-, di- or tri-substituted phenyl or naphthyl wherein the substituent is selected from the group consisting of
(1) hydrogen,
(2) halo,
(3) C₁₋₆alkoxy,
(4) C₁₋₆alkylthio,
(5) CN,
(6) CF₃,
(7) C₁₋₆alkyl,
(8) N₃,
(9) -CO₂H,
(10) -CO₂-C₁₋₄alkyl,
(11) -C(R⁵)(R⁶)-OH,
(12) -C(R⁵)(R⁶)-O-C₁₋₄alkyl, and
(13) -C₁₋₆alkyl-CO₂-R⁵;
(14) benzyloxy,
(15) -O-(C₁₋₆alkyl)-CO₂R⁵
(16) -O-(C₁₋₆alkyl)-NR⁵R⁶
(d) mono- or di-substituted heteroaryl selected from the group consisting of
(1) furanyl,
(2) diazinyl, triazinyl and tetrazinyl,
(3) imidazolyl,
(4) isooxazolyl,
(5) isothiazolyl,
(6) oxadiazolyl,
(7) oxazolyl,
(8) pyrazolyl,
(9) pyrrolyl,
(10) thiadiazolyl,
(11) thiazolyl,
(12) thienyl,
(13) triazolyl, and
(14) tetrazolyl,
wherein said substituents are selected from the group consisting of
(1) hydrogen,
(2) fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
(e) a mono- or di-substituted benzoheterocycle, benzocarbocycle or heterocycloalkyl selected from the group consisting of
(1) 2-indolyl,
(2) 3-indolyl,
(3) 1-methyl-5-indolyl
(4) 2-benzofuranyl,
(5) 3-benzofuranyl,
(6) 5-benzofuranyl,
(7) 6-benzofuranyl,
(8) 2-benzothienyl,
(9) 3-benzothienyl,
(10) 5-benzothienyl,
(11) 6-benzothienyl, 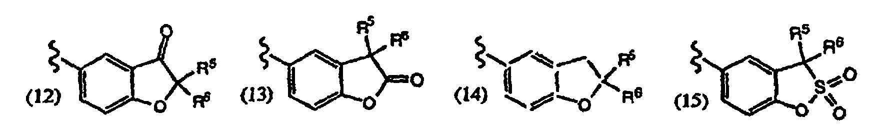 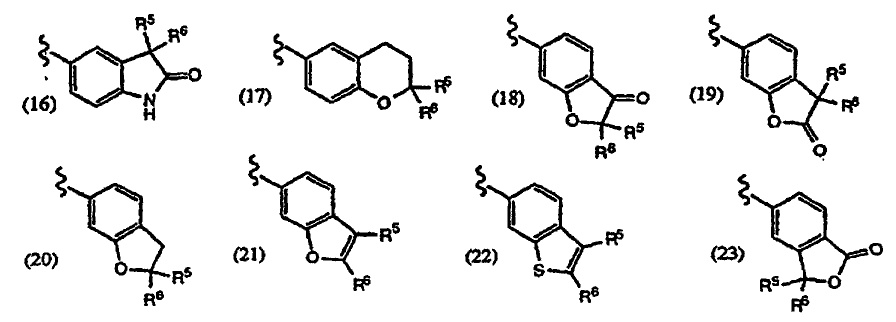 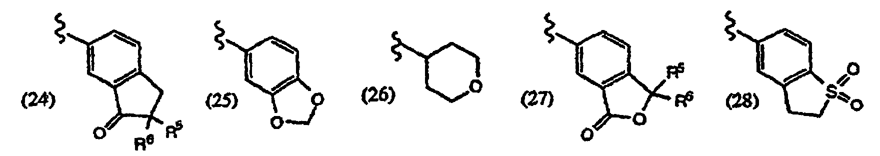 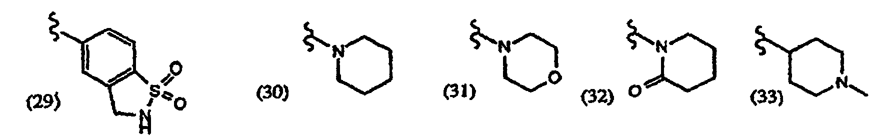
wherein said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl;
R³ is CH₂OR⁷, CN, CH₂CN, or C₁₋₆fluoroalkyl;
R⁴ is
(a) a mono- or di- substituted phenyl or a mono- or di-substituted heteroaryl, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl, or
(b) C₁₋₆alkyl, CH₂OR⁷, CN, CH₂CN, or C₁₋₆fluoroalkyl; or R³ and R⁴ together with the carbon to which they are attached form a saturated monocyclic ring of 3, 4, 5, 6 or 7 atoms, optionally containing 1 or 2 heteroatoms chosen independently from O S, N, and optionally hexa-substituted by R¹²,
each R¹² is independently selected from the group consisting of
(a) hydrogen,
(b) C₁₋₆alkyl,
(c) phenyl or monosubstituted phenyl wherein the substituents may be halo, C₁₋₆alkyl, C₁₋₆alkoxy, C₁₋₆alkylthio, CN, or CF₃,
(d) benzyl or monosubstituted benzyl wherein the substituents may be halo, C₁₋₆alkyl, C₁₋₆alkoxy, C₁₋₆alkylthio, CN, or CF₃,
(e) -CO₂-alkyl,
(f) halo;
R⁵, R⁶ and R⁷ are each independently selected from the group consisting of
(a) hydrogen, and
(b) C₁₋₆alkyl,
or R⁵ and R⁶ together with the carbon to which they are attached form a saturated monocyclic carbon ring of 3, 4, 5, 6 or 7 atoms.

3. A compound according to Claim 2 of formula Ia 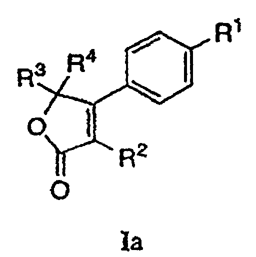 wherein:
R¹ is selected from the group consisting of
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH_{2,}
(g) P(O)(CH3)2
R² is selected from the group consisting of
(a) cyclohexyl, and
(b) mono-, di- or tri-substituted phenyl or naphthyl wherein the substituent is selected from the group consisting of
(1) hydrogen,
(2) halo,
(3) C₁₋₄alkoxy,
(4) C₁₋₄alkylthio,
(5) CN,
(6) CF₃,
(7) C₁₋₄alkyl,
(8) N₃,
(9) -CO₂H,
(10) -CO₂-C₁₋₄alkyl,
(11) -C(R⁵)(R⁶)-OH,
(12) -C(R⁵)(R⁶)-O-C₁₋₄alkyl, and
(13) -C₁₋₆alkyl-CO₂-R⁵,
(14) benzyloxy,
(15) -O-(C₁₋₄alkyl)-CO₂R⁵,
(16) -O-(C₁₋₄alkyl)-NR⁵R⁶;
R³ is CH₂OR⁷, CN, CH₂CN, or C₁₋₆fluoroalkyl;
R⁴ is
(a) a mono- or di- substituted phenyl or a mono- or di-substituted heteroaryl, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl, or
(b) C₁₋₆alkyl, CH₂OR⁷, CN, CH₂CN, or C₁₋₆fluoroalkyl; or R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tetra-R¹²,
each R¹² is independently selected from the group consisting of
(a) hydrogen,
(b) C₁₋₆alkyl,
(c) phenyl or monosubstituted phenyl wherein the substituents may be halo, C₁₋₆alkyl, C₁₋₆alkoxy, C₁₋₆alkylthio, CN, or CF₃,
(d) benzyl or monosubstituted benzyl wherein the substituents may be halo, C₁₋₆alkyl, C₁₋₆alkoxy, C₁₋₆alkylthio, CN, or CF₃,
(e) -CO₂-alkyl,
(f) halo,
R⁵, R⁶ and R⁷ are each independently selected from the group consisting of
(a) hydrogen, and
(b) C₁₋₆alkyl,
or R⁵ and R⁶ together with the carbon to which they are attached form a saturated monocyclic carbon ring of 3, 4, 5, 6 or 7 atoms.

4. A compound according to Claim 3 of formula Ia 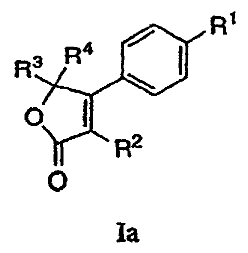 wherein:
R¹ is selected from the group consisting of
(a) S(O)₂CH₃,
(b) S(O)₂NH₂,
(c) S(O)(NH)NH₂,
R² is selected from the group consisting of mono-, di- or tri-substituted phenyl or naphthyl wherein the substituent is selected from the group consisting of
(1) hydrogen,
(2) halo,
(3) C₁₋₄alkoxy,
(4) C₁₋₄alkylthio,
(5) CN,
(6) CF₃,
(7) C₁₋₄alkyl,
(8) N₃,
(9) -CO₂H,
(10) -CO₂-C₁₋₄alkyl,
(11) -C(R⁵)(R⁶)-OH,
(12) -C(R⁵)(R⁶)-O-C₁₋₄alkyl, and
(13) -C₁₋₄alkyl-CO₂-R⁵;
R³ is CH₂OR⁷, CN, CH₂CN, or C₁₋₄fluoroalkyl;
R⁴ is
(a) a mono- or di- substituted phenyl or a mono- or di-substituted heteroaryl, said substituents are selected from the group consisting of
(1) hydrogen,
(2) fluoro, chloro and bromo,
(3) C₁₋₄alkyl,
(4) C₁₋₄alkoxy,
(5) C₁₋₄alkylthio,
(6) CN,
(7) CF₃, or
(b) C₁₋₄alkyl, CH₂OR⁷, CN, CH₂CN, or C₁₋₄fluoroalkyl; or R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tri-R¹²,
each R¹² is independently selected from the group consisting of
(a) hydrogen,
(b) C₁₋₄alkyl,
(c) phenyl or monosubstituted phenyl wherein the substituents may be halo, C₁₋₄alkyl, C₁₋₄alkoxy, C₁₋₆alkylthio, CN, or CF₃,
(d) benzyl or monosubstituted benzyl wherein the substituents may be halo, C₁₋₄alkyl, C₁₋₄alkoxy, C₁₋₄alkylthio, CN, or CF₃,
(e) -CO₂-C₁₋₄alkyl,
(f) halo,
R⁵, R⁶ and R⁷ are each independently selected from the group consisting of
(a) hydrogen, and
(b) C₁₋₄alkyl,
or R⁵ and R⁶ together with the carbon to which they are attached form a saturated monocyclic carbon ring of 3, 4, 5, 6 or 7 atoms.

5. A compound according to Claim 4 of formula Ia 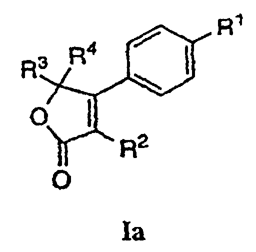 wherein:
R¹ is selected from the group consisting of
(a) S(O)₂CH₃,
(b) S(O)₂NH₂,
R² is selected from the group consisting of mono-, di- or tri-substituted phenyl or naphthyl wherein the substituent is selected from the group consisting of
(1) hydrogen,
(2) halo,
(3) C₁₋₄alkoxy,
(4) C₁₋₄alkylthio,
(5) CN,
(6) CF₃,
(7) C₁₋₄alkyl,
(8) N₃, and
(9) -C(R⁵)(R⁶)-OH;
R³ is CH₂OR⁷, CN, CH₂CN, or C₁₋₃fluoroalkyl;
R⁴ is
(a) a mono- or di- substituted phenyl or a mono- or di-substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₄alkylthio,
(6) CN,
(7) CF₃, or
(b) C₁₋₃alkyl, CH₂OR⁷, CN, CH₂CN, or C₁₋₃fluoroalkyl;
R⁵, R⁶ and R⁷ are each independently selected from the group consisting of
(a) hydrogen, and
(b) C₁₋₃alkyl.

6. A compound according to Claim 5 of formula Ia 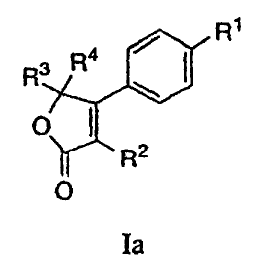 wherein:
R¹ is selected from the group consisting of
(a) S(O)₂CH₃,
(b) S(O)₂NH₂,
R² is selected from the group consisting of mono-, di- or tri-substituted phenyl or naphthyl wherein the substituent is selected from the group consisting of
(1) hydrogen,
(2) halo, selected from the group consisting of fluoro, chloro and bromo,
(3) C₁₋₃alkoxy,
(4) C₁₋₃alkylthio,
(5) CN, and
(6) C₁₋₃alkyl.
R³ is CH₂OR⁷, CN, CH₂CN, or C₁₋₃fluoroalkyl;
R⁴ is
(a) a mono- or di- substituted phenyl or a mono- or di-substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₄alkylthio,
(6) CF₃, or
(b) C₁₋₃alkyl, CH₂OR⁷, CN, CH₂CN, or C₁₋₃fluoroalkyl;
R⁵, R⁶ and R⁷ are each independently selected from the group consisting of
(a) hydrogen, and
(b) C₁₋₃alkyl.

7. A compound according to Claim 6 of formula Ia 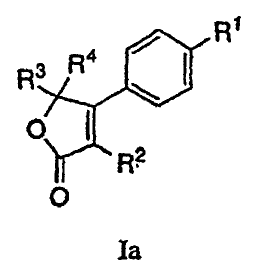 wherein:
R¹ is selected from the group consisting of S(O)₂CH₃,
R² is selected from the group consisting of mono-, di- or tri-substituted phenyl or naphthyl wherein the substituent is selected from the group consisting of
(1) hydrogen,
(2) halo, selected from the group consisting of fluoro, chloro and bromo,
(3) methoxy,
(4) methylthio,
(5) CN, and
(6) methyl and ethyl,
R³ is CH₂OR⁷, CN, CH₂CN, or trifluoromethyl;
R⁴ is
(a) a mono- or di- substituted phenyl or a mono- or di-substituted heteroaryl, said substituents are selected from the group consisting of
(1) hydrogen,
(2) fluoro, chloro andbromo,
(3) methyl and ethyl,
(4) methoxy,
(5) methylthio,
(6) CF₃, or
(b) C₁₋₃alkyl, CH₂OR⁷, CN, CH₂CN, or trifluoromethyl;
R⁵, R⁶ and R⁷ are each independently selected from the group consisting of
(a) hydrogen, and
(b) methyl.

8. A compound according to Claim 2 wherein R₂ is selected from the group consisting of
(a) mono- or di-substituted heteroaryl selected from the group consisting of
(1) furanyl,
(2) diazinyl, triazinyl and tetrazinyl,
(3) imidazolyl,
(4) isooxazolyl,
(5) isothiazolyl,
(6) oxadiazolyl,
(7) oxazolyl,
(8) pyrazolyl,
(9) pyrrolyl,
(10) thiadiazolyl,
(11) thiazolyl,
(12) thienyl,
(13) triazolyl, and
(14) tetrazolyl,
wherein said substituents are selected from the group consisting of
(1) hydrogen,
(2) fluoro, chloro, bromo and iodo,
(3) C₁₋₆alkyl,
(4) C₁₋₆alkoxy,
(5) C₁₋₆alkylthio,
(6) CN,
(7) CF₃,
(8) N3,
(9) -C(R⁵)(R⁶)-OH, and
(10) -C(R⁵)(R⁶)-O-C₁₋₄alkyl;
(b) a mono- or di-substituted benzoheterocycle, benzocarbocycle or heterocycloalkyl selected from the group consisting of
(1) 2-indolyl,
(2) 3-indolyl,
(3) 1-methyl-5-indolyl
(4) 2-benzofuranyl,
(5) 3-benzofuranyl,
(6) 5-benzofuranyl,
(7) 6-benzofuranyl,
(8) 2-benzothienyl,
(9) 3-benzothienyl,
(10) 5-benzothienyl,
(11) 6-benzothienyl, 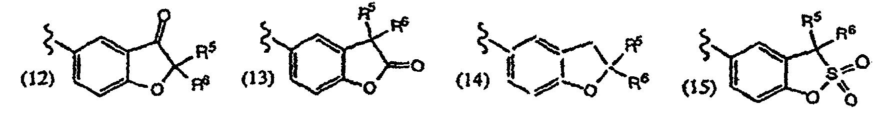 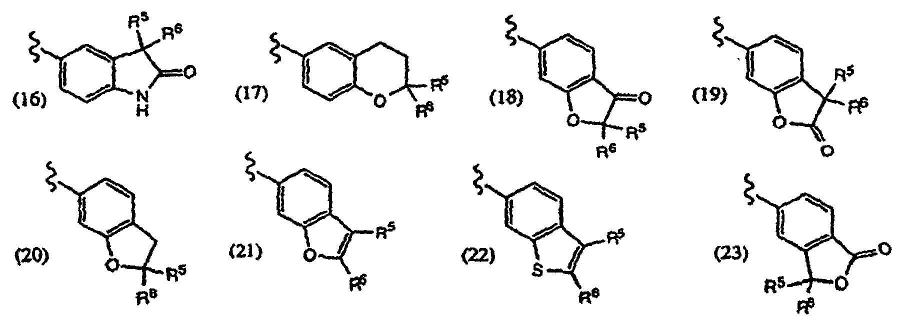 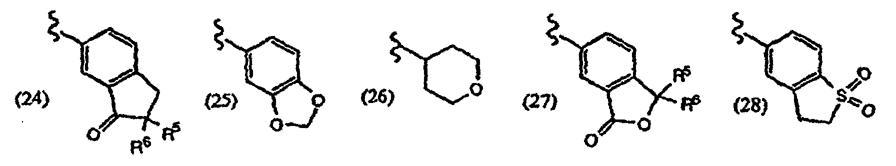 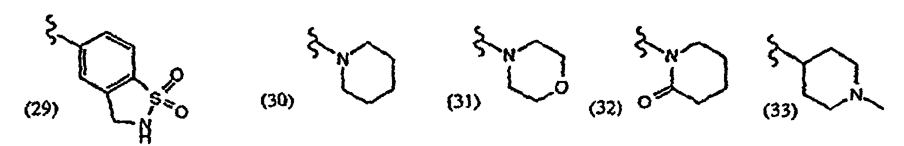
wherein said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl.

9. A compound according to Claim 8 wherein R² is a mono or di substituted heteroaryl wherein heteroaryl is selected from the group consisting of
(1) furanyl,
(2) diazinyl, triazinyl, tetrazinyl,
(3) imidazolyl,
(4) isooxazolyl,
(5) isothiazolyl,
(6) oxadiazolyl,
(7) oxazolyl,
(8) pyrazolyl,
(9) pyrrolyl,
(10) thiadiazolyl,
(11) thiazolyl,
(12) thienyl,
(13) triazolyl, and
(14) tetrazolyl,
wherein the substitutents are selected from the group consisting of
(1) hydrogen,
(2) fluoro or chloro,
(3) C₁₋₃alkoxy,
(4) C₁₋₆alkylthio,
(5) CN,
(6) CF₃,
(7) C₁₋₃alkyl,
(8) -C(R⁵)(R⁶)-OH;
(9) -C(R⁵)(R⁶)-O-C₁₋₄alkyl.

10. A compound according to Claim 9 wherein R² is a mono or di substituted heteroaryl wherein heteroaryl is selected from the group consisting of
(1) 2-furanyl,
(2) 3-furanyl,
(3) 2-thienyl,
(4) 3-thienyl,
(5) 3-isoxazolyl,
(6) 4-isoxazolyl,
(7) 5-isoxazolyl,
(8) 3-isothiazolyl,
(9) 4-isothiazolyl,
(10) 5-isothiazolyl,
(11) 2-oxazolyl,
(12) 4-oxazolyl,
(13) 5-oxazolyl,
(14) 2-thiazolyl,
(15) 4-thiazolyl,
(16) 5-thiazolyl,
(17) 1,2,3-thiadiazol-4-yl,
(18) 1,2,3-thiadiazol-5-yl,
(19) 1,2,4-thiadiazol-3-yl,
(20) 1,2,4-thiadiazol-5-yl,
(21) 1,3,4-thiadiazol-2-yl,
(22) 1,2,5-thiadiazol-3-yl,
(23) 1,2,3-oxadiazol-4-yl,
(24) 1,2,3-oxadiazol-5-yl,
(25) 1,2,4-oxadiazol-3-yl,
(26) 1,2,4-oxadiazol-5-yl,
(27) 1,3,4-oxadiazol-2-yl,
(28) 1,2,5-oxadiazol-3-yl,
(29) pyrazol-4-yl,
(30) pyrazol-5-yl,
(31) 1,2,3-triadiazol-4-yl,
(32) 1,2,3-triadiazol-5-yl,
(33) 1,2,4-triadiazol-3-yl,
(34) 1,2,4-triadiazol-5-yl,
(35) 1,2-diazinyl,
(36) 1,3-diazinyl,
(37) 1,4-diazinyl,
(38) 1,2,3,4-tetrazin-5-yl,
(39) 1,2,4,5-tetrazin-4-yl,
(40) 1,3,4,5-tetrazin-2-yl,and
(41) 1,2,3,5-tetrazin-4-yl.

11. A compound according to Claim 10 wherein R² is a mono or di substituted heteroaryl wherein heteroaryl is selected from the group consisting of
(1) 3-isoxazolyl,
(2) 4-isoxazolyl,
(3) 5-isoxazolyl,
(4) 3-isothiazolyl,
(5) 4-isothiazolyl,
(6) 5-isothiazolyl,
(7) 2-oxazolyl,
(8) 4-oxazolyl,
(9) 5-oxazolyl,
(10) 2-thiazolyl,
(11) 4-thiazolyl,
(12) 5-thiazolyl,
(13) 1,2,3-thiadiazol-4-yl,
(14) 1,2,3-thiadiazol-5-yl,
(15) 1,2,4-thiadiazol-3-yl,
(16) 1,2,4-thiadiazol-5-yl,
(17) 1,3,4-thiadiazol-2-yl,
(18) 1,2,5-thiadiazol-3-yl,
(19) 1,2,3-oxadiazol-4-yl,
(20) 1,2,3-oxadiazol-5-yl,
(21) 1,2,4-oxadiazol-3-yl,
(22) 1,2,4-oxadiazol-5-yl,
(23) 1,3,4-oxadiazol-2-yl,
(24) 1,2,5-oxadiazol-3-yl,
(25) 1,2-diazinyl,
(26) 1,3-diazinyl, and
(27) 1,4-diazinyl.

12. A compound according to Claim 11 wherein the hetreoaryl is selected from the group consisting of
(1) 3-isothiazolyl,
(2) 4-isothiazolyl,
(3) 5-isothiazolyl,
(4) 2-oxazolyl,
(5) 4-oxazolyl,
(6) 5-oxazolyl,
(7) 2-thiazolyl,
(8) 4-thiazolyl,
(9) 5-thiazolyl,
(10) 1,2-diazinyl,
(11) 1,3-diazinyl, and
(12) 1,4-diazinyl, and
wherein the substitutents are selected from the group consisting of
(1) hydrogen,
(2) fluoro or chloro,
(3) C₁₋₃alkoxy,
(4) C₁₋₃alkylthio,
(5) CN,
(6) C₁₋₃alkyl, and
(7) -C(R⁵)(R⁶)-OH,
R³ is CH₂OR⁷, CN, CH₂CN, or C₁₋₃fluoroalkyl;
R⁴ is
(a) substituted phenyl or substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₄alkylthio,
(6) CN,
(7) CF₃, or
(b) C₁₋₃alkyl, CH₂OR⁷, CN, CH₂CN, or C₁₋₃fluoroalkyl;
R5 and R6 are each independently hydrogen, methyl or ethyl.

13. A compound according to Claim 1 of formula Ib 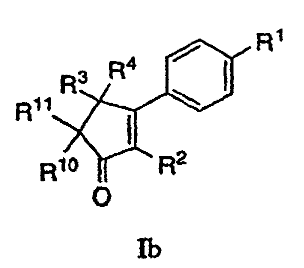 wherein:
R¹ is selected from the group consisting of
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH3)2
R² is selected from the group consisting of cyclohexyl, and
mono-, di- or tri-substituted phenyl or naphthyl wherein the substituent is selected from the group consisting of
(1) hydrogen,
(2) halo,
(3) C₁₋₆alkoxy,
(4) C₁₋₆alkylthio,
(5) CN,
(6) CF₃,
(7) C₁₋₆alkyl,
(8) N₃,
(9) -CO₂H,
(10) -CO₂-C₁₋₄alkyl,
(11) -C(R⁵)(R⁶)-OH,
(12) -C(R⁵)(R⁶)-O-C₁₋₄alkyl, and
(13) -C₁₋₆alkyl-CO₂-R⁵;
(14) benzyloxy,
(15) -O-(C₁₋₆alkyl)-CO₂R⁵
(16) -O-(C₁₋₆alkyl)-NR⁵R⁶
R³ is CH₂OR⁷, CN, CH₂CN, or C₁₋₆fluoroalkyl;
R⁴ is
(a) substituted phenyl or substituted heteroaryl, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R⁵)(R⁶)-OH, and
(10) -C(R⁵)(R⁶)-O-C₁₋₄alkyl, or
(b) C₁₋₆alkyl, CH₂OR⁷, CN, CH₂CN, or C₁₋₆fluoroalkyl; or
R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tetra-R¹²,
each R¹² is independently selected from the group consisting of
(a) hydrogen,
(b) C₁₋₆alkyl,
(c) phenyl or monosubstituted phenyl wherein the substituents may be halo, C₁₋₆alkyl, C₁₋₆alkoxy, C₁₋₆alkylthio, CN, or CF₃,
(d) benzyl or monosubstituted benzyl wherein the substituents may be halo, C₁₋₆alkyl, C₁₋₆alkoxy, C₁₋₆alkylthio, CN, or CF₃,
(e) -CO₂-alkyl,
(f) halo,
R⁵, R⁶ and R⁷ are each independently selected from the group consisting of
(a) hydrogen, and
(b) C₁₋₆alkyl,
or R⁵ and R⁶ together with the carbon to which they are attached form a saturated monocyclic carbon ring of 3, 4, 5, 6 or 7 atoms;
R¹⁰ and R¹¹ are independently
(a) hydrogen,
(b) substituted phenyl or substituted heteroaryl, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(8) N₃,
(9) -C(R¹³)(R¹⁴)-OH, and
(10) -C(R¹³)(R¹⁴)-O-C₁₋₄alkyl, or
(c) C₁₋₇alkyl, CH₂OR⁷, CN, CH₂CN, or C₁₋₆fluoroalkyl; or R¹⁰ and R¹¹ together with the carbon to which they are attached form a saturated monocyclic carbon ring of 3, 4, 5, 6 or 7 atoms;
R¹³ and R¹⁴ are independently selected from the group consisting of:
(a) hydrogen,
(b) C₁₋₆alkyl, or
R¹³ and R¹⁴ together form a double bonded O or S.

14. A compound according to Claim 13 of formula Ib 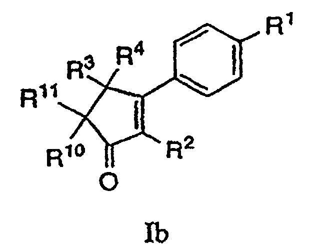 wherein:
R¹ is selected from the group consisting of
(a) S(O)₂CH₃,
(b) S(O)₂NH₂,
(c) S(O)(NH)NH₂,
R² is selected from the group consisting of mono-, di- or tri-substituted phenyl or naphthyl wherein the substituent is selected from the group consisting of
(1) hydrogen,
(2) halo,
(3) C₁₋₆alkoxy,
(4) C₁₋₄alkylthio,
(5) CN,
(6) CF₃,
(7) C₁₋₄alkyl,
(8) N₃,
(9) -CO₂H,
(10) -CO₂-C₁₋₄alkyl,
(11) -C(R⁵)(R⁶)-OH,
(12) -C(R⁵)(R⁶)-O-C₁₋₄alkyl, and
(13) -C₁₋₄alkyl-CO₂-R⁵;
R³ is CH₂OR⁷, CN, CH₂CN, or C₁₋₆fluoroalkyl;
R⁴ is
(a) substituted phenyl or substituted heteroaryl, said substituents are selected from the group consisting of
(1) hydrogen,
(2) fluoro, chloro and bromo,
(3) C₁₋₄alkyl,
(4) C₁₋₄alkoxy,
(5) C₁₋₄alkylthio,
(6) CN,
(7) CF₃, or
(b) C₁₋₄alkyl, CH₂OR⁷, CN, CH₂CN, or C₁₋₄fluoroalkyl; or R³ and R⁴ together with the carbon to which they are attached form a saturated monocyclic ring of 3, 4, 5, 6 or 7 atoms, optionally containing 1 or 2 heteroatoms chosen independently from O, S, N, and optionally substituted by tri-R¹²,
each R¹² is independently selected from the group consisting of
(a) hydrogen,
(b) C₁₋₄alkyl,
(c) phenyl or monosubstituted phenyl wherein the substituents may be halo, C₁₋₄alkyl, C₁₋₄alkoxy, C₁₋₆alkylthio, CN, or CF₃,
(d) benzyl or monosubstituted benzyl wherein the substituents may be halo, C₁₋₄alkyl, C₁₋₄alkoxy, C₁₋₄alkylthio, CN, or CF₃,
(e) -CO₂-C₁₋₄alkyl,
(f) halo,
R⁵, R⁶ and R⁷ are each independently selected from the group consisting of
(a) hydrogen, and
(b) C₁₋₄alkyl,
or R⁵ and R⁶ together with the carbon to which they are attached form a saturated monocyclic carbon ring of 3, 4, 5, 6 or 7 atoms;
R¹⁰ and R¹¹ are independently
(a) hydrogen,
(b) substituted phenyl or substituted heteroaryl, said substituents are selected from the group consisting of
(1) hydrogen,
(2) halo, including fluoro, chloro, bromo and iodo,
(3) C₁₋₆alkyl,
(4) C₁₋₆alkoxy,
(5) C₁₋₆alkylthio,
(6) CN,
(7) CF₃,
(c) C₁₋₄alkyl, CH₂OR⁷, CN, CH₂CN, or C₁₋₄fluoroalkyl; or
R¹⁰ and R¹¹ together with the carbon to which they are attached form a saturated monocyclic carbon ring of 3, 4, 5, 6 or 7 atoms;
R¹³ and R¹⁴ are independently selected from the group consisting of:
(a) hydrogen,
(b) C₁₋₄alkyl.

15. A compound according to Claim 14 of formula Ib 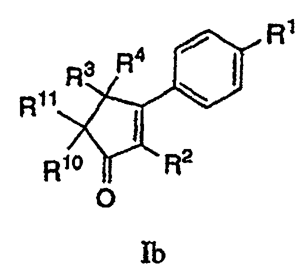 wherein:
R¹ is selected from the group consisting of
(a) S(O)₂CH₃,
(b) S(O)₂NH₂,
R² is selected from the group consisting of mono-, di- or tri-substituted phenyl or naphthyl wherein the substituent is selected from the group consisting of
(1) hydrogen,
(2) halo,
(3) C₁₋₄alkoxy,
(4) C₁₋₄alkylthio,
(5) CN,
(6) CF₃,
(7) C₁₋₄alkyl,
(8) CO₂H, and
(9) -C(R⁵)(R⁶)-OH;
R³ is CH₂OR⁷, CN, CH₂CN, or C₁₋₃fluoroalkyl;
R⁴ is
(a) substituted phenyl or substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₄alkylthio,
(6) CN,
(7) CF₃, or
(b) C₁₋₃alkyl, CH₂OR⁷, CN, CH₂CN, or C₁₋₃fluoroalkyl;
R⁵, R⁶ and R⁷ are each independently selected from the group consisting of
(a) hydrogen, and
(b) C₁₋₃alkyl;
R¹⁰ and R¹¹ are independently
(a) hydrogen,
(b) substituted phenyl or substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₃alkylthio,
(6) CN,
(7) CF₃,
(c) C₁₋₃alkyl, CH₂OR⁷, CN, CH₂CN, or C₁₋₄fluoroalkyl;
R¹³ and R¹⁴ are independently selected from the group consisting of:
(a) hydrogen,
(b) C₁₋₃alkyl.

16. A compound according to Claim 15 of formula Ib 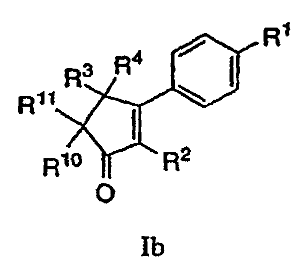 wherein:
R¹ is selected from the group consisting of
(a) S(O)₂CH₃,
(b) S(O)₂NH₂,
R² is selected from the group consisting of mono-, di- or tri-substituted phenyl or naphthyl wherein the substituent is selected from the group consisting of
(1) hydrogen,
(2) halo, selected from the group consisting of fluoro, chloro and bromo,
(3) C₁₋₃alkoxy,
(4) C₁₋₃alkylthio,
(5) CN, and
(6) C₁₋₃alkyl.
R³ is CH₂OR⁷, CN, CH₂CN, or C₁₋₃fluoroalkyl;
R⁴ is
(a) substituted phenyl or substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₄alkylthio.
(6) CF₃, or
(b) C₁₋₃alkyl, CH₂OR⁷, CN, CH₂CN, or C₁₋₃fluoroalkyl;
R⁵, R⁶ and R⁷ are each independently selected from the group consisting of
(a) hydrogen, and
(b) C₁₋₃alkyl,
R¹⁰ and R¹¹ are independently
(a) hydrogen,
(b) a mono- or di- substituted phenyl or a mono- or di-substituted heteroaryl, said substituents are selected from the group consisting of
(1) hydrogen,
(2) halo, including fluoro, chloro, bromo and iodo,
(3) C₁₋₃alkyl,
(4) C₁₋₃alkoxy,
(5) C₁₋₃alkylthio,
(6) CF₃,
(c) C₁₋₃alkyl, CH₂OR⁷, CN, CH₂CN, or C₁₋₄fluoroalkyl;
R¹³ and R¹⁴ are independently selected from the group consisting of:
(a) hydrogen,
(b) C₁₋₃alkyl.

17. A compound selected from the group consisting of
(a) 5,5-Dimethyl-4-(4-(methylsulfonyl)phenyl)-3-(4-methoxyphenyl)-2-(5H)-furanone,
(b) 5,5-Dimethyl-3-(3-methoxyphenyl)-4-(4-(methylsulfonyl)phenyl)-2-(5H)-furanone,
(c) 5,5-Dimethyl-3-(4-isopropylphenyl)-4-(4-(methylsulfonyl)phenyl)-2-(5H)-furanone,
(d) 5,5-Dimethyl-4-(4-(methylsulfonyl)phenyl)-3-phenyl-2-(5H)-furanone,
(e) 3-(Benzo[1,3]dioxol-5-yl)-5,5-dimethyl-4-(4-(methylsulfonyl)phenyl)-2-(5H)-furanone,
(f) 5,5-Dimethyl-3-(4-methylphenyl)-4-(4-(methylsulfonyl)phenyl)-2-(5H)-furanone,
(g) 5,5-Dimethyl-4-(4-(methylsulfonyl)phenyl)-3-(4-trifluoromethylphenyl)-2-(5H)-furanone,
(h) 5,5-Dimethyl-3-(3-methylphenyl)-4-(4-(methylsulfonyl)phenyl)-2-(5H)-furanone,
(i) 5,5-Dimethyl-3-cyclohexyl-4-(4-(methylsulfonyl) phenyl)-2-(5H)-furanone,
(j) 4-(4-(Methylsulfonyl)phenyl)-3-phenyl-1-oxa-spiro[4,4]non-3-en-2-one,
(k) 5,5-Dimethyl-3-(4-(methylsulfonyl)phenyl)-2-phenylocyclopent-2-enone,
(l) 4,4-Dimethyl-3-(4-(methylsulfonyl)phenyl)-2-phenylcyclopent-2-enone,
(m) 7-(4-(methylsulfonyl)phenyl)-6-phenyl-4-oxa-spiro[2.4]-hept-6-en-5-one, and
(n) 5,5-Bis(fluoromethyl)-3-phenyl-4-(4-(methylsulfonyl)phenyl)-2-(5H)-furanone.

18. A compound according to Claim 1 selected from
(b) 3-(3,5-Difluoro-phenyl)-4-(4-methylsufonyl-phenyl)-1-oxa-spire[4.4]non-3-en-2-one,
(d) 4-(4-Methylsulfonyl-phenyl)-3-phenyl-5,5-bis-trifluoromethyl-*5H*-furanone,
(e) 8-(4-Methylsulfonyl-phenyl)-7-phenyl-5-oxa-spiro[3.4]oct-7-en-6-one,
(f) 6-(3-Fluorophenyl)-7-(4-(methylsulfonyl)phenyl)-4-oxa-spiro[2.4]hept-6-en-5-one,
(g) 7-(4-Methylsulfonylphenyl)-6-naphthalen-2-yl-4-oxa-spiro[2.4]hept-6-en-5-one,
(h) 4-(6-Naphthalen-2-yl-5-oxo-4-oxa-spiro[2.4]kept-6-en-7-yl)-benzenesulfonamide,
(i) 7-(4-Fluorophenyl)-8-(4-methylsulfonylphenyl)-5-oxa-spiro[3.4]oct-7-en-6-one,
(j) 7-(3-Fluorophenyl)-8-(4-methylsulfonylphenyl)-5-oxa-spiro[3.4]oct-7-en-6-one,
(k) 7-(3,4-Difluorophenyl)-8-(4-methylsulfonylphenyl)-5-oxa-spiro[3.4]oct-7-en-6-one,
(l) 4-(6-Oxo-7-phenyl-5-oxa-spiro[3.4]oct-7-en-8-yl)-benzenesulfonamide,
(m) 4-[7-(4-Fluorophenyl)-6-oxo-5-oxa-spiro[3.4]oct-7-en-8-yl]-benzenesulfonamide,
(n) 4-[7-(3-Fluorophenyl)-6-oxo-5-oxa-spiro[3.4]oct-7-en-8-yl]-benzenesulfonamide,
(o) 4-[7-(3,4-Difluorophenyl)-6-oxo-5-oxa-spiro[3.4]oct-7-en-8-yl]-benzenesulfonamide,
(p) 3-(4-Fluorophenyl)-4-(4-(methylsulfonyl)phenyl)-1-oxa-spiro[4.4]non-3-en-2-one,
(q) 3-(3-Fluorophenyl)-4-(4-(methylsulfonyl)phenyl)-1-oxa-spiro[4.4]non-3-en-2-one,
(r) 3-(3,4-Difluorophenyl)-4-(4-(methylsulfonyl)phenyl)-1-oxa-spiro[4.4]non-3-en-2-one,
(s) 3-(3-Chloro-4-fluorophenyl)-4-(4-(methylsulfonyl)phenyl)-1-oxa-spiro[4.4]non-3-en-2-one,
(t) 4-(2-Oxo-3-phenyl-1-oxa-spiro[4.4]non-3-en-4-yl)-benzenesulfonamide,
(u) 4-[3-(4-Fluorophenyl)-2-oxo-1-oxa-spiro[4.4]non-3-en-4-yl)]-benzenesulfonamide,
(v) 4-[3-(3-Fluorophenyl)-2-oxo-1-oxa-spiro[4.4]non-3-en-4-yl)]-benzenesulfonamide,
(w) 4-[3-(3,4-Difluorophenyl)-2-oxo-1-oxa-spiro[4.4]non-3-en-4-yl)]-benzenesulfonamide,
(x) 4-[3-(3-Chloro-4-fluorophenyl)-2-oxo-1-oxa-spiro[4.4non-3en-4yl]-benzenesulfonamide,
(z) 4-(2-Oxo-3-phenyl-1-oxa-spiro[4,5]dec-3-en-4-yl)-benzenesulfonamide, and
(aa) 4-[3-(4-Fluorophenyl)-2-oxo-1-oxa-spiro[4.5]dec-3-en-4-yl)-benzenesulfonamide.

19. A pharmaceutical composition comprising: a non-toxic therapeutically effective amount of a compound according to Claim 1, 2, 3, 4, 5, 6, 7, 8, 9, 10, 11, 12, 13, 14, 15, 16, 17 or 18 or a pharmaceutically acceptable salt thereof and a pharmaceutically acceptable carrier.

20. A pharmaceutically acceptable salt of a compound of the formula in Claim 1, 2, 3, 4, 5, 6, 7, 8, 9, 10, 11, 12, 13, 14, 15 or 16.

21. A compound or pharmaceutically acceptable salt thereof, as defined in claim Claim 1, 2, 3, 4, 5, 6, 7, 8, 9, 10, 11, 12, 13, 14, 15, 16, 17 or 18 for use in treating an inflammatory disease susceptible to treatment with a non-steroidal anti-inflammatory agent

22. Use of a compound or pharmaceutically acceptable salt thereof, of Claim 1, 2, 3, 4, 5, 6, 7, 8, 9, 10, 11, 12, 13, 14, 15, 16, 17 or 18, in the manufacture of a medicament for treating cyclooxygenase mediated diseases.

## Patentansprüche

1. Eine Verbindung der Formel I 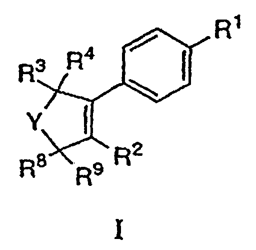 oder ein pharmazeutisch annehmbares Salz davon,
wobei:
Y ausgewählt ist aus der Gruppe, bestehend aus
(a) C(R¹⁰) (R¹¹),
(b) Sauerstoff,
(c) Schwefel,
R¹ ausgewählt ist aus der Gruppe, bestehend aus
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² ausgewählt ist aus der Gruppe, bestehend aus
(a) C₁₋₆-Alkyl,
(b) C₃₋₇-Cycloalkyl,
(c) mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₆-Alkoxy,
(4) C₁₋₆-Alkylthio,
(5) CN,
(6) CF₃,
(7) C₁₋₆-Alkyl,
(8) N₃,
(9) -CO₂H,
(10) -CO₂-C₁₋₄-Alkyl,
(11) -C(R⁵)(R⁶)-OH,
(12) -C(R⁵)(R⁶)-O-C₁₋₄-Alkyl und
(13) -C₁₋₆-Alkyl-CO₂-R⁵,
(14) Benzyloxy,
(15) -O-(C₁₋₆-Alkyl)-CO₂R⁵,
(16) -O-(C₁₋₆-Alkyl)-NR⁵R⁶,
(d) mono-, di- oder trisubstituiertem Heteroaryl, wobei das Heteroaryl ein monocyclischer aromatischer Ring aus 5 Atomen ist, der Ring ein Heteroatom, das S, O oder N ist, und gegebenenfalls 1, 2 oder 3 zusätzliche N-Atome enthält, oder das Heteroaryl ein monocyclischer Ring aus 6 Atomen ist, der Ring ein Heteroatom, das N ist, und gegebenenfalls 1, 2, 3 oder 4 zusätzliche N-Atome enthält, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl,
(e) einem mono- oder disubstituierten Benzoheterocyclus, wobei der Heterocyclus ein 5, 6 oder 7gliedriger Ring ist, der 1 oder 2 Heteroatome, unabhängig ausgewählt aus O, S oder N, enthalten kann und der eine Carbonylgruppe oder eine Sulfonylgruppe enthalten kann, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl,
(f) einer Heterocycloalkylgruppe mit 5-7 Elementen, die 1 oder 2 Heteroatome, ausgewählt aus O, S oder N, und gegebenenfalls eine Carbonylgruppe oder eine Sulfonylgruppe enthält,
(g) einem mono- oder disubstituierten Benzocarbocyclus, wobei der Carbocyclus ein 5-, 6- oder 7gliedriger Ring ist, der gegebenenfalls eine Carbonylgruppe enthält, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl,
R³ ein mono- oder disubstituiertes Phenyl, ein mono- oder disubstituiertes Benzyl, ein mono- oder disubstituiertes Heteroaryl, ein mono- oder disubstituiertes Heteroarylmethyl, wobei die Substituenten ausgewählt sind aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl,
C₁₋₇-Alkyl, CH₂OR⁷, CN, CH₂CN, C₁₋₆-Fluoralkyl, F oder CONR⁷₂ ist, wenn Y C(R¹⁰)(R¹¹) ist, oder
R³ ein mono- oder disubstituiertes Phenyl, ein mono- oder disubstituiertes Benzyl, ein mono- oder disubstituiertes Heteroaryl, ein mono- oder disubstituiertes Heteroarylmethyl, wobei die Substituenten ausgewählt sind aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵) (R⁶)-OH und
(10) -C(R⁵) (R⁶)-O-C₁₋₄-Alkyl,
CH₂OR⁷, CN, CH₂CN, C₁₋₆-Fluoralkyl, F oder CONR⁷₂ ist, wenn Y O oder S ist,
R⁴ ist
(a) ein mono- oder disubstituiertes Phenyl, ein mono- oder disubstituiertes Benzyl, ein mono- oder disubstituiertes Heteroaryl oder ein mono- oder disubstituiertes Heteroarylmethyl, wie sie oben definiert sind, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl, oder
(b) C₁₋₇-Alkyl, CH₂OR⁷, CN, CH₂CN, C₁₋₆-Fluoralkyl, F oder CONR⁷₂, oder
R³ und R⁴ zusammen mit dem Kohlenstoff, an den sie gebunden sind, einen gesättigten monocyclischen Ring aus 3, 4, 5, 6 oder 7 Atomen bilden, der gegebenenfalls 1 oder 2 Heteroatome, unabhängig ausgewählt aus O, S, N, enthält und gegebenenfalls durch R¹² hexasubstituiert ist und gegebenenfalls eine Carbonyl- oder Sulfonylgruppe enthält,
jedes R¹² unabhängig ausgewählt ist aus der Gruppe, bestehend aus
(a) Wasserstoff,
(b) C₁₋₆-Alkyl,
(c) Phenyl oder monosubstituiertem Phenyl, wobei die Substituenten Halogen, C₁₋₆-Alkyl, C₁₋₆-Alkoxy, C₁₋₆-Alkylthio, CN oder CF₃ sein können,
(d) Benzyl oder monosubstituiertem Benzyl, wobei die Substituenten Halogen, C₁₋₆-Alkyl, C₁₋₆-Alkoxy, C₁₋₆-Alkylthio, CN oder CF₃ sein können,
(e) -CO₂-Alkyl,
(f) Halogen,
R⁵, R⁶ und R⁷ jeweils unabhängig ausgewählt sind aus der Gruppe, bestehend aus
(a) Wasserstoff und
(b) C₁₋₆-Alkyl,
oder R⁵ und R⁶ zusammen mit dem Kohlenstoff, an den sie gebunden sind, einen gesättigten monocyclischen Kohlenstoffring aus 3, 4, 5, 6 oder 7 Atomen bilden,
R⁸ und R⁹ unabhängig ausgewählt sind aus der Gruppe, bestehend aus:
(a) Wasserstoff,
(b) C₁₋₇-Alkyl, oder
R⁸ und R⁹ zusammen ein doppelt gebundenes O oder S bilden, R¹⁰ und R¹¹ unabhängig
(a) Wasserstoff,
(b) ein mono- oder disubstituiertes Phenyl, ein mono- oder disubstituiertes Benzyl, ein mono- oder disubstituiertes Heteroaryl oder ein mono- oder disubstituiertes Heteroarylmethyl, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R¹³)(R¹⁴)-OH und
(10) -C(R¹³)(R¹⁴)-O-C₁₋₄-Alkyl, oder
(c) C₁₋₇-Alkyl, CH₂OR⁷, CN, CH₂CN, C₁₋₆-Fluoralkyl, F oder CONR⁷₂ sind oder
(d) R¹⁰ und R¹¹ zusammen ein doppelt gebundenes O bilden, oder
R¹⁰ und R¹¹ zusammen mit dem Kohlenstoff, an den sie gebunden sind, einen gesättigten monocyclischen Kohlenstoffring aus 3, 4, 5, 6 oder 7 Atomen bilden,
R¹³ und R¹⁴ unabhängig ausgewählt sind aus der Gruppe, bestehend aus:
(a) Wasserstoff,
(b) C₁₋₇-Alkyl, oder
R¹³ und R¹⁴ zusammen ein doppelt gebundenes O oder S bilden.

2. Eine Verbindung gemäß Anspruch 1 der Formel Ia 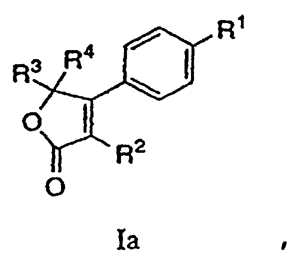 wobei:
R¹ ausgewählt ist aus der Gruppe, bestehend aus
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O) (CH₃)NH₂,
(g) P(O) (CH₃)₂,
R² ausgewählt ist aus der Gruppe, bestehend aus
(a) C₁₋₆-Alkyl,
(b) C₃-, C₄-, C₅- oder C₆-Cycloalkyl,
(c) mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₆-Alkoxy,
(4) C₁₋₆-Alkylthio,
(5) CN,
(6) CF₃,
(7) C₁₋₆-Alkyl,
(8) N₃,
(9) -CO₂H,
(10) -CO₂-C₁₋₄-Alkyl,
(11) -C(R⁵)(R⁶)-OH,
(12) -C(R⁵)(R⁶)-O-C₁₋₄-Alkyl und
(13) -C₁₋₆-Alkyl-CO₂-R⁵,
(14) Benzyloxy,
(15) -O-(C₁₋₆-Alkyl)-CO₂R⁵,
(16) -O-(C₁₋₆-Alkyl)-NR⁵R⁶,
(d) mono- oder disubstituiertem Heteroaryl, ausgewählt aus der Gruppe, bestehend aus
(1) Furanyl,
(2) Diazinyl, Triazinyl und Tetrazinyl,
(3) Imidazolyl,
(4) Isooxazolyl,
(5) Isothiazolyl,
(6) Oxadiazolyl,
(7) Oxazolyl,
(8) Pyrazolyl,
(9) Pyrrolyl,
(10) Thiadiazolyl,
(11) Thiazolyl,
(12) Thienyl,
(13) Triazolyl und
(14) Tetrazolyl,
wobei die Substituenten ausgewählt sind aus der Gruppe, bestehend aus
(1) Wasserstoff,
(2) Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl,
(e) einem mono- oder disubstituierten Benzoheterocyclus, Benzocarbocyclus oder Heterocycloalkyl, ausgewählt aus der Gruppe, bestehend aus
(1) 2-Indolyl,
(2) 3-Indolyl,
(3) 1-Methyl-5-indolyl,
(4) 2-Benzofuranyl,
(5) 3-Benzofuranyl,
(6) 5-Benzofuranyl,
(7) 6-Benzofuranyl,
(8) 2-Benzothienyl,
(9) 3-Benzothienyl,
(10) 5-Benzothienyl,
(11) 6-Benzothienyl, 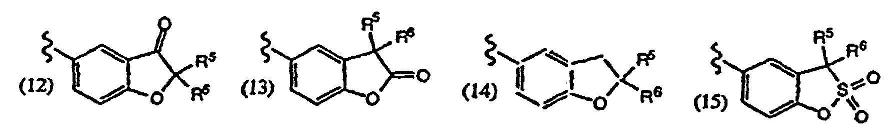 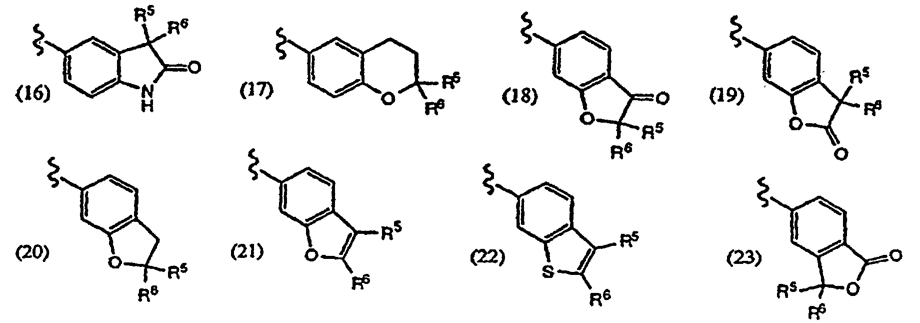 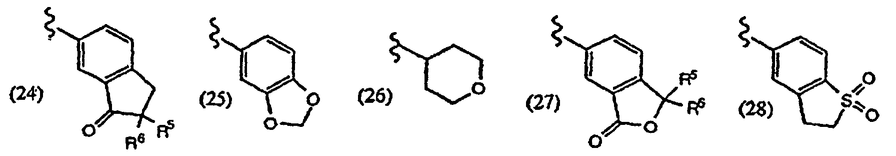 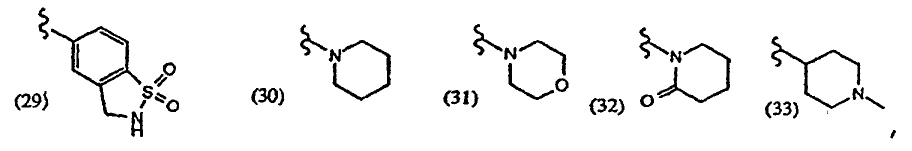
wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl,
R³ CH₂OR⁷, CN, CH₂CN oder C₁₋₆-Fluoralkyl ist,
R⁴ ist
(a) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl, oder
(b) C₁₋₆-Alkyl, CH₂OR⁷, CN, CH₂CN oder C₁₋₆-Fluoralkyl, oder R³ und R⁴ zusammen mit dem Kohlenstoff, an den sie gebunden sind, einen gesättigten monocyclischen Ring aus 3, 4, 5, 6 oder 7 Atomen bilden, der gegebenenfalls 1 oder 2 Heteroatome, unabhängig ausgewählt aus O, S, N, enthält und gegebenenfalls durch R¹² hexasubstituiert ist,
jedes R¹² unabhängig ausgewählt ist aus der Gruppe, bestehend aus
(a) Wasserstoff,
(b) C₁₋₆-Alkyl,
(c) Phenyl oder monosubstituiertem Phenyl, wobei die Substituenten Halogen, C₁₋₆-Alkyl, C₁₋₆-Alkoxy, C₁₋₆-Alkylthio, CN oder CF₃ sein können,
(d) Benzyl oder monosubstituiertem Benzyl, wobei die Substituenten Halogen, C₁₋₆-Alkyl, C₁₋₆-Alkoxy, C₁₋₆-Alkylthio, CN oder CF₃ sein können,
(e) -CO₂-Alkyl,
(f) Halogen,
R⁵, R⁶ und R⁷ jeweils unabhängig ausgewählt sind aus der Gruppe, bestehend aus
(a) Wasserstoff und
(b) C₁₋₆-Alkyl,
oder R⁵ und R⁶ zusammen mit dem Kohlenstoff, an den sie gebunden sind, einen gesättigten monocyclischen Kohlenstoffring aus 3, 4, 5, 6 oder 7 Atomen bilden.

3. Eine Verbindung gemäß Anspruch 2 der Formel Ia 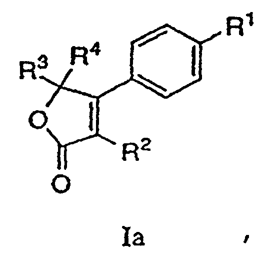 wobei:
R¹ ausgewählt ist aus der Gruppe, bestehend aus
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² ausgewählt ist aus der Gruppe, bestehend aus
(a) Cyclohexyl und
(b) mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₄-Alkoxy,
(4) C₁₋₄-Alkylthio,
(5) CN,
(6) CF₃,
(7) C₁₋₄-Alkyl,
(8) N₃,
(9) -CO₂H,
(10) -CO₂-C₁₋₄-Alkyl,
(11) -C(R⁵)(R⁶)-OH,
(12) -C(R⁵)(R⁶)-O-C₁₋₄-Alkyl und
(13) -C₁₋₆-Alkyl-CO₂-R⁵,
(14) Benzyloxy,
(15) -O-(C₁₋₄-Alkyl)-CO₂R⁵,
(16) -O-(C₁₋₄-Alkyl)-NR⁵R⁶,
R³ CH₂OR⁷, CN, CH₂CN oder C₁₋₆-Fluoralkyl ist,
R⁴ ist
(a) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl, oder
(b) C₁₋₆-Alkyl, CH₂OR⁷, CN, CH₂CN oder C₁₋₆-Fluoralkyl, oder R³ und R⁴ zusammen mit dem Kohlenstoff, an den sie gebunden sind, einen gesättigten monocyclischen Ring aus 3, 4, 5, 6 oder 7 Atomen bilden, der gegebenenfalls 1 oder 2 Heteroatome, unabhängig ausgewählt aus O, S, N, enthält und gegebenenfalls durch R¹² tetrasubstituiert ist,
jedes R¹² unabhängig ausgewählt ist aus der Gruppe, bestehend aus
(a) Wasserstoff,
(b) C₁₋₆-Alkyl,
(c) Phenyl oder monosubstituiertem Phenyl, wobei die Substituenten Halogen, C₁₋₆-Alkyl, C₁₋₆-Alkoxy, C₁₋₆-Alkylthio, CN oder CF₃ sein können,
(d) Benzyl oder monosubstituiertem Benzyl, wobei die Substituenten Halogen, C₁₋₆-Alkyl, C₁₋₆-Alkoxy, C₁₋₆-Alkylthio, CN oder CF₃ sein können,
(e) -CO₂-Alkyl,
(f) Halogen,
R⁵, R⁶ und R⁷ jeweils unabhängig ausgewählt sind aus der Gruppe, bestehend aus
(a) Wasserstoff und
(b) C₁₋₆-Alkyl,
oder R⁵ und R⁶ zusammen mit dem Kohlenstoff, an den sie gebunden sind, einen gesättigten monocyclischen Kohlenstoffring aus 3, 4, 5, 6 oder 7 Atomen bilden.

4. Eine Verbindung gemäß Anspruch 3 der Formel Ia 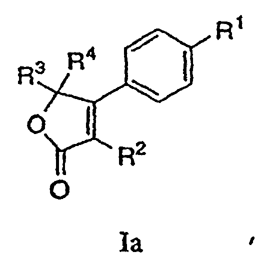 wobei:
R¹ ausgewählt ist aus der Gruppe, bestehend aus
(a) S(O)₂CH₃,
(b) S(O)₂NH₂,
(c) S(O)(NH)NH₂,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₄-Alkoxy,
(4) C₁₋₄-Alkylthio,
(5) CN,
(6) CF₃,
(7) C₁₋₄-Alkyl,
(8) N₃,
(9) -CO₂H,
(10) -CO₂-C₁₋₄-Alkyl,
(11) -C(R⁵) (R⁶)-OH,
(12) -C(R⁵)(R⁶)-O-C₁₋₄-Alkyl und
(13) -C₁₋₄-Alkyl-CO₂-R⁵,
R³ CH₂OR⁷, CN, CH₂CN oder C₁₋₄-Fluoralkyl ist,
R⁴ ist
(a) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Fluor, Chlor und Brom,
(3) C₁₋₄-Alkyl,
(4) C₁₋₄-Alkoxy,
(5) C₁₋₄-Alkylthio,
(6) CN,
(7) CF₃, oder
(b) C₁₋₄-Alkyl, CH₂OR⁷, CN, CH₂CN oder C₁₋₄-Fluoralkyl, oder R³ und R⁴ zusammen mit dem Kohlenstoff, an den sie gebunden sind, einen gesättigten monocyclischen Ring aus 3, 4, 5, 6 oder 7 Atomen bilden, der gegebenenfalls 1 oder 2 Heteroatome, unabhängig ausgewählt aus O, S, N, enthält und gegebenenfalls durch R¹² trisubstituiert ist,
jedes R¹² unabhängig ausgewählt ist aus der Gruppe, bestehend aus
(a) Wasserstoff,
(b) C₁₋₄-Alkyl,
(c) Phenyl oder monosubstituiertem Phenyl, wobei die Substituenten Halogen, C₁₋₄-Alkyl, C₁₋₄-Alkoxy, C₁₋₆-Alkylthio, CN oder CF₃ sein können,
(d) Benzyl oder monosubstituiertem Benzyl, wobei die Substituenten Halogen, C₁₋₄-Alkyl, C₁₋₄-Alkoxy, C₁₋₄-Alkylthio, CN oder CF₃ sein können,
(e) -CO₂-C₁₋₄-Alkyl,
(f) Halogen,
R⁵, R⁶ und R⁷ jeweils unabhängig ausgewählt sind aus der Gruppe, bestehend aus
(a) Wasserstoff und
(b) C₁₋₄-Alkyl,
oder R⁵ und R⁶ zusammen mit dem Kohlenstoff, an den sie gebunden sind, einen gesättigten monöcyclischen Kohlenstoffring aus 3, 4, 5, 6 oder 7 Atomen bilden.

5. Eine Verbindung gemäß Anspruch 4 der Formel Ia 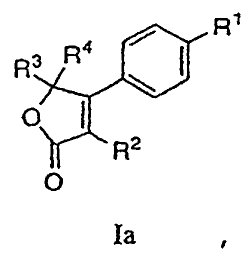 wobei:
R¹ ausgewählt ist aus der Gruppe, bestehend aus
(a) S(O)₂CH₃,
(b) S(O)₂NH₂,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₄-Alkoxy,
(4) C₁₋₄-Alkylthio,
(5) CN,
(6) CF₃,
(7) C₁₋₄-Alkyl,
(8) N₃ und
(9) -C(R⁵)(R⁶)-OH,
R³ CH₂OR⁷, CN, CH₂CN oder C₁₋₃-Fluoralkyl ist,
R⁴ ist
(a) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₄-Alkylthio,
(6) CN,
(7) CF₃, oder
(b) C₁₋₃-Alkyl, CH₂OR⁷, CN, CH₂CN oder C₁₋₃-Fluoralkyl,
R⁵, R⁶ und R⁷ jeweils unabhängig ausgewählt sind aus der Gruppe, bestehend aus
(a) Wasserstoff und
(b) C₁₋₃-Alkyl.

6. Eine Verbindung gemäß Anspruch 5 der Formel Ia 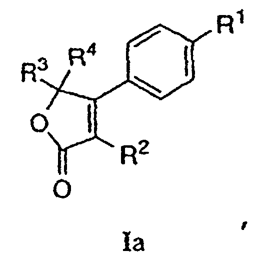 wobei:
R¹ ausgewählt ist aus der Gruppe, bestehend aus
(a) S(O)₂CH₃,
(b) S(O)₂NH₂,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1 Wasserstoff,
(2) Halogen, ausgewählt aus der Gruppe, bestehend aus Fluor, Chlor und Brom,
(3) C₁₋₃-Alkoxy,
(4) C₁₋₃-Alkylthio,
(5) CN und
(6) C₁₋₃-Alkyl,
R³ CH₂OR⁷, CN, CH₂CN oder C₁₋₃-Fluoralkyl ist,
R⁴ ist
(a) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₄-Alkylthio,
(6) CF₃, oder
(b) C₁₋₃-Alkyl, CH₂OR⁷, CN, CH₂CN oder C₁₋₃-Fluoralkyl, R⁵, R⁶ und R⁷ jeweils unabhängig ausgewählt sind aus der Gruppe, bestehend aus
(a) Wasserstoff und
(b) C₁₋₃-Alkyl.

7. Eine Verbindung gemäß Anspruch 6 der Formel Ia 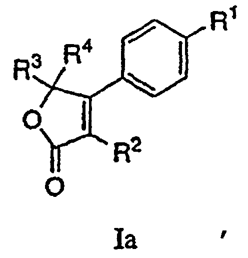 wobei:
R¹ ausgewählt ist aus der Gruppe, bestehend aus
(a) S(O)₂CH₃,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1) Wasserstoff,
(2) Halogen, ausgewählt aus der Gruppe, bestehend aus Fluor, Chlor und Brom,
(3) Methoxy,
(4) Methylthio,
(5) CN und
(6) Methyl und Ethyl,
R³ CH₂OR⁷, CN, CH₂CN oder Trifluormethyl ist,
R⁴ ist
(a) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Fluor, Chlor und Brom,
(3) Methyl und Ethyl,
(4) Methoxy,
(5) Methylthio,
(6) CF₃, oder
(b) C₁₋₃-Alkyl, CH₂OR⁷, CN, CH₂CN oder Trifluormethyl, R⁵, R⁶ und R⁷ jeweils unabhängig ausgewählt sind aus der Gruppe, bestehend aus
(a) Wasserstoff und
(b) Methyl.

8. Eine Verbindung gemäß Anspruch 2, wobei
R₂ ausgewählt ist aus der Gruppe, bestehend aus
(a) mono- oder disubstituiertem Heteroaryl, ausgewählt aus der Gruppe, bestehend aus
(1) Furanyl,
(2) Diazinyl, Triazinyl und Tetrazinyl,
(3) Imidazolyl,
(4) Isooxazolyl,
(5) Isothiazolyl,
(6) Oxadiazolyl,
(7) Oxazolyl,
(8) Pyrazolyl,
(9) Pyrrolyl,
(10) Thiadiazolyl,
(11) Thiazolyl,
(12) Thienyl,
(13) Triazolyl und
(14) Tetrazolyl,
wobei die Substituenten ausgewählt sind aus der Gruppe, bestehend aus
(1) Wasserstoff,
(2) Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵) (R⁶)-O-C₁₋₄-Alkyl,
(b) einem mono- oder disubstituierten Benzoheterocyclus, Benzocarbocyclus oder Heterocycloalkyl, ausgewählt aus der Gruppe, bestehend aus
(1) 2-Indolyl,
(2) 3-Indolyl,
(3) 1-Methyl-5-indolyl,
(4) 2-Benzofuranyl,
(5) 3-Benzofuranyl,
(6) 5-Benzofuranyl,
(7) 6-Benzofuranyl,
(8) 2-Benzothienyl,
(9) 3-Benzothienyl,
(10) 5-Benzothienyl,
(11) 6-Benzothienyl, 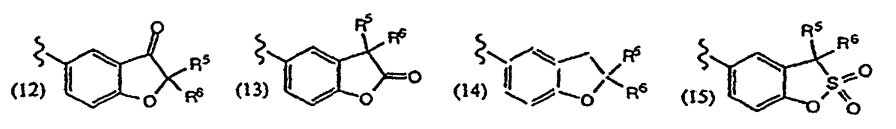 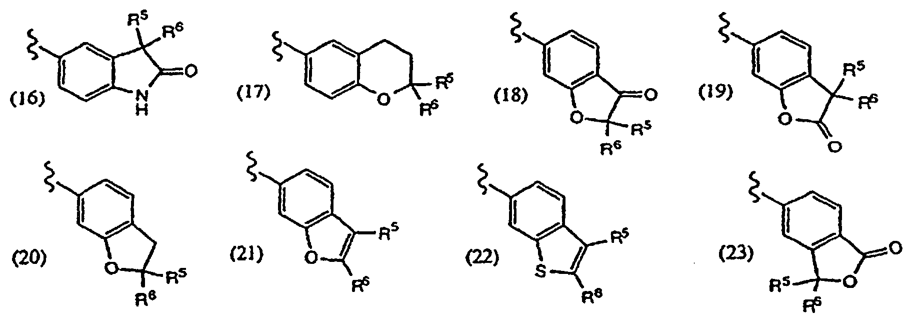 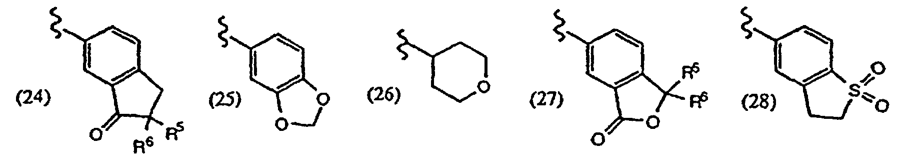 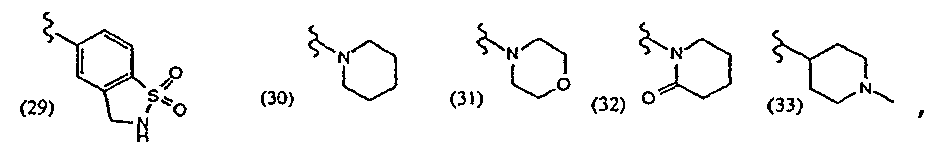
wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵)(R⁶)-OH und
(10) -C(R⁵)(R⁶)-O-C₁₋₄-Alkyl.

9. Eine Verbindung gemäß Anspruch 8, wobei
R² ein mono- oder disubstituiertes Heteroaryl ist, wobei das Heteroaryl ausgewählt ist aus der Gruppe, bestehend aus
(1) Furanyl,
(2) Diazinyl, Triazinyl und Tetrazinyl,
(3) Imidazolyl,
(4) Isooxazolyl,
(5) Isothiazolyl,
(6) Oxadiazolyl,
(7) Oxazolyl,
(8) Pyrazolyl,
(9) Pyrrolyl,
(10) Thiadiazolyl,
(11) Thiazolyl,
(12) Thienyl,
(13) Triazolyl und
(14) Tetrazolyl,
wobei die Substituenten ausgewählt sind aus der Gruppe, bestehend aus
(1) Wasserstoff,
(2) Fluor oder Chlor,
(3) C₁₋₃-Alkoxy,
(4) C₁₋₆-Alkylthio,
(5) CN,
(6) CF₃,
(7) C₁₋₃-Alkyl,
(8) -C(R⁵)(R⁶)-OH,
(9) -C(R⁵)(R⁶)-O-C₁₋₄-Alkyl.

10. Eine Verbindung gemäß Anspruch 9, wobei
R² ein mono- oder disubstituiertes Heteroaryl ist, wobei das Heteroaryl ausgewählt ist aus der Gruppe, bestehend aus
(1) 2-Furanyl,
(2) 3-Furanyl,
(3) 2-Thienyl,
(4) 3-Thienyl,
(5) 3-Isoxazolyl,
(6) 4-Isoxazolyl,
(7) 5-Isoxazolyl,
(8) 3-Isothiazolyl,
(9) 4-Isothiazolyl,
(10) 5-Isothiazolyl,
(11) 2-Oxazolyl,
(12) 4-Oxazolyl,
(13) 5-Oxazolyl,
(14) 2-Thiazolyl,
(15) 4-Thiazolyl,
(16) 5-Thiazolyl,
(17) 1,2,3-Thiadiazol-4-yl,
(18) 1,2,3-Thiadiazol-5-yl,
(19) 1,2,4-Thiadiazol-3-yl,
(20) 1,2,4-Thiadiazol-5-yl,
(21) 1,3,4-Thiadiazol-2-yl,
(22) 1,2,5-Thiadiazol-3-yl,
(23) 1,2,3-Oxadiazol-4-yl,
(24) 1,2,3-Oxadiazol-5-yl,
(25) 1,2,4-Oxadiazol-3-yl,
(26) 1,2,4-Oxadiazol-5-yl,
(27) 1,3,4-Oxadiazol-2-yl,
(28) 1,2,5-Oxadiazol-3-yl,
(29) Pyrazol-4-yl,
(30) Pyrazol-5-yl,
(31) 1,2,3-Triadiazol-4-yl,
(32) 1,2,3-Triadiazol-5-yl,
(33) 1,2,4-Triadiazol-3-yl,
(34) 1,2,4-Triadiazol-5-yl,
(35) 1,2-Diazinyl,
(36) 1,3-Diazinyl,
(37) 1,4-Diazinyl,
(38) 1,2,3,4-Tetrazin-5-yl,
(39) 1,2,4,5-Tetrazin-4-yl,
(40) 1,3,4,5-Tetrazin-2-yl und
(41) 1,2,3,5-Tetrazin-4-yl.

11. Eine Verbindung gemäß Anspruch 10, wobei
R² ein mono- oder disubstituiertes Heteroaryl ist, wobei Heteroaryl ausgewählt ist aus der Gruppe, bestehend aus
(1) 3-Isoxazolyl,
(2) 4-Isoxazolyl,
(3) 5-Isoxazolyl,
(4) 3-Isothiazolyl,
(5) 4-Isothiazolyl,
(6) 5-Isothiazolyl,
(7) 2-Oxazolyl,
(8) 4-Oxazolyl,
(9) 5-Oxazolyl,
(10) 2-Thiazolyl,
(11) 4-Thiazolyl,
(12) 5-Thiazolyl,
(13) 1,2,3-Thiadiazol-4-yl,
(14) 1,2,3-Thiadiazol-5-yl,
(15) 1,2,4-Thiadiazol-3-yl,
(16) 1,2,4-Thiadiazol-5-yl,
(17) 1,3,4-Thiadiazol-2-yl,
(18) 1,2,5-Thiadiazol-3-yl,
(19) 1,2,3-Oxadiazol-4-yl,
(20) 1,2,3-Oxadiazol-5-yl,
(21) 1,2,4-Oxadiazol-3-yl,
(22) 1,2,4-Oxadiazol-5-yl,
(23) 1,3,4-Oxadiazol-2-yl,
(24) 1,2,5-Oxadiazol-3-yl,
(25) 1,2-Diazinyl,
(26) 1,3-Diazinyl und
(27) 1,4-Diazinyl.

12. Eine Verbindung gemäß Anspruch 11, wobei das Heteroaryl ausgewählt ist aus der Gruppe, bestehend aus
(1) 3-Isothiazolyl,
(2) 4-Isothiazolyl,
(3) 5-Isothiazolyl,
(4) 2-Oxazolyl,
(5) 4-Oxazolyl,
(6) 5-Oxazolyl,
(7) 2-Thiazolyl,
(8) 4-Thiazolyl,
(9) 5-Thiazolyl,
(10) 1,2-Diazinyl,
(11) 1,3-Diazinyl und
(12) 1,4-Diazinyl, und
wobei die Substituenten ausgewählt sind aus der Gruppe, bestehend aus
(1) Wasserstoff,
(2) Fluor oder Chlor,
(3) C₁₋₃-Alkoxy,
(4) C₁₋₃-Alkylthio,
(5) CN,
(6) C₁₋₃-Alkyl und
(7) -C(R⁵) (R⁶)-OH,
R³ CH₂OR⁷, CN, CH₂CN oder C₁₋₃-Fluoralkyl ist,
R⁴ ist
(a) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₄-Alkylthio,
(6) CN,
(7) CF₃, oder
(b) C₁₋₃-Alkyl, CH₂OR⁷, CN, CH₂CN oder C₁₋₃-Fluoralkyl, R⁵ und R⁶ jeweils unabhängig Wasserstoff, Methyl oder Ethyl sind.

13. Eine Verbindung gemäß Anspruch 1 der Formel Ib 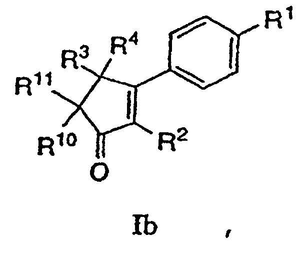 wobei
R¹ ausgewählt ist aus der Gruppe, bestehend aus
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² ausgewählt ist aus der Gruppe, bestehend aus Cyclohexyl und
mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₆-Alkoxy,
(4) C₁₋₆-Alkylthio,
(5) CN,
(6) CF₃,
(7) C₁₋₆-Alkyl,
(8) N₃,
(9) -CO₂H,
(10) -CO₂-C₁₋₄-Alkyl,
(11) -C(R⁵)(R⁶)-OH,
(12) -C(R⁵)(R⁶)-O-C₁₋₄-Alkyl und
(13) -C₁₋₆-Alkyl-CO₂-R⁵,
(14) Benzyloxy,
(15) -O-(C₁₋₆-Alkyl)-CO₂R⁵,
(16) -O-(C₁₋₆-Alkyl)-NR⁵R⁶,
R³ CH₂OR⁷, CN, CH₂CN oder C₁₋₆-Fluoralkyl ist,
R⁴ ist
(a) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R⁵) (R⁶)-OH und
(10) -C(R⁵) (R⁶)-O-C₁₋₄-Alkyl, oder
(b) C₁₋₆-Alkyl, CH₂OR⁷, CN, CH₂CN oder C₁₋₆-Fluoralkyl, oder R³ und R⁴ zusammen mit dem Kohlenstoff, an den sie gebunden sind, einen gesättigten monocyclischen Ring aus 3, 4, 5, 6 oder 7 Atomen bilden, der gegebenenfalls 1 oder 2 Heteroatome, unabhängig ausgewählt aus O, S, N, enthält und gegebenenfalls durch R¹² tetrasubstituiert ist,
jedes R¹² unabhängig ausgewählt ist aus der Gruppe, bestehend aus
(a) Wasserstoff,
(b) C₁₋₆-Alkyl,
(c) Phenyl oder monosubstituiertem Phenyl, wobei die Substituenten Halogen, C₁₋₆-Alkyl, C₁₋₆-Alkoxy, C₁₋₆-Alkylthio, CN oder CF₃ sein können,
(d) Benzyl oder monosubstituiertem Benzyl, wobei die Substituenten Halogen, C₁₋₆-Alkyl, C₁₋₆-Alkoxy, C₁₋₆-Alkylthio, CN oder CF₃ sein können,
(e) -CO₂-Alkyl,
(f) Halogen,
R⁵, R⁶ und R⁷ jeweils unabhängig ausgewählt sind aus der Gruppe, bestehend aus
(a) Wasserstoff und
(b) C₁₋₆-Alkyl,
oder R⁵ und R⁶ zusammen mit dem Kohlenstoff, an den sie gebunden sind, einen gesättigten monocyclischen Kohlenstoffring aus 3, 4, 5, 6 oder 7 Atomen bilden,
R¹⁰ und R¹¹ unabhängig
(a) Wasserstoff,
(b) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(8) N₃,
(9) -C(R¹³)(R¹⁴)-OH und
(10) -C(R¹³)(R¹⁴)-O-C₁₋₄-Alkyl, oder
(c) C₁₋₇-Alkyl, CH₂OR⁷, CN, CH₂CN, C₁₋₆-Fluoralkyl sind, oder R¹⁰ und R¹¹ zusammen mit dem Kohlenstoff, an den sie gebunden sind, einen gesättigten monocyclischen Kohlenstoffring aus 3, 4, 5, 6 oder 7 Atomen bilden,
R¹³ und R¹⁴ unabhängig ausgewählt sind aus der Gruppe, bestehend aus:
(a) Wasserstoff,
(b) C₁₋₆-Alkyl, oder
R¹³ und R¹⁴ zusammen ein doppelt gebundenes O oder S bilden.

14. Eine Verbindung gemäß Anspruch 13 der Formel Ib 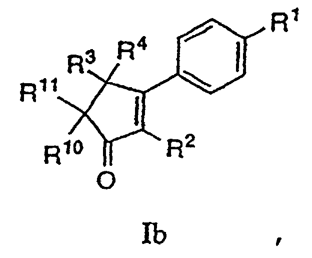 wobei:
R¹ ausgewählt ist aus der Gruppe, bestehend aus
(a) S(O)₂CH₃,
(b) S(O)₂NH₂,
(c) S(O)(NH)NH₂,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₆-Alkoxy,
(4) C₁₋₄-Alkylthio,
(5) CN,
(6) CF₃,
(7) C₁₋₄-Alkyl,
(8) N₃,
(9) -CO₂H,
(10) -CO₂-C₁₋₄-Alkyl,
(11) -C(R⁵) (R⁶)-OH,
(12) -C(R⁵) (R⁶)-O-C₁₋₄-Alkyl und
(13) -C₁₋₄-Alkyl-CO₂-R⁵,
R³ CH₂OR⁷, CN, CH₂CN oder C₁₋₆-Fluoralkyl ist,
R⁴ ist
(a) substituiertes Phenyl oder substituiertes Heteroaryl, wobei die Substituenten ausgewählt sind aus der Gruppe, bestehend aus
(1) Wasserstoff,
(2) Fluor, Chlor und Brom,
(3) C₁₋₄-Alkyl,
(4) C₁₋₄-Alkoxy,
(5) C₁₋₄-Alkylthio,
(6) CN,
(7) CF₃, oder
(b) C₁₋₄-Alkyl, CH₂OR⁷, CN, CH₂CN oder C₁₋₄-Fluoralkyl, oder R³ und R⁴ zusammen mit dem Kohlenstoff, an den sie gebunden sind, einen gesättigten monocyclischen Ring aus 3, 4, 5, 6 oder 7 Atomen bilden, der gegebenenfalls 1 oder 2 Heteroatome, unabhängig ausgewählt aus O, S, N, enthält und gegebenenfalls durch R¹² trisubstituiert ist,
jedes R¹² unabhängig ausgewählt ist aus der Gruppe, bestehend aus
(a) Wasserstoff,
(b) C₁₋₄-Alkyl,
(c) Phenyl oder monosubstituiertem Phenyl, wobei die Substituenten Halogen, C₁₋₄-Alkyl, C₁₋₄-Alkoxy, C₁₋₄-Alkylthio, CN oder CF₃ sein können,
(d) Benzyl oder monosubstituiertem Benzyl, wobei die Substituenten Halogen, C₁₋₄-Alkyl, C₁₋₄-Alkoxy, C₁₋₄-Alkylthio, CN oder CF₃ sein können,
(e) -CO₂-C₁₋₄-Alkyl,
(f) Halogen,
R⁵, R⁶ und R⁷ jeweils unabhängig ausgewählt sind aus der Gruppe, bestehend aus
(a) Wasserstoff und
(b) C₁₋₄-Alkyl,
oder R⁵ und R⁶ zusammen mit dem Kohlenstoff, an den sie gebunden sind, einen gesättigten monocyclischen Kohlenstoffring aus 3, 4, 5, 6 oder 7 Atomen bilden,
R¹⁰ und R¹¹ unabhängig
(a) Wasserstoff,
(b) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₆-Alkyl,
(4) C₁₋₆-Alkoxy,
(5) C₁₋₆-Alkylthio,
(6) CN,
(7) CF₃,
(c) C₁₋₄-Alkyl, CH₂OR⁷, CN, CH₂CN oder C₁₋₄-Fluoralkyl sind, oder
R¹⁰ und R¹¹ zusammen mit dem Kohlenstoff, an den sie gebunden sind, einen gesättigten monocyclischen Kohlenstoffring aus 3, 4, 5, 6 oder 7 Atomen bilden,
R¹³ und R¹⁴ unabhängig ausgewählt sind aus der Gruppe, bestehend aus:
(a) Wasserstoff,
(b) C₁₋₄-Alkyl.

15. Eine Verbindung gemäß Anspruch 14 der Formel Ib 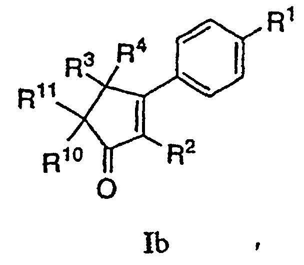 wobei:
R¹ ausgewählt ist aus der Gruppe, bestehend aus
(a) S(O)₂CH₃,
(b) S(O)₂NH₂,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1) Wasserstoff,
(2) Halogen,
(3) C₁₋₄-Alkoxy,
(4) C₁₋₄-Alkylthio,
(5) CN,
(6) CF₃,
(7) C₁₋₄-Alkyl,
(8) CO₂H und
(9) -C(R⁵)(R⁶)-OH,
R³ CH₂OR⁷, CN, CH₂CN oder C₁₋₃-Fluoralkyl ist,
R⁴ ist
(a) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₄-Alkylthio,
(6) CN,
(7) CF₃, oder
(b) C₁₋₃-Alkyl, CH₂OR⁷, CN, CH₂CN oder C₁₋₃-Fluoralkyl,
R⁵, R⁶ und R⁷ jeweils unabhängig ausgewählt sind aus der Gruppe, bestehend aus
(a) Wasserstoff und
(b) C₁₋₃-Alkyl,
R¹⁰ und R¹¹ unabhängig
(a) Wasserstoff,
(b) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₃-Alkylthio,
(6) CN,
(7) CF₃,
(c) C₁₋₃-Alkyl, CH₂OR⁷, CN, CH₂CN, C₁₋₄-Fluoralkyl sind, R¹³ und R¹⁴ unabhängig ausgewählt sind aus der Gruppe, bestehend aus:
(a) Wasserstoff,
(b) C₁₋₃-Alkyl.

16. Eine Verbindung gemäß Anspruch 15 der Formel Ib 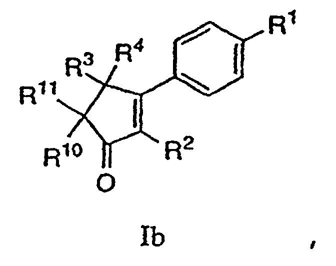 wobei:
R¹ ausgewählt ist aus der Gruppe, bestehend aus
(a) S(O)₂CH₃,
(b) S(O)₂NH₂,
R² ausgewählt ist aus der Gruppe, bestehend aus mono-, di- oder trisubstituiertem Phenyl oder Naphthyl, wobei der Substituent ausgewählt ist aus der Gruppe, bestehend aus
(1) Wasserstoff,
(2) Halogen, ausgewählt aus der Gruppe, bestehend aus Fluor, Chlor und Brom,
(3) C₁₋₃-Alkoxy,
(4) C₁₋₃-Alkylthio,
(5) CN und
(6) C₁₋₃-Alkyl,
R³ CH₂OR⁷, CN, CH₂CN oder C₁₋₃-Fluoralkyl ist,
R⁴ ist
(a) substituiertes Phenyl oder substituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₄-Alkylthio,
(6) CF₃, oder
(b) C₁₋₃-Alkyl, CH₂OR⁷, CN, CH₂CN oder C₁₋₃-Fluoralkyl,
R⁵, R⁶ und R⁷ jeweils unabhängig ausgewählt sind aus der Gruppe, bestehend aus
(a) Wasserstoff und
(b) C₁₋₃-Alkyl,
R¹⁰ und R¹¹ unabhängig
(a) Wasserstoff,
(b) ein mono- oder disubstituiertes Phenyl oder ein monooder disubstituiertes Heteroaryl, wobei die Substituenten ausgewählt sind aus der Gruppe, bestehend aus
(1) Wasserstoff,
(2) Halogen, einschließlich Fluor, Chlor, Brom und Iod,
(3) C₁₋₃-Alkyl,
(4) C₁₋₃-Alkoxy,
(5) C₁₋₃-Alkylthio,
(6) CF₃,
(c) C₁₋₃-Alkyl, CH₂OR⁷, CN, CH₂CN oder C₁₋₄-Fluoralkyl sind,
R¹³ und R¹⁴ unabhängig ausgewählt sind aus der Gruppe, bestehend aus:
(a) Wasserstoff,
(b) C₁₋₃-Alkyl.

17. Eine Verbindung, ausgewählt aus der Gruppe, bestehend aus
(a) 5,5-Dimethyl-4-(4-(methylsulfonyl)phenyl)-3-(4-methoxyphenyl)-2-(5H)-furanon,
(b) 5,5-Dimethyl-3-(3-methoxyphenyl)-4-(4-(methylsulfonyl)-phenyl)-2-(5H)-furanon,
(c) 5,5-Dimethyl-3-(4-isopropylphenyl)-4-(4-(methylsulfonyl)-phenyl)-2-(5H)-furanon,
(d) 5,5-Dimethyl-4-(4-(methylsulfonyl)phenyl)-3-phenyl-2-(5H)-furanon,
(e) 3-(Benzo[1,3]dioxol-5-yl)-5,5-dimethyl-4-(4-(methylsulfonyl)phenyl)-2-(5H)-furanon,
(f) 5,5-Dimethyl-3-(4-methylphenyl)-4-(4-(methylsulfonyl)-phenyl)-2-(5H)-furanon,
(g) 5,5-Dimethyl-4-(4-(methylsulfonyl)phenyl)-3-(4-trifluormethylphenyl)-2-(5H)-furanon,
(h) 5,5-Dimethyl-3-(3-methylphenyl)-4-(4-(methylsulfonyl)-phenyl)-2-(5H)-furanon,
(i) 5,5-Dimethyl-3-cyclohexyl-4-(4-(methylsulfonyl)phenyl)-2-(5H)-furanon,
(j) 4-(4-(Methylsulfonyl)phenyl)-3-phenyl-1-oxaspiro[4,4]non-3-en-2-on,
(k) 5,5-Dimethyl-3-(4-(methylsulfonyl)phenyl)-2-phenylcyclopent-2-enon,
(l) 4,4-Dimethyl-3-(4-(methylsulfonyl)phenyl)-2-phenylcyclopent-2-enon,
(m) 7-(4-(Methylsulfonyl)phenyl)-6-phenyl-4-oxaspiro[2.4]-hept-6-en-5-on und
(n) 5,5-Bis(fluormethyl)-3-phenyl-4-(4-(methylsulfonyl)-phenyl)-2-(5H)-furanon.

18. Eine Verbindung gemäß Anspruch 1, ausgewählt aus
(b) 3-(3,5-Difluorphenyl)-4-(4-methylsulfonylphenyl)-1-oxaspiro[4.4]non-3-en-2-on,
(d) 4-(4-Methylsulfonylphenyl)-3-phenyl-5,5-bistrifluormethyl-5H-furanon,
(e) 8- (4-Methylsulfonylphenyl)-7-phenyl-5-oxaspiro[3.4]oct-7-en-6-on,
(f) 6-(3-Fluorphenyl)-7-(4-(methylsulfonyl)phenyl)-4-oxaspiro[2.4]hept-6-en-5-on,
(g) 7-(4-Methylsulfonylphenyl)-6-naphthalin-2-yl-4-oxaspiro-[2.4]hept-6-en-5-on,
(h) 4-(6-Naphthalin-2-yl-5-oxo-4-oxaspiro[2.4]hept-6-en-7-yl)benzolsulfonamid,
(i) 7-(4-Fluorphenyl)-8-(4-methylsulfonylphenyl)-5-oxaspiro-[3.4]oct-7-en-6-on,
(j) 7-(3-Fluorphenyl)-8-(4-methylsulfonylphenyl) -5-oxaspiro-[3.4]oct-7-en-6-on,
(k) 7-(3,4-Difluorphenyl)-8-(4-methylsulfonylphenyl)-5-oxaspiro[3.4]oct-7-en-6-on,
(l) 4- (6-Oxo-7-phenyl-5-oxaspiro[3.4]oct-7-en-8-yl)benzolsulfonamid,
(m) 4-[7-(4-Fluorphenyl)-6-oxo-5-oxaspiro[3.4]oct-7-en-8-yl]-benzolsulfonamid,
(n) 4- [7-(3-Fluorphenyl)-6-oxo-5-oxaspiro[3.4]oct-7-en-8-yl]-benzolsulfonamid,
(o) 4-[7-(3,4-Difluorphenyl)-6-oxo-5-oxaspiro[3.4]oct-7-en-8-yl]benzolsulfonamid,
(p) 3-(4-Fluorphenyl)-4-(4-(methylsulfonyl)phenyl)-1-oxaspiro[4.4]non-3-en-2-on,
(q) 3-(3-Fluorphenyl)-4-(4-(methylsulfonyl)phenyl)-1-oxaspiro[4.4]non-3-en-2-on,
(r) 3-(3,4-Difluorphenyl)-4-(4-(methylsulfonyl)phenyl)-1-oxaspiro[4.4]non-3-en-2-on,
(s) 3-(3-Chlor-4-fluorphenyl)-4-(4-(methylsulfonyl)phenyl)-1-oxaspiro[4.4]non-3-en-2-on,
(t) 4-(2-Oxo-3-phenyl-1-oxaspiro[4.4]non-3-en-4-yl)benzolsulfonamid,
(u) 4-[3-(4-Fluorphenyl)-2-oxo-1-oxaspiro[4.4]non-3-en-4-yl)]benzolsulfonamid,
(v) 4-[3-(3-Fluorphenyl)-2-oxo-1-oxaspiro[4.4]non-3-en-4-yl)]benzolsulfonamid,
(w) 4-[3-(3,4-Difluorphenyl)-2-oxo-1-oxaspiro[4.4]non-3-en-4-yl)]benzolsulfonamid,
(x) 4-[3-(3-Chlor-4-fluorphenyl)-2-oxo-1-oxaspiro[4.4]non-3-en-4-yl]benzolsulfonamid,
(z) 4-(2-Oxo-3-phenyl-1-oxaspiro[4.5]dec-3-en-4-yl)benzolsulfonamid und
(aa) 4-[3-(4-Fluorphenyl)-2-oxo-1-oxaspiro[4.5]dec-3-en-4-yl)benzolsulfonamid.

19. Eine pharmazeutische Zusammensetzung, die enthält:
eine nichttoxische, therapeutisch wirksame Menge einer Verbindung gemäß Anspruch 1, 2, 3, 4, 5, 6, 7, 8, 9, 10, 11, 12, 13, 14, 15, 16, 17 oder 18 oder eines pharmazeutisch annehmbaren Salzes davon und einen pharmazeutisch annehmbaren Träger.

20. Ein pharmazeutisch annehmbares Salz einer Verbindung der Formel in Anspruch 1, 2, 3, 4, 5, 6, 7, 8, 9, 10, 11, 12, 13, 14, 15 oder 16.

21. Eine wie in Anspruch 1, 2, 3, 4, 5, 6, 7, 8, 9, 10, 11, 12, 13, 14, 15, 16, 17 oder 18 definierte Verbindung oder ein pharmazeutisch annehmbares Salz davon zur Verwendung bei der Behandlung einer Entzündungserkrankung, die der Behandlung mit einem nichtsteroidalen Antiphlogistikum zugänglich ist.

22. Die Verwendung einer Verbindung nach Anspruch 1, 2, 3, 4, 5, 6, 7, 8, 9, 10, 11, 12, 13, 14, 15, 16, 17 oder 18 oder eines pharmazeutisch annehmbaren Salzes davon bei der Herstellung eines Medikaments zur Behandlung cyclooxygenasevermittelter Erkrankungen.

## Revendications

1. Composé de formule I 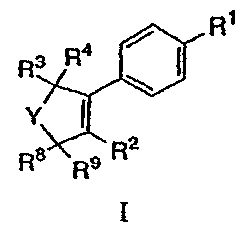 ou un de ses sels pharmaceutiquement acceptables
où
Y est choisi dans le groupe constitué par
(a) C(R¹⁰)(R¹¹),
(b) l'oxygène,
(c) le soufre,
R¹ est choisi dans le groupe constitué par
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² est choisi dans le groupe constitué par
(a) alkyle en C₁₋₆,
(b) cycloalkyle en C₃₋₇,
(c) phényle ou naphtyle mono-, di- ou trisubstitués dans lesquels le substituant est choisi dans le groupe constitué par
(1) hydrogène,
(2) halogéno,
(3) alcoxy en C₁₋₆,
(4) alkylthio en C₁₋₆,
(5) CN,
(6) CF₃,
(7) alkyle en C₁₋₆,
(8) N₃,
(9) -CO₂H,
(10) -CO₂-(alkyle en C₁₋₄)
(11) -C(R⁵)(R⁶)-OH,
(12) -C(R⁵)(R⁶)-O(alkyle en C₁₋₄) et
(13) -(alkyl en C₁₋₆)-CO₂R⁵,
(14) benzyloxy,
(15) -O-(alkyl en C₁₋₆)-CO₂R⁵,
(16) -O-(alkyl en C₁₋₆)-NR⁵R⁶ ;
(d) hétéroaryle mono-, di- ou trisubstitué où le reste hétéroaryle est un cycle aromatique monocyclique à 5 atomes, ledit cycle ayant un hétéroatome S, O ou N et éventuellement 1, 2 ou 3 atomes N supplémentaires ; ou le reste hétéroaryle est un cycle monocyclique à 6 atomes, ledit cycle ayant un hétéroatome N et éventuellement 1, 2, 3 ou 4 atomes N supplémentaires, lesdits substituants étant choisis dans le groupe constitué par
(1) hydrogène,
(2) halogéno, incluant fluoro, chloro, bromo et iodo,
(3) alkyle en C₁₋₆
(4) alcoxy en C₁₋₆,
(5) alkylthio en C₁₋₆,
(6) CN,
(7) CF₃,
(8) N₃,
(9) ―C(R⁵)(R⁶)-OH et
(10) ―C(R⁵)(R⁶)-O(alkyle en C₁₋₄);
(e) un benzohétérocycle mono- ou disubstitué dans lequel le reste hétérocycle est un cycle à 5, 6 ou 7 côtés qui peut contenir 1 ou 2 hétéroatomes choisis de façon indépendante parmi O, S ou N et qui peut contenir un groupe carbonyle ou un groupe sulfonyle ; lesdits substituants étant choisis dans le groupe constitué par
(1) hydrogène,
(2) halogéno, incluant fluoro, chloro, bromo et iodo,
(3) alkyle en C₁₋₆
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ;
(f) un groupe hétérocycloalkyle à 5 à 7 côtés contenant 1 ou 2 hétéroatomes choisis parmi O, S ou N et éventuellement contenant un groupe carbonyle ou un groupe sulfonyle,
(g) un benzocarbocycle mono- ou disubstitué dans lequel le reste carbocycle est un cycle à 5, 6 ou 7 côtés qui peut contenir éventuellement un groupe carbonyle, lesdits substituants étant choisis dans le groupe constitué par
(1) hydrogène,
(2) halogéno, incluant fluoro, chloro, bromo et iodo,
(3) alkyle en C₁₋₆
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ;
R³ est un phényle mono- ou disubstitué, un benzyle mono- ou disubstitué, un hétéroaryle mono- ou disubstitué, un hétéroarylméthyle mono- ou disubstitué, les substituants étant choisis parmi
(1) hydrogène,
(2) halogéno, incluant fluoro, chloro, bromo et iodo,
(3) alkyle en C₁₋₆
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄);
alkyle en C₁₋₇, CH₂OR⁷, CN, CH₂CN, fluoro(alkyle en C₁₋₆), F ou CONR⁷₂, si Y est C(R¹⁰)(R¹¹) ou
R³ est un phényle mono- ou disubstitué, un benzyle mono- ou disubstitué, un hétéroaryle mono- ou disubstitué, un hétéroarylméthyle mono- ou disubstitué, les substituants étant choisis parmi
(1) hydrogène,
(2) halogéno, incluant fluoro, chloro, bromo et iodo,
(3) alkyle en C₁₋₆
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ;
CH₂OR⁷, CN, CH₂CN, fluoro(alkyle en C₁₋₆), F ou CONR⁷₂, si Y est O ou S ;
R⁴ est
(a) un phényle mono- ou disubstitué, un benzyle mono- ou disubstitué, un hétéroaryle mono- ou disubstitué ou un hétéroarylméthyle mono- ou disubstitué comme défini ci-dessus, lesdits substituants étant choisis dans le groupe constitué par
(1) hydrogène,
(2) halogéno, incluant fluoro, chloro, bromo et iodo,
(3) alkyle en C₁₋₆
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄);
(b) alkyle en C₁₋₇, CH₂OR⁷, CN, CH₂CN, fluoro(alkyle en C₁₋₆), F ou CONR⁷₂ ou
R³ et R⁴ forment ensemble avec l'atome de carbone auquel ils sont attachés un cycle monocyclique saturé à 3, 4, 5, 6 ou 7 atomes, contenant éventuellement 1 ou 2 hétéroatomes choisis de façon indépendante parmi O, S, N et éventuellement hexasubstitué par R¹² et éventuellement contenant un groupe carbonyle ou sulfonyle ;
chaque R¹² est, de façon indépendante, choisi dans le groupe constitué par
(a) hydrogène
(b) alkyle en C₁₋₆,
(c) phényle ou phényle monosubstitué, les substituants pouvant être halogéno, alkyle en C₁₋₆, alcoxy en C₁₋₆, alkylthio en C₁₋₆, CN ou CF₃,
(d) benzyle ou benzyle monosubstitué, les substituants pouvant être halogéno, alkyle en C₁₋₆, alcoxy en C₁₋₆, alkylthio en C₁₋₆, CN ou CF₃,
(e) -CO₂-alkyle,
(f) halogéno,
R⁵, R⁶ et R⁷ sont chacun, de façon indépendante, choisis dans le groupe constitué par
(a) hydrogène et
(b) alkyle en C₁₋₆,
ou R⁵ et R⁶ forment, ensemble avec l'atome de carbone auquel ils sont rattachés un cycle carboné monocyclique saturé à 3, 4, 5, 6 ou 7 atomes ;
R⁸ et R⁹ sont choisis, de façon indépendante, dans le groupe constitué par
(a) hydrogène et
(b) alkyle en C₁₋₇, ou
R⁸ et R⁹ forment ensemble un O ou un S lié par double liaison ;
R¹⁰ et R¹¹ sont de façon indépendante
(a) un hydrogène,
(b) un phényle mono- ou disubstitué, un benzyle mono- ou disubstitué, un hétéroaryle mono- ou disubstitué ou un hétéroarylméthyle mono- ou disubstitué, lesdits substituants étant choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R¹³)(R¹⁴)-OH et
(10) -C(R¹³)(R¹⁴)-O(alkyle en C₁₋₄) ou
(c) alkyle en C₁₋₇, CH₂OR⁷, CN, CH₂CN, fluoro(alkyle en C₁₋₆), F ou CONR⁷₂ ou
(d) R¹⁰ et R¹¹ forment ensemble un O lié par une double liaison ou R¹⁰ et R¹¹ forment ensemble avec l'atome de carbone auquel ils sont attachés un cycle carboné monocyclique saturé à 3, 4, 5, 6 où 7 atomes ;
R¹³ et R¹⁴ sont, de façon indépendante, choisis dans le groupe constitué par :
(a) hydrogène et
(b) alkyle en C₁₋₇, ou
R¹³ et R¹⁴ forment ensemble un O ou un S lié par une double liaison.

2. Composé selon la revendication 1, de formule la 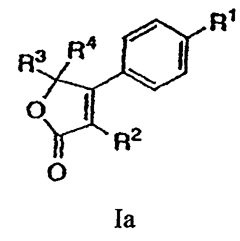 dans laquelle
R¹ est choisi dans le groupe constitué par
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² est choisi dans le groupe constitué par
(a) alkyle en C₁₋₆,
(b) cycloalkyle en C₃, C₄, C₅ ou C₆,
(c) phényle ou naphtyle mono-, di- ou trisubstitués dans lesquels le substituant est choisi dans le groupe constitué par
(1) hydrogène,
(2) halogéno,
(3) alcoxy en C₁₋₆,
(4) alkylthio en C₁₋₆,
(5) CN,
(6) CF₃,
(7) alkyle en C₁₋₆,
(8) N₃,
(9) -CO₂H,
(10) -CO₂-(alkyle en C₁₋₄)
(11) -C(R⁵)(R⁶)-OH,
(12) -(R⁵)(R⁶)-O(alkyle en C₁₋₄) et
(13) -(alkyl en C₁₋₆)-CO₂R⁵,
(14) benzyloxy,
(15) -O-(alkyl en C₁₋₆)-CO₂R⁵,
(16) -O-(alkyl en C₁₋₆)-NR⁵R⁶
(d) hétéroaryle mono- ou disubstitué choisi dans le groupe constitué par
(1) furanyle,
(2) diazinyle, triazinyle et tétrazinyle,
(3) imidazolyle,
(4) isooxazolyle,
(5) isothiazolyle,
(6) oxadiazolyle,
(7) oxazolyle,
(8) pyrazolyle,
(9) pyrrolyle,
(10) thiadiazolyle,
(11) thiazolyle,
(12) thiényle,
(13) triazolyle et
(14) tétrazolyle,
où lesdits substituants sont choisis dans le groupe constitué par
(1) hydrogène,
(2) fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄);
(e) un benzohétérocycle, un benzocarbocycle ou un hétérocycloalkyle mono- ou disubstitués choisis dans le groupe constitué par
(1) 2-indolyle,
(2) 3-indolyle,
(3) 1-méthyl-5-indolyle,
(4) 2-benzofuranyle,
(5) 3-benzofuranyle,
(6) 5-benzofuranyle,
(7) 6-benzofuranyle,
(8) 2-benzothiényle,
(9) 3-benzothiényle,
(10) 5-benzothiényle,
(11) 6-benzothiényle, 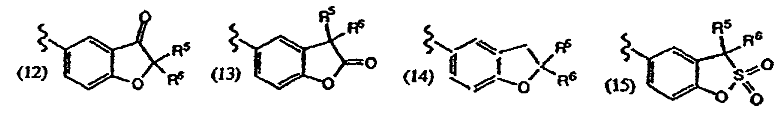 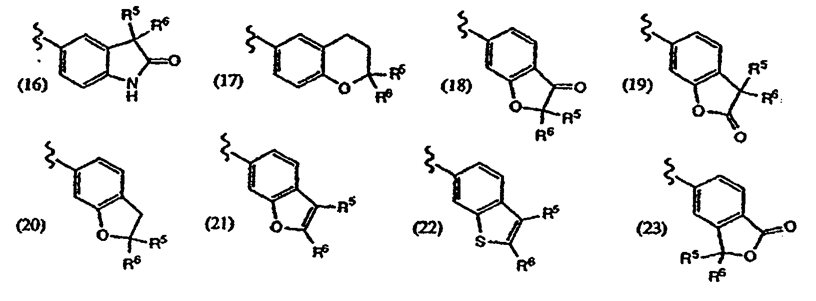 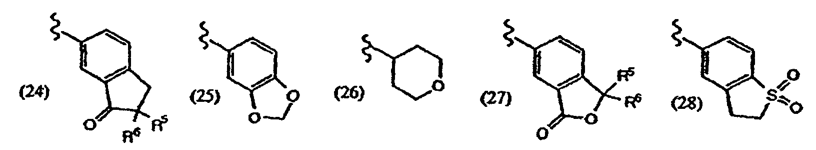 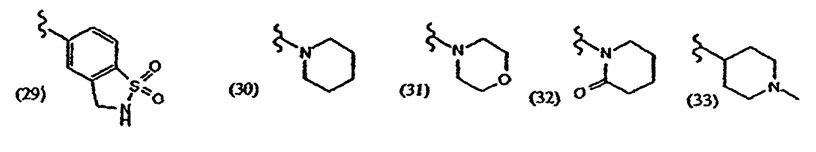
où lesdits substituants sont choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ;
R³ est CH₂OR⁷, CN, CH₂CN ou fluoro(alkyle en C₁₋₆) ;
R⁴ est
(a) un phényle mono- ou disubstitué ou un hétéroaryle mono- ou disubstitué, lesdits substituants étant choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ou;
(b) alkyle en C₁₋₆, CH₂OR⁷, CN, CH₂CN ou fluoro(alkyle en C₁₋₆) ou
R³ et R⁴ forment ensemble avec l'atome de carbone auquel ils sont attachés un cycle monocyclique saturé à 3, 4, 5, 6 ou 7 atomes, contenant éventuellement 1 ou 2 hétéroatomes choisis de façon indépendante parmi O, S, N et éventuellement hexasubstitué par R¹²,
chaque R¹² est, de façon indépendante, choisi dans le groupe constitué par
(a) hydrogène,
(b) alkyle en C₁₋₆,
(c) phényle ou phényle monosubstitué, les substituants pouvant être halogéno, alkyle en C₁₋₆, alcoxy en C₁₋₆, alkylthio en C₁₋₆, CN ou CF₃,
(d) benzyle ou benzyle monosubstitué, les substituants pouvant être halogéno, alkyle en C₁₋₆, alcoxy en C₁₋₆, alkylthio en C₁₋₆, CN ou CF₃,
(e) -CO₂-alkyle,
(f) halogéno ;
R⁵, R⁶ et R⁷ sont chacun, de façon indépendante, choisis dans le groupe constitué par
(a) hydrogène et
(b) alkyle en C₁₋₆,
ou R⁵ et R⁶ forment, ensemble, avec l'atome de carbone auquel ils sont rattachés un cycle carboné monocyclique saturé à 3, 4, 5, 6 ou 7 atomes.

3. Composé selon la revendication 2, de formule la 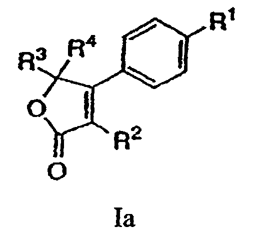 dans laquelle
R¹ est choisi dans le groupe constitué par
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² est choisi dans le groupe constitué par
(a) cyclohexyle et
(b) phényle ou naphtyle mono-, di- ou trisubstitués dans lesquels le substituant est choisi dans le groupe constitué par
(1) hydrogène,
(2) halogéno,
(3) alcoxy en C₁₋₄,
(4) alkylthio en C₁₋₄,
(5) CN,
(6) CF₃,
(7) alkyle en C₁₋₄,
(8) N₃,
(9) -CO₂H,
(10) -CO₂-(alkyle en C₁₋₄),
(11) -C(R⁵)(R⁶)-OH,
(12) -(R⁵)(R⁶)-O(alkyle en C₁₋₄) et
(13) -(alkyl en C₁₋₆)-CO₂R⁵,
(14) benzyloxy,
(15) -O-(alkyl en C₁₋₄)-CO₂R⁵,
(16) -O-(alkyl en C₁₋₄)-NR⁵R⁶
R³ est CH₂OR⁷, CN, CH₂CN ou fluoro(alkyle en C₁₋₆);
R⁴ est
(a) un phényle mono- ou disubstitué ou un hétéroaryle mono- ou disubstitué, lesdits substituants étant choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) ―C(R⁵)(R⁶)-OH et
(10) ―C(R⁵)(R⁶)-O(alkyle en C₁₋₄) ; ou
(b) alkyle en C₁₋₆, CH₂OR⁷, CN, CH₂CN ou fluoro(alkyle en C₁₋₆) ou R³ et R⁴ forment ensemble avec l'atome de carbone auquel ils sont attachés un cycle monocyclique saturé à 3, 4, 5, 6 ou 7 atomes, contenant éventuellement 1 ou 2 hétéroatomes choisis de façon indépendante parmi O, S, N et éventuellement tétrasubstitué par R¹²,
chaque R¹² est, de façon indépendante, choisi dans le groupe constitué par
(a) hydrogène,
(b) alkyle en C₁₋₆,
(c) phényle ou phényle monosubstitué, les substituants pouvant être halogéno, alkyle en C₁₋₆, alcoxy en C₁₋₆, alkylthio en C₁₋₆, CN ou CF₃,
(d) benzyle ou benzyle monosubstitué, les substituants pouvant être halogéno, alkyle en C₁₋₆, alcoxy en C₁₋₆, alkylthio en C₁₋₆, CN ou CF₃,
(e) -CO₂-alkyle,
(f) halogéno ;
R⁵, R⁶ et R⁷ sont chacun, de façon indépendante, choisis dans le groupe constitué par
(a) hydrogène et
(b) alkyle en C₁₋₆,
ou R⁵ et R⁶ forment, ensemble avec l'atome de carbone auquel ils sont rattachés un cycle carboné monocyclique saturé à 3, 4, 5, 6 ou 7 atomes.

4. Composé selon la revendication 3, de formule la 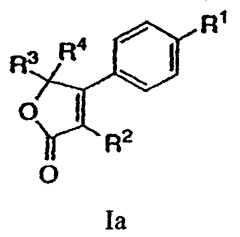 dans laquelle
R¹ est choisi dans le groupe constitué par
(a) S(O)₂CH₃,
(b) S(O)₂NH₂,
(c) S(O)₂NHNH₂,
R² est choisi dans le groupe constitué par phényle ou naphtyle mono-, di- ou trisubstitués dans lesquels le substituant est choisi dans le groupe constitué par
(1) hydrogène,
(2) halogéno,
(3) alcoxy en C₁₋₄,
(4) alkylthio en C₁₋₄,
(5) CN,
(6) CF₃,
(7) alkyle en C₁₋₄,
(8) N₃,
(9) -CO₂H,
(10) -CO₂-(alkyle en C₁₋₄),
(11) -C(R⁵)(R⁶)-OH,
(12) -C(R⁵)(R⁶)-O(alkyle en C₁₋₄) et
(13) -(alkyl en C₁₋₄)-CO₂R⁵,
R³ est CH₂OR⁷, CN, CH₂CN ou fluoro(alkyle en C₁₋₄) ;
R⁴ est
(a) un phényle mono- ou disubstitué ou un hétéroaryle mono- ou disubstitué, lesdits substituants étant choisis dans le groupe constitué par
(1) hydrogène,
(2) fluoro, chloro et bromo,
(3) alkyle en C₁₋₄,
(4) alcoxy en C₁₋₄,
(5) alkylthio en C₁₋₄,
(6) CN,
(7) CF₃ ou
(b) alkyle en C₁₋₄, CH₂OR⁷, CN, CH₂CN ou fluoro(alkyle en C₁₋₄) ou R³ et R⁴ forment ensemble avec l'atome de carbone auquel ils sont attachés un cycle monocyclique saturé à 3, 4, 5, 6 ou 7 atomes, contenant éventuellement 1 ou 2 hétéroatomes choisis de façon indépendante parmi O, S, N et éventuellement trisubstitué par R¹²,
chaque R¹² est, de façon indépendante, choisi dans le groupe constitué par
(a) hydrogène,
(b) alkyle en C₁₋₄,
(c) phényle ou phényle monosubstitué, les substituants pouvant être halogéno, alkyle en C₁₋₄, alcoxy en C₁₋₄, alkylthio en C₁₋₆, CN ou CF₃,
(d) benzyle ou benzyle monosubstitué, les substituants pouvant être halogéno, alkyle en C₁₋₄, alcoxy en C₁₋₄, alkylthio en C₁₋₄, CN ou CF₃,
(e) -CO₂-alkyle en C₁₋₄,
(f) halogéno ;
R⁵, R⁶ et R⁷ sont chacun, de façon indépendante, choisis dans le groupe constitué par
(a) hydrogène et
(b) alkyle en C₁₋₄,
ou R⁵ et R⁶ forment, ensemble, avec l'atome de carbone auquel ils sont rattachés un cycle carboné monocyclique saturé à 3, 4, 5, 6 ou 7 atomes.

5. Composé selon la revendication 4, de formule la 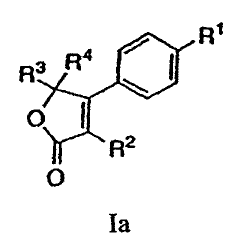 dans laquelle
R¹ est choisi dans le groupe constitué par
(a) S(O)₂CH₃,
(b) S(O)₂NH₂,
R² est choisi dans le groupe constitué par phényle ou naphtyle mono-, di- ou trisubstitués dans lesquels le substituant est choisi dans le groupe constitué par
(1) hydrogène,
(2) halogéno,
(3) alcoxy en C₁₋₄,
(4) alkylthio en C₁₋₄,
(5) CN,
(6) CF₃,
(7) alkyle en C₁₋₄,
(8) N₃, et
(9) -C(R⁵)(R⁶)-OH,
R³ est CH₂OR⁷, CN, CH₂CN ou fluoro(alkyle en C₁₋₃) ;
R⁴ est
(a) un phényle mono- ou disubstitué ou un hétéroaryle mono- ou disubstitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₄,
(6) CN,
(7) CF₃ ou
(b) alkyle en C₁₋₃, CH₂OR⁷, CN, CH₂CN ou fluoro(alkyle en C₁₋₃) ;
R⁵, R⁶ et R⁷ sont chacun, de façon indépendante, choisis dans le groupe constitué par
(a) hydrogène et
(b) alkyle en C₁₋₃.

6. Composé selon la revendication 5, de formule la 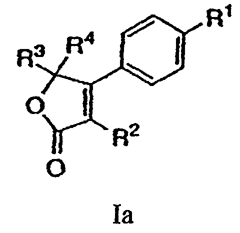 dans laquelle
R¹ est choisi dans le groupe constitué par
(a) S(O)₂CH₃,
(b) S(O)₂NH₂,
R² est choisi dans le groupe constitué par phényle ou naphtyle mono-, di- ou trisubstitués dans lesquels le substituant est choisi dans le groupe constitué par
(1) hydrogène,
(2) halogéno, choisi dans le groupe constitué par fluoro, chloro et bromo,
(3) alcoxy en C₁₋₃,
(4) alkylthio en C₁₋₃,
(5) CN et
(6) alkyle en C₁₋₃,
R³ est CH₂OR⁷, CN, CH₂CN ou fluoro(alkyle en C₁₋₃) ;
R⁴ est
(a) un phényle mono- ou disubstitué ou un hétéroaryle mono- ou disubstitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₄,
(6) CF₃ ou
(b) alkyle en C₁₋₃, CH₂OR⁷, CN, CH₂CN ou fluoro(alkyle en C₁₋₃) ;
R⁵, R⁶ et R⁷ sont chacun, de façon indépendante, choisis dans le groupe constitué par
(a) hydrogène et
(b) alkyle en C₁₋₃.

7. Composé selon la revendication 6, de formulé la 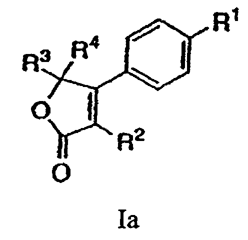 dans laquelle
R¹ est choisi dans le groupe constitué par
S(O)₂CH₃,
R² est choisi dans le groupe constitué par phényle ou naphtyle mono-, di- ou trisubstitués dans lesquels le substituant est choisi dans le groupe constitué par
(1) hydrogène,
(2) halogéno, choisi dans le groupe constitué par fluoro, chloro et bromo,
(3) méthoxy,
(4) méthylthio,
(5) CN et
(6) méthyle et éthyle,
R³ est CH₂OR⁷, CN, CH₂CN ou trifluorométhyle ;
R⁴ est
(a) un phényle mono- ou disubstitué ou un hétéroaryle mono- ou disubstitué, lesdits substituants étant choisis dans le groupe constitué par
(1) hydrogène,
(2) fluoro, chloro et bromo,
(3) méthyle et éthyle,
(4) méthoxy,
(5) méthylthio,
(6) CF₃ ou
(b) alkyle en C₁₋₃, CH₂OR⁷, CN, CH₂CN ou trifluorométhyle ;
R⁵, R⁶ et R⁷ sont chacun, de façon indépendante, choisis dans le groupe constitué par
(a) hydrogène et
(b) méthyle.

8. Composé selon la revendication 2, dans lequel
R² est choisi dans le groupe constitué par
(a) hétéroaryle mono- ou disubstitué choisi dans le groupe constitué par
(1) furanyle,
(2) diazinyle, triazinyle et tétrazinyle,
(3) imidazolyle,
(4) isoxazolyle,
(5) isothiazolyle,
(6) oxadiazolyle,
(7) oxazolyle,
(8) pyrazolyle,
(9) pyrrolyle,
(10) thiadiazolyle,
(11) thiazolyle,
(12) thiényle,
(13) triazolyle et
(14) tétrazolyle,
où lesdits substituants sont choisis dans le groupe constitué par
(1) hydrogène,
(2) fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ;
(b) un benzohétérocycle, un benzocarbocycle ou un hétérocycloalkyle mono- ou disubstitués choisis dans le groupe constitué par
(1) 2-indolyle,
(2) 3-indolyle,
(3) 1-méthyl-5-indolyle,
(4) 2-benzofuranyle,
(5) 3-benzofuranyle,
(6) 5-benzofuranyle,
(7) 6-benzofuranyle,
(8) 2-benzothiényle,
(9) 3-benzothiényle,
(10) 5-benzothiényle,
(11) 6-benzothiényle, 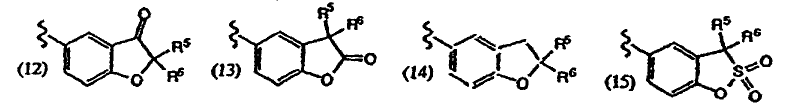 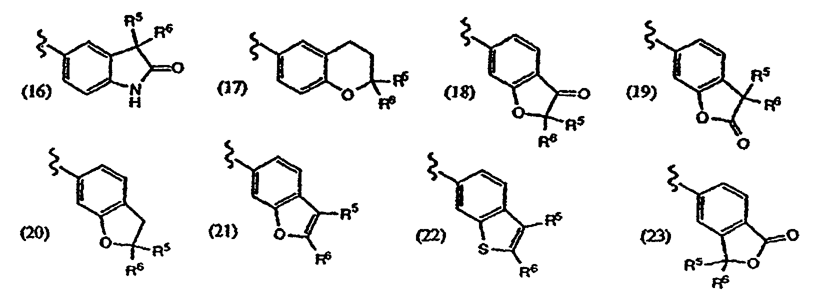 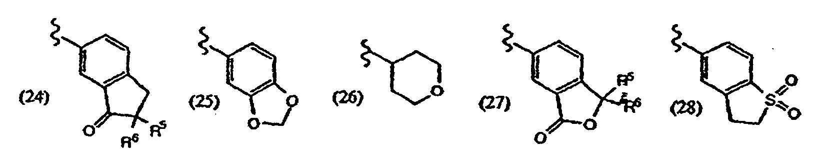 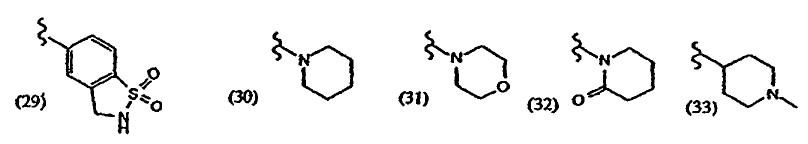
où lesdits substituants sont choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄) ;

9. Composé selon la revendication 8, dans lequel
R² est un hétéroaryle mono- ou disubstitué, l'hétéroaryle étant choisi dans le groupe constitué par
(1) furanyle,
(2) diazinyle, triazinyle et tétrazinyle,
(3) imidazolyle,
(4) isoxazolyle,
(5) isothiazolyle,
(6) oxadiazolyle,
(7) oxazolyle,
(8) pyrazolyle,
(9) pyrrolyle,
(10) thiadiazolyle,
(11) thiazolyle,
(12) thiényle,
(13) triazolyle et
(14) tétrazolyle,
où lesdits substituants sont choisis dans le groupe constitué par
(1) hydrogène,
(2) fluoro ou chloro,
(3) alcoxy en C₁₋₃,
(4) alkylthio en C₁₋₆,
(5) CN,
(6) CF₃,
(7) alkyle en C₁₋₃,
(8) -C(R⁵)(R⁶)-OH ;
(9) -C(R⁵)(R⁶)-O(alkyle en C₁₋₄).

10. Composé selon la revendication 9, dans lequel
R² est un hétéroaryle mono- ou disubstitué, l'hétéroaryle étant choisi dans le groupe constitué par
(1) 2-furanyle,
(2) 3-furanyle,
(3) 2-thiényle,
(4) 3-thiényle,
(5) 3-isoxazolyle,
(6) 4-isoxazolyle,
(7) 5-isoxazolyle,
(8) 3-isothiazolyle,
(9) 4-isothiazolyle,
(10) 5-isothiazolyle,
(11) 2-oxazolyle,
(12) 4-oxazolyle,
(13) 5-oxazolyle,
(14) 2-thiazolyle,
(15) 4-thiazolyle,
(16) 5-thiazolyle,
(17) 1,2,3-thiadiazol-4-yle,
(18) 1,2,3-thiadiazol-5-yle,
(19) 1,2,4-thiadiazol-3-yle,
(20) 1,2,4-thiadiazol-5-yle,
(21) 1,3,4-thiadiazol-2-yle,
(22) 1,2,5-thiadiazol-3-yle,
(23) 1,2,3-oxadiazol-4-yle,
(24) 1,2,3-oxadiazol-5-yle,
(25) 1,2,4-oxadiazol-3-yle,
(26) 1,2,4-oxadiazol-5-yle,
(27) 1,3,4-oxadiazol-2-yle,
(28) 1,2,5-oxadiazol-3-yle,
(29) pyrazol-4-yle,
(30) pyrazol-5-yle,
(31) 1,2,3-triadiazol-4-yle,
(32) 1,2,3-triadiazol-5-yle,
(33) 1,2,4-triadiazol-3-yle,
(34) 1,2,4-triadiazol-5-yle,
(35) 1,2-diazinyle,
(36) 1,3-diazinyle,
(37) 1,4-diazinyle,
(38) 1,2,3,4-tétrazin-5-yle,
(39) 1,2,4,5-tétrazin-4-yle,
(40) 1,3,4,5-tétrazin-2-yle, et
(41) 1,2,3,5-tétrazin-4-yle.

11. Composé selon la revendication 10, dans lequel
R² est un hétéroaryle mono- ou disubstitué, l'hétéroaryle étant choisi dans le groupe constitué par
(1) 3-isoxazolyle,
(2) 4-isoxazolyle,
(3) 5-isoxazolyle,
(4) 3-isothiazolyle,
(5) 4-isothiazolyle,
(6) 5-isothiazolyle,
(7) 2-oxazolyle,
(8) 4-oxazolyle,
(9) 5-oxazolyle,
(10) 2-thiazolyle,
(11) 4-thiazolyle,
(12) 5-thiazolyle,
(13) 1,2,3-thiadiazol-4-yle,
(14) 1,2,3-thiadiazol-5-yle,
(15) 1,2,4-thiadiazol-3-yle,
(16) 1,2,4-thiadiazol-5-yle,
(17) 1,3,4-thiadiazol-2-yle,
(18) 1,2,5-thiadiazol-3-yle,
(19) 1,2,3-oxadiazol-4-yle,
(20) 1,2,3-oxadiazol-5-yle,
(21) 1,2,4-oxadiazol-3-yle,
(22) 1,2,4-oxadiazol-5-yle,
(23) 1,3,4-oxadiazol-2-yle,
(24) 1,2,5-oxadiazol-3-yle,
(25) 1,2-diazinyle,
(26) 1,3-diazinyle et
(27) 1,4-diazinyle.

12. Composé selon la revendication 11, dans lequel
l'hétéroaryle est choisi dans le groupe constitué par
(1) 3-isothiazolyle,
(2) 4-isothiazolyle,
(3) 5-isothiazolyle,
(4) 2-oxazolyle,
(5) 4-oxazolyle,
(6) 5-oxazolyle,
(7) 2-thiazolyle,
(8) 4-thiazolyle,
(9) 5-thiazolyle,
(10) 1,2-diazinyle,
(11) 1,3-diazinyle,
(12) 1,4-diazinyle et
où les substituants sont choisis dans le groupe constitué par
(1) hydrogène,
(2) fluoro ou chloro,
(3) alcoxy en C₁₋₃,
(4) alkylthio en C₁₋₃,
(5) CN,
(6) alkyle en C₁₋₃ et
(7) -C(R⁵)(R⁶)-OH,
R³ est CH₂OR⁷, CN, CH₂CN ou fluoro(alkyle en C₁₋₃) ;
R⁴ est
(a) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₄,
(6) CN,
(7) CF₃ ou
(b) alkyle en C₁₋₃, CH₂OR⁷, CN, CH₂CN ou fluoro(alkyle en C₁₋₃) ;
R⁵ et R⁶ sont, chacun de façon indépendante, un hydrogène, un méthyle ou un éthyle.

13. Composé selon la revendication 1 de formule Ib 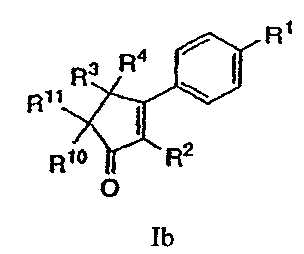 où
R¹ est choisi dans le groupe constitué par
(a) S(O)₂CH₃,
(b) S(O)₂NH₂,
(c) S(O)₂NHC(O)CF₃,
(d) S(O)(NH)NH₂,
(e) S(O)(NH)NHC(O)CF₃,
(f) P(O)(CH₃)NH₂,
(g) P(O)(CH₃)₂,
R² est choisi dans le groupe constitué par cyclohexyle et phényle ou naphtyle mono-, di- ou trisubstitués dans lesquels le substituant est choisi dans le groupe constitué par
(1) hydrogène,
(2) halogéno,
(3) alcoxy en C₁₋₆,
(4) alkylthio en C₁₋₆,
(5) CN,
(6) CF₃,
(7) alkyle en C₁₋₆,
(8) N₃,
(9) ―CO₂H,
(10) ―CO₂-(alkyle en C₁₋₄)
(11) ―C(R⁵)(R⁶)-OH,
(12) ―(R⁵)(R⁶)-O(alkyle en C₁₋₄) et
(13) ―(alkyl en C₁₋₆)-CO₂R⁵,
(14) benzyloxy,
(15) ―O-(alkyl en C₁₋₆)-CO₂R⁵,
(16) ―O-(alkyl en C₁₋₆)-NR⁵R⁶;
R³ est CH₂OR⁷, CN, CH₂CN ou fluoro(alkyle en C₁₋₆);
R⁴ est
(a) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) -C(R⁵)(R⁶)-OH et
(10) -C(R⁵)(R⁶)-O(alkyle en C₁₋₄), ou
(b) alkyle en C₁₋₆, CH₂OR⁷, CN, CH₂CN ou fluoro(alkyle en C₁₋₆) ; ou R³ et R⁴ forment ensemble avec l'atome de carbone auquel ils sont attachés un cycle monocyclique saturé à 3, 4, 5, 6 ou 7 atomes, contenant éventuellement 1
ou 2 hétéroatomes choisis de façon indépendante parmi O, S, N et éventuellement tétrasubstitué par R¹²,
chaque R¹² est, de façon indépendante, choisi dans le groupe constitué par
(a) hydrogène,
(b) alkyle en C₁₋₆,
(c) phényle ou phényle monosubstitué, les substituants pouvant être halogéno, alkyle en C₁₋₆, alcoxy en C₁₋₆, alkylthio en C₁₋₆, CN ou CF₃,
(d) benzyle ou benzyle monosubstitué, les substituants pouvant être halogéno, alkyle en C₁₋₆, alcoxy en C₁₋₆, alkylthio en C₁₋₆, CN ou CF₃,
(e) ―CO₂-alkyle,
(f) halogéno,
R⁵, R⁶ et R⁷ sont chacun, de façon indépendante, choisis dans le groupe constitué par
(a) hydrogène et
(b) alkyle en C₁₋₆,
ou R⁵ et R⁶ forment, ensemble avec l'atome de carbone auquel ils sont rattachés un cycle carboné monocyclique saturé à 3, 4, 5, 6 ou 7 atomes ;
R¹⁰ et R¹¹ sont de façon indépendante
(a) un hydrogène,
(b) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(8) N₃,
(9) ―C(R¹³)(R¹⁴)-OH et
(10) ―C(R¹³)(R¹⁴)-O(alkyle en C₁₋₄) ou
(c) alkyle en C₁₋₇, CH₂OR⁷, CN, CH₂CN ou fluoro(alkyle en C₁₋₆) ou R¹⁰ et R¹¹ forment ensemble avec l'atome de carbone auquel ils sont attachés un cycle carboné monocyclique saturé à 3, 4, 5, 6 ou 7 atomes ;
R¹³ et R¹⁴ sont, de façon indépendante, choisis dans le groupe constitué par
(a) hydrogène et
(b) alkyle en C₁₋₆, ou
R¹³ et R¹⁴ forment ensemble un O ou un S lié par une double liaison.

14. Composé selon la revendication 13 de formule Ib 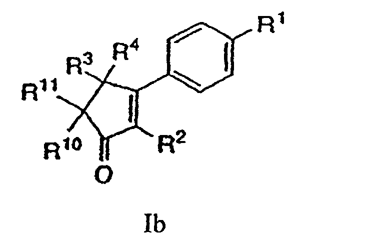 où
R¹ est choisi dans le groupe constitué par
(a) S(O)₂CH₃,
(b) S(O)₂NH₂,
(c) S(O)(NH)NH₂,
R² est choisi dans le groupe constitué par phényle ou naphtyle mono-, di- ou trisubstitués dans lesquels le substituant est choisi dans le groupe constitué par
(1) hydrogène,
(2) halogéno,
(3) alcoxy en C₁₋₆,
(4) alkylthio en C₁₋₄,
(5) CN,
(6) CF₃,
(7) alkyle en C₁₋₄,
(8) N₃,
(9) ―CO₂H,
(10) ―CO₂-(alkyle en C₁₋₄)
(11) ―C(R⁵)(R⁶)-OH,
(12) ―C(R⁵)(R⁶)-O(alkyle en C₁₋₄) et
(13) ―(alkyl en C₁₋₆)-CO₂R⁵,
R³ est CH₂OR⁷, CN, CH₂CN ou fluoro(alkyle en C₁₋₆);
R⁴ est
(a) un phényle substitué ou un hétéroaryle substitué, lesdits substituants étant choisis dans le groupe constitué par
(1) hydrogène,
(2) fluoro, chloro et bromo,
(3) alkyle en C₁₋₄,
(4) alcoxy en C₁₋₄,
(5) alkylthio en C₁₋₄,
(6) CN,
(7) CF₃ ou
(b) alkyle en C₁₋₄, CH₂OR⁷, CN, CH₂CN ou fluoro(alkyle en C₁₋₄); ou R³ et R⁴ forment ensemble avec l'atome de carbone auquel ils sont attachés un cycle monocyclique saturé à 3, 4, 5, 6 ou 7 atomes, contenant éventuellement 1 ou 2 hétéroatomes choisis de façon indépendante parmi O, S, N et éventuellement trisubstitué par R¹²,
chaque R¹² est, de façon indépendante, choisi dans le groupe constitué par
(a) hydrogène,
(b) alkyle en C₁₋₄,
(c) phényle ou phényle monosubstitué, les substituants pouvant être halogéno, alkyle en C₁₋₄, alcoxy en C₁₋₄, alkylthio en C₁₋₆, CN ou CF₃,
(d) benzyle ou benzyle monosubstitué, les substituants pouvant être halogéno, alkyle en C₁₋₄, alcoxy en C₁₋₄, alkylthio en C₁₋₄, CN ou CF₃,
(e) -CO₂-alkyle en C₁₋₄,
(f) halogéno,
R⁵, R⁶ et R⁷ sont chacun, de façon indépendante, choisis dans le groupe constitué par
(a) hydrogène et
(b) alkyle en C₁₋₄,
ou R⁵ et R⁶ forment, ensemble avec l'atome de carbone auquel ils sont rattachés un cycle carboné monocyclique saturé à 3, 4, 5, 6 ou 7 atomes ;
R¹⁰ et R¹¹ sont de façon indépendante
(a) un hydrogène,
(b) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₆,
(4) alcoxy en C₁₋₆,
(5) alkylthio en C₁₋₆,
(6) CN,
(7) CF₃,
(c) alkyle en C₁₋₄, CH₂OR⁷, CN, CH₂CN ou fluoro(alkyle en C₁₋₄) ou R¹⁰ et R¹¹ forment ensemble avec l'atome de carbone auquel ils sont attachés un cycle carboné monocyclique saturé à 3, 4, 5, 6 ou 7 atomes ;
R¹³ et R¹⁴ sont, de façon indépendante, choisis dans le groupe constitué par
(a) hydrogène et
(b) alkyle en C₁₋₄.

15. Composé selon la revendication 14 de formule Ib 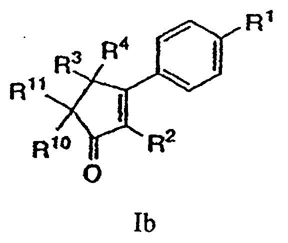 où
R¹ est choisi dans le groupe constitué par
(a) S(O)₂CH₃,
(b) S(O)₂NH₂,
R² est choisi dans le groupe constitué par
phényle ou naphtyle mono-, di- ou trisubstitués dans lesquels le substituant est choisi dans le groupe constitué par
(1) hydrogène,
(2) halogéno,
(3) alcoxy en C₁₋₄,
(4) alkylthio en C₁₋₄,
(5) CN,
(6) CF₃,
(7) alkyle en C₁₋₄,
(8) ―CO₂H, et
(9) ―C(R⁵)(R⁶)-OH,
R³ est CH₂OR⁷, CN, CH₂CN ou fluoro(alkyle en C₁₋₃) ;
R⁴ est
(a) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₄,
(6) CN,
(7) CF₃ ou
(b) alkyle en C₁₋₃, CH₂OR⁷, CN, CH₂CN ou fluoro(alkyle en C₁₋₃) ;
R⁵, R⁶ et R⁷ sont chacun, de façon indépendante, choisis dans le groupe constitué par
(a) hydrogène et
(b) alkyle en C₁₋₃,
R¹⁰ et R¹¹ sont de façon indépendante
(a) un hydrogène,
(b) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₃,
(6) CN,
(7) CF₃,
(c) alkyle en C₁₋₃, CH₂OR⁷, CN, CH₂CN ou fluoro(alkyle en C₁₋₄) ;
R¹³ et R¹⁴ sont, de façon indépendante, choisis dans le groupe constitué par
(a) hydrogène,
(b) alkyle en C₁₋₃.

16. Composé selon la revendication 15 de formule Ib 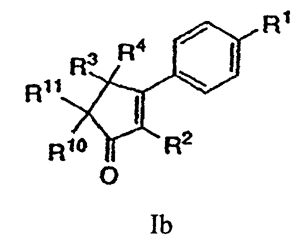 où
R¹ est choisi dans le groupe constitué par
(a) S(O)₂CH₃,
(b) S(O)₂NH₂,
R² est choisi dans le groupe constitué par phényle ou naphtyle mono-, di- ou trisubstitués dans lesquels le substituant est choisi dans le groupe constitué par
(1) hydrogène,
(2) halogéno, choisi dans le groupe constitué par fluoro, chloro et bromo,
(3) alcoxy en C₁₋₃,
(4) alkylthio en C₁₋₃,
(5) CN et
(6) alkyle en C₁₋₃,
R³ est CH₂OR⁷, CN, CH₂CN ou fluoro(alkyle en C₁₋₃) ;
R⁴ est
(a) un phényle substitué ou un hétéroaryle substitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₄,
(6) CF₃, ou
(b) alkyle en C₁₋₃, CH₂OR⁷, CN, CH₂CN ou fluoro(alkyle en C₁₋₃);
R⁵, R⁶ et R⁷ sont chacun, de façon indépendante, choisis dans le groupe constitué par
(a) hydrogène et
(b) alkyle en C₁₋₃,
R¹⁰ et R¹¹ sont de façon indépendante
(a) un hydrogène,
(b) un phényle mono- ou disubstitué ou un hétéroaryle mono- ou disubstitué, lesdits substituants étant choisis dans le groupe constitué par
(1) hydrogène,
(2) halogéno, incluant fluoro, chloro, bromo et iodo,
(3) alkyle en C₁₋₃,
(4) alcoxy en C₁₋₃,
(5) alkylthio en C₁₋₃,
(6) CF₃,
(c) alkyle en C₁₋₃, CH₂OR⁷, CN, CH₂CN ou fluoro(alkyle en C₁₋₄) ;
R¹³ et R¹⁴ sont, de façon indépendante, choisis dans le groupe constitué par
(a) hydrogène,
(b) alkyle en C₁₋₃.

17. Composé choisi dans le groupe constitué par
(a) 5,5-diméthyl-4-(4-(méthylsulfonyl)phényl)-3-(4-méthoxyphényl)-2-(5H)-furanone,
(b) 5,5-diméthyl-3-(3-méthoxyphényl)-4-(4-(méthylsulfonyl)phényl)-2-(5H)-furanone,
(c) 5,5-diméthyl-3-(4-isopropylphényl)-4-(4-(méthylsulfonyl)phényl)-2-(5H)-furanone,
(d) 5,5-diméthyl-4-(4-(méthylsulfonyl)phényl)-3-phényl-2-(5H)-furanone,
(e) 3-(benzo[1,3]dioxol-5-yl)-5,5-diméthyl-4-(4-méthylsulfonyl)phényl)-2-(5H)-furanone,
(f) 5,5-diméthyl-3-(4-méthylphényl)-4-(4-(méthylsulfonyl)phényl)-2-(5H)-furanone,
(g) 5,5-diméthyl-4-(4-(méthylsulfonyl)phényl)-3-(4-tri fluorométhylphényl)-2-(5H)-furanone,
(h) 5,5-diméthyl-3-(3-méthylphényl)-4-(4-(méthylsulfonyl)phényl)-2-(5H)-furanone,
(i) 5,5-diméthyl-3-cyclohexyl-4-(4-(méthylsulfonyl)phényl)-2-(5H)-furanone,
(j) 4-(4-(méthylsulfonyl)phényl)-3-phényl-1-oxa-spiro[4.4]non-3-én-2-one,
(k) 5,5-diméthyl-3-(4-méthylsulfonyl)phényl)-2-phénylcyclopent-2-énone,
(l) 4,4-diméthyl-3-(4-méthylsulfonyl)phényl)-2-phénylcyclopent-2-énone,
(m) 7-(4-(méthylsulfonyl)phényl)-6-phényl-4-oxa-spiro[2.4]hept-6-én-5-one et
(n) 5,5-bis(fluorométhyl)-3-phényl-4-(4-(méthylsulfonyl)phényl)-2-(5H)-furanone.

18. Composé selon la revendication 1, choisi parmi
(b) 3-(3,5-difluorophényl)-4-(4-(méthylsulfonyl)phényl)-1-oxa-spiro[4.4]non-3-én-2-one,
(d) 4-(4-(méthylsulfonyl)phényl)-3-phényl-5,5-bis(trifluorométhyl)-(5H)-furanone,
(e) 8-(4-(méthylsulfonyl)phényl)-7-phényl-5-oxa-spiro[2.4]oct-7-én-6-one,
(f) 6-(3-fluorophényl)-7-(4-(méthylsulfonyl)phényl)-4-oxa-spiro[2.4]hept-6-én-5-one,
(g) 7-(4-(méthylsulfonyl)phényl)-6-naphtalèn-2-yl-4-oxa-spiro[2.4]hept-6-én-5-one,
(h) 4-(6-naphtalèn-2-yl-5-oxo-4-oxa-spiro[2.4]hept-6-én-7-yl)benzènesulfonamide,
(i) 7-(4-fluorophényl)-8-(4-(méthylsulfonyl)phényl)-5-oxa-spiro[3.4]oct-7-én-6-one,
(j) 7-(3-fluorophényl)-8-(4-(méthylsulfonyl)phényl)-5-oxa-spiro[3.4]oct-7-én-6-one,
(k) 7-(3,4-difluorophényl)-8-(4-(méthylsulfonyl)phényl)-5-oxa-spiro[3.4]oct-7-én-6-one,
(l) 4-(6-oxo-7-phényl-5-oxa-spiro[3.4]oct-7-én-8-yl)benzènesulfonamide,
(m) 4-[7-(4-fluorophényl)-6-oxo-5-oxa-spiro[3.4]oct-7-én-8-yl)benzènesulfonamide,
(n) 4-[7-(3-fluorophényl)-6-oxo-5-oxa-spiro[3.4]oct-7-én-8-yl)benzènesulfonamide,
(o) 4-[7-(3,4-difluorophényl)-6-oxo-5-oxa-spiro[3.4]oct-7-én-8-yl)benzènesulfonamide,
(p) 3-(4-fluorophényl)-4-(4-(méthylsulfonyl)phényl)-1-oxa-spiro[4.4]non-3-én-2-one,
(q) 3-(3-fluorophényl)-4-(4-(méthylsulfonyl)phényl)-1-oxa-spiro[4.4]non-3-én-2-one,
(r) 3-(3,4-difluorophényl)-4-(4-(méthylsulfonyl)phényl)-1-oxa-spiro[4.4]non-3-én-2-one,
(s) 3-(3-chloro-4-fluorophényl)-4-(4-(méthylsulfonyl)phényl)-1-oxa-spiro[4.4]non-3-én-2-one,
(t) 4-(2-oxo-3-phényl-1-oxa-spiro[4.4]non-3-én-4-yl)benzènesul fonamide,
(u) 4-[3-(4-fluorophényl)-2-oxo-1-oxa-spiro[4.4]non-3-én-4-yl)benzènesulfonamide,
(v) 4-[3-(3-fluorophényl)-2-oxo-1-oxa-spiro[4.4]non-3-én-4-yl)benzènesulfonamide,
(w) 4-[3-(3,4-difluorophényl)-2-oxo-1-oxa-spiro[4.4]non-3-én-4-yl)benzènesulfonamide,
(x) 4-[3-(3-chloro-4-fluorophényl)-2-oxo-1-oxa-spiro[4.4]non-3-én-4-yl)benzènesulfonamide,
(z) 4-(2-oxo-3-phényl-1-oxa-spiro[4.5]déc-3-én-4-yl)benzènesulfonamide et
(aa) 4-[3-(4-fluorophényl)-2-oxo-1-oxa-spiro[4.5]déc-3-én-4-yl)benzènesulfonamide.

19. Composition pharmaceutique comprenant une quantité thérapeutiquement efficace non-toxique d'un composé selon la revendication 1, 2, 3, 4, 5, 6, 7, 8, 9, 10, 11, 12, 13, 14, 15, 16, 17 ou 18 ou d'un de leurs sels pharmaceutiquement acceptables et un support pharmaceutiquement acceptable.

20. Sel pharmaceutiquement acceptable d'un composé dont la formule est définie dans la revendication 1, 2, 3, 4, 5, 6, 7, 8, 9, 10, 11, 12, 13, 14, 15 ou 16.

21. Composé ou un de ses sels pharmaceutiquement acceptables selon la revendication 1, 2, 3, 4, 5, 6, 7, 8, 9, 10, 11, 12, 13, 14, 15, 16, 17 ou 18 pour son utilisation dans le traitement d'une maladie inflammatoire répondant à un traitement par un agent anti-inflammatoire non stéroïdien.

22. Utilisation d'un composé ou d'un de ses sels pharmaceutiquement acceptables selon la revendication 1, 2, 3, 4, 5, 6, 7, 8, 9, 10, 11, 12, 13, 14, 15, 16, 17 ou 18 dans la fabrication d'un médicament pour le traitement de maladies faisant intervenir la cyclooxygénase.
